(19) Europäisches Patentamt
European Patent Office
Office européen des brevets

(11) **EP 4 455 273 A1**

(12) **EUROPEAN PATENT APPLICATION**
published in accordance with Art. 153(4) EPC


(43) Date of publication:
**30.10.2024 Bulletin 2024/44**

(21) Application number: **22896168.6**

(22) Date of filing: **21.11.2022**

(51) International Patent Classification (IPC):
***C12N 5/0775** (2010.01)* ***A61K 35/28** (2015.01)*
***A61P 25/00** (2006.01)*

(52) Cooperative Patent Classification (CPC):
**A61K 35/28; A61P 25/00; C12N 5/00; C12N 5/06**

(86) International application number:
**PCT/KR2022/018430**

(87) International publication number:
**WO 2023/090971 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.11.2021 KR 20210161446**



(71) Applicant: **YJ Cerapeutics Inc.**
**Seoul 01811 (KR)**

(72) Inventors:
- **YUNE, Tae Young**
  **Seoul 01801 (KR)**
- **LEE, Jee Youn**
  **Seoul 02020 (KR)**

(74) Representative: **Isarpatent**
**Patent- und Rechtsanwälte**
**Barth Hassa Peckmann & Partner mbB**
**Friedrichstraße 31**
**80801 München (DE)**


(54) **COMPOSITION FOR INDUCING TRANSDIFFERENTIATION AND STEM CELLS TREATED THEREWITH**


(57) The present invention relates to a composition for inducing transdifferentiation through oligomerization of CtBPs, and uses of stem cells treated therewith. Since the compounds of the present invention can induce transdifferentiation of adult stem cells into neural progenitor cells by regulating the oligomerization of CtBPs, the compounds can be used for inducing oligomerization or transdifferentiation of CtBPs. In addition, since ciNSC5 derived from transdifferentiation exhibits remarkable therapeutic effects in an ALS mouse model, an MS mouse model, a PD rat model, and a chronic spinal cord injury rat model, ciNSC5 can be used as a cellular therapeutic agent.


[FIG.3]
A

YJ101-CTBP1 YJ102-CTBP1

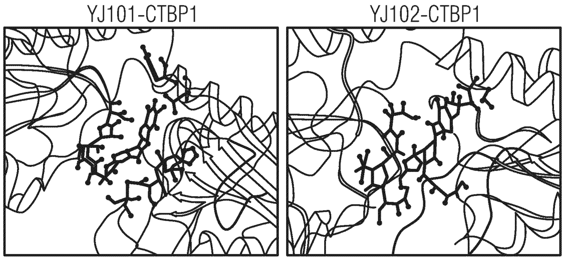

Docking score (ΔG)=4.980 kcal/ mol

Docking score (ΔG)=-5.458 kcal/ mol
NADH Docking score (ΔG) =-8 kcal/ mol

YJ103-CTBP1

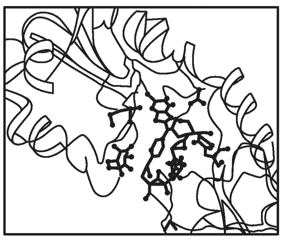

Docking score (ΔG)=3.767 kcal/ mol

| Ligand | Docking score (kcal/mol) |
|---|---|
| YJ101 | -4.980 |
| YJ102 | -5.458 |
| YJ103 | -3.767 |

EP 4 455 273 A1

[FIG.3]

B

| | | |
|---|---|---|
| BA | BB | BC |

[FIG.3]

BA

YJ101-CTBP1

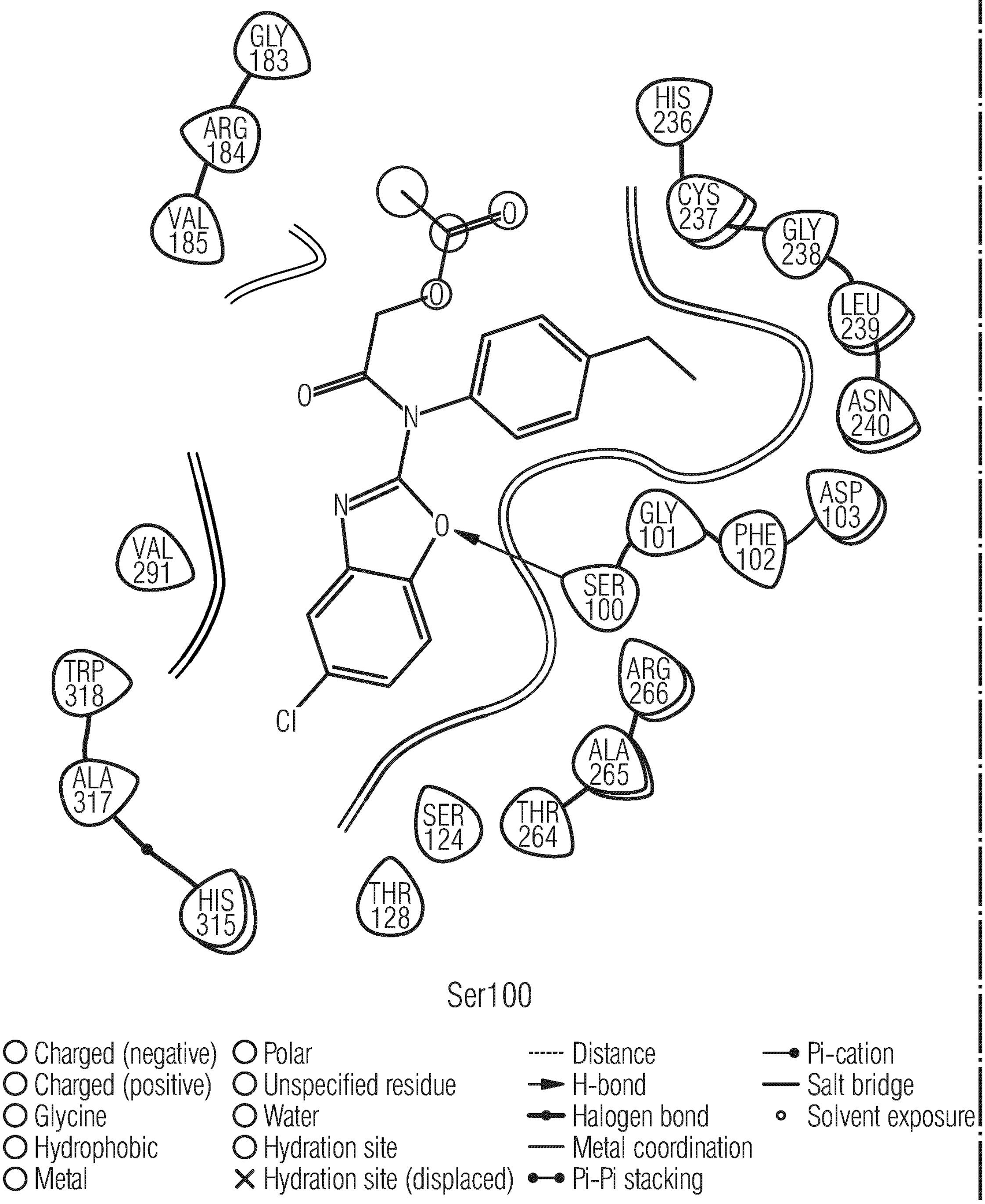




(Cont. next page)

[FIG.3]

BB

YJ102-CTBP1

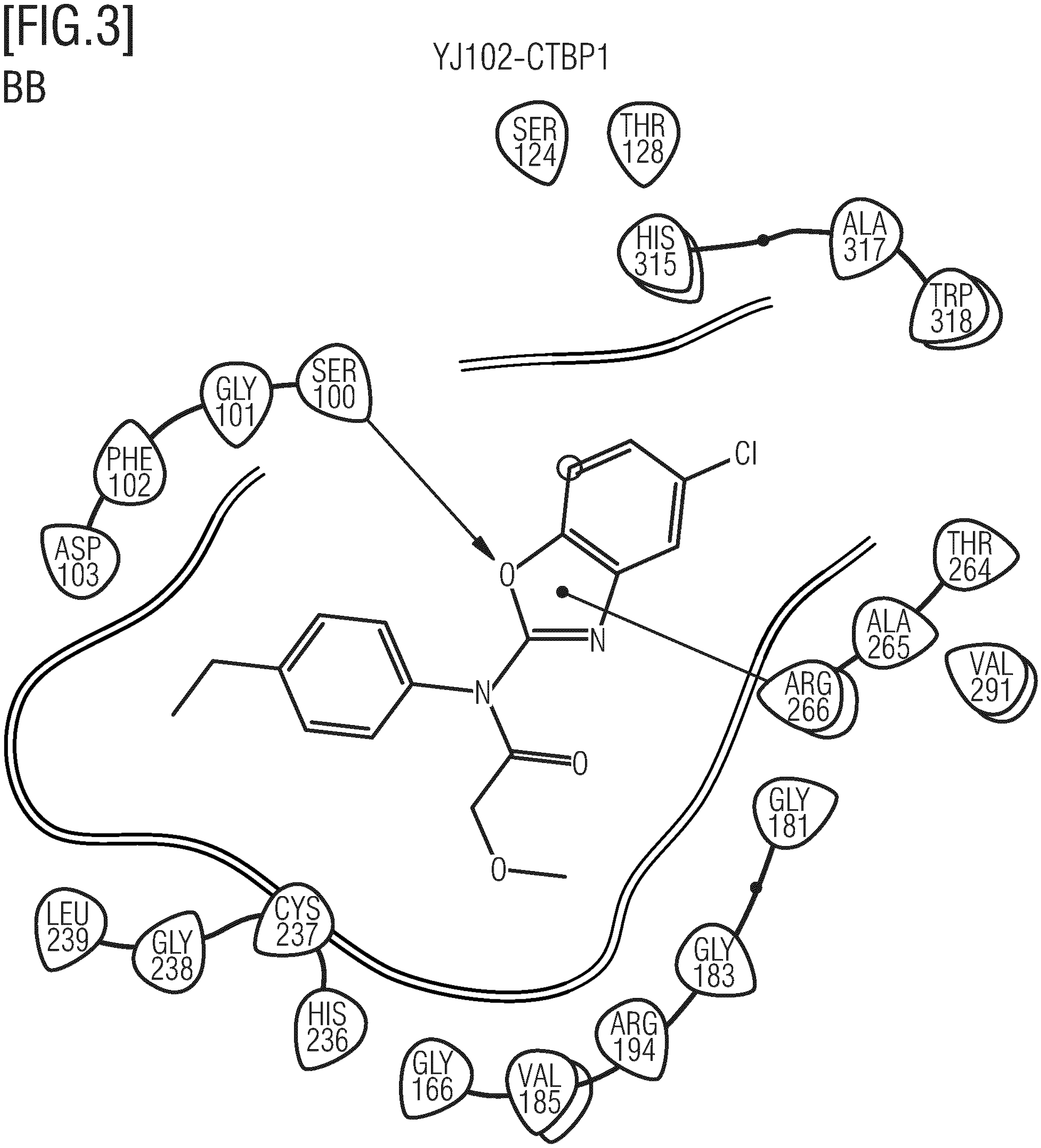


Ser110, Arg266

Charged (negative)
Charged (positive)
Glycine
Hydrophobic
Metal
Polar
Unspecified residue
Water
Hydration site
Hydration site (displaced)
Distance
H-bond
Halogen bond
Metal coordination
Pi-Pi stacking
Pi-cation
Salt bridge
Solvent exposure



(Cont. next page)

[FIG.3]

BC

YJ103-CTBP1

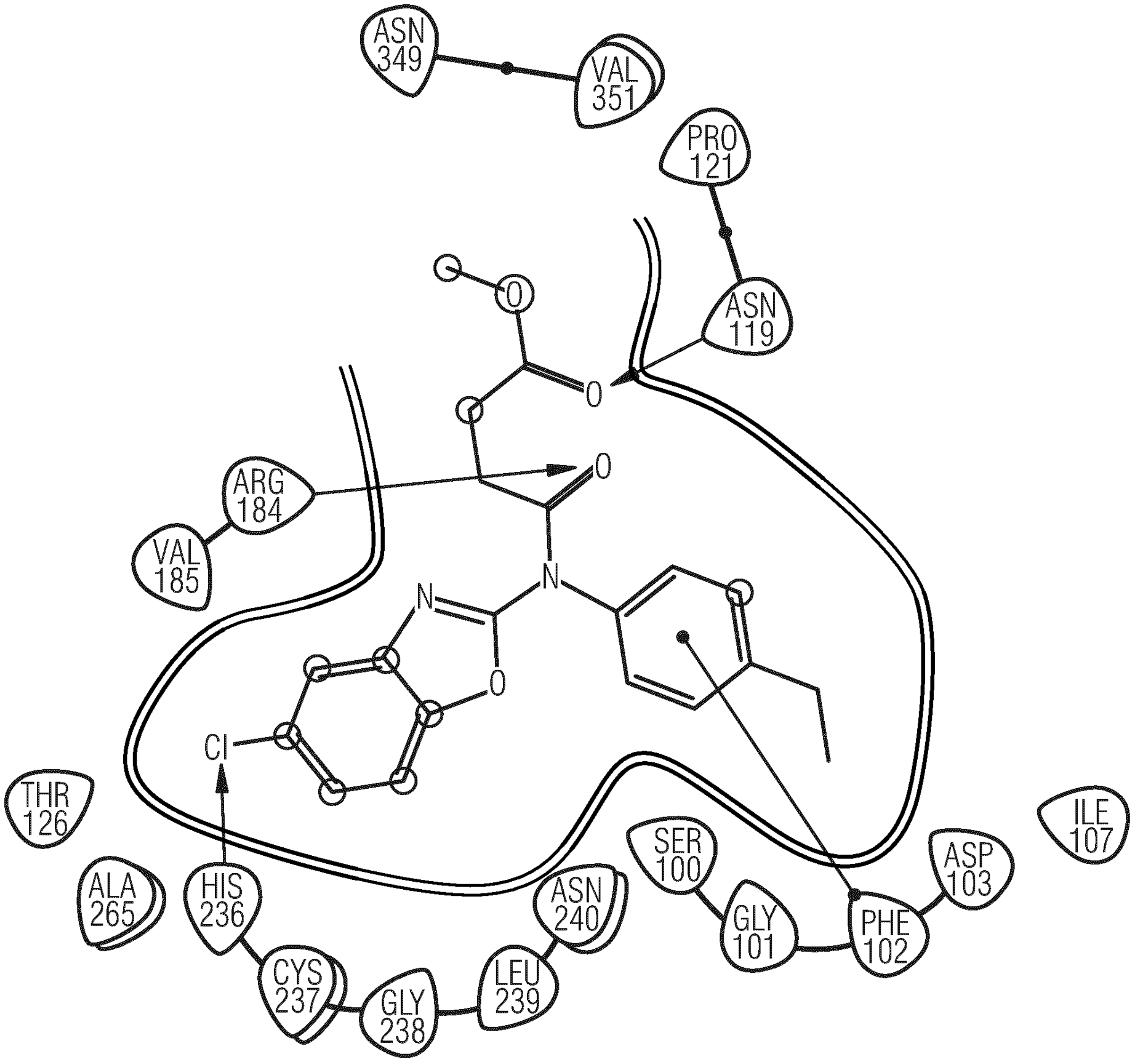

Phe102, Arg184, His236

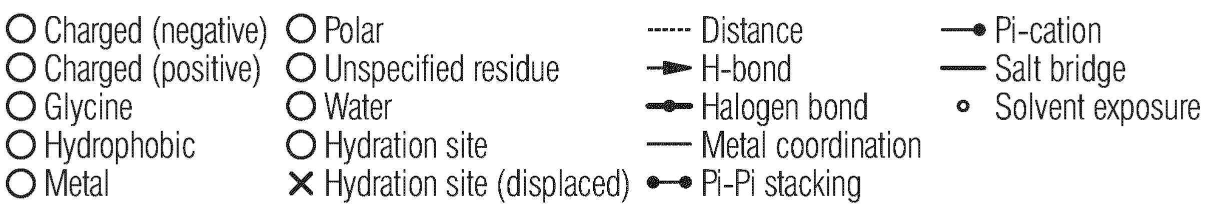

## Description

[Technical Field]

The present invention relates to a composition for inducing transdifferentiation through oligomerization of CtBPs, and uses of stem cells treated therewith.

[Background Art]

The nervous system is divided into the central nervous system and the peripheral nervous system, and the brain and spinal cord correspond to the central nervous system, and the rest corresponds to the peripheral nervous system. The peripheral nervous system is divided into the somatic nervous system, which includes motor and sensory nerves, and the autonomic nervous system. Neurons, astrocytes, and oligodendrocytes that constitute the central nervous system (brain and spinal cord) may be generated by differentiating neural stem cells (NSCs) or neural progenitor cells (NPCs) differentiated from pluripotent stem cells, whereas peripheral nerves (autonomic nerves, motor nerves, sensory nerves) and Schwann cells that constitute the peripheral nervous system are derived from neural crest stem cells (NCSCs) differentiated from the pluripotent stem cells. Neurons that constitute nervous tissue serve to transmit command signals from the central nervous system to the peripheral nervous system or transmit information input from the peripheral nervous system to the central nervous system through dendrites and axons. The axons transmit electrical signals to neurons farther from the central nervous system and are surrounded by myelin, which is an insulating structure to speed up signaling, which is called myelination. The mammalian brain is able to perform complex functions by generating a systematic neural network through a series of processes such as division and differentiation, survival and death, and synapse formation of neural stem cells. Even during the adult period, animal brain neurons produce many substances required for nerve growth, so that axons and dendrites grow, and continue to differentiate through constant remodeling of synaptic connections and neural networks every time new learning and memory occurs. When neurons do not receive target-derived survival factors such as nerve growth factor during the process of cell differentiation and synapse formation, cell death occurs, and cell death occurs due to stress and cytotoxic agents is a major cause of degenerative brain diseases. Unlike the central nervous system, the axons regenerate over a long period of time when the peripheral nervous system of animals is damaged. Axons behind of nerve injury site degenerate in a process known as Wallerian degeneration, and neural cell bodies begin axonal regrowth again, and Schwann cells divide, and then go through the developmental process again, such as determining the target nerve through survival and death and differentiating again to regenerate.

Degenerative neurological disease is a disease in which the central nervous system functions such as cognition, movement, and sensation deteriorate due to gradual structural and functional loss of neurons occurring by various causes. The degenerative neurological disease is accompanied by symptoms such as dementia, extrapyramidal abnormalities, cerebellar abnormalities, sensory disorders, and motor disorders, as neuron degeneration in specific parts of the nervous system progresses, and may also cause complex symptoms as abnormalities may occur in multiple areas at the same time. In this regard, the disease is diagnosed according to the clinical features shown in a patient, but the symptoms variously occur and different diseases often show common clinical symptoms to make diagnosis difficult. These neurodegenerative diseases show signs of the diseases gradually and often develop with aging. Once the disease develops, the disease continues to progress for several years or decades until death, and fundamental treatment is difficult, resulting in a significant social burden, and the cause of the disease is a genetic effect due to family history, but acquired factors are also known to play an important role therein. Depending on the clinical symptoms, the degenerative neurological diseases are broadly classified into progressive dementia (Alzheimer's disease, etc.), neurological abnormalities (Pick's disease, etc.), posture and motor abnormalities (Parkinson's disease, etc.), progressive ataxia, muscle atrophy and weakness, sensory and motor disorders, etc.

Multiple sclerosis (MS) occurs in genetically susceptible subjects and is an autoimmune, chronic inflammatory, and demyelinating central nervous system disease associated with immunological factors such as mediators, antibodies, and complements of the innate immune responses caused by environmental factors such as viruses. The MS is classified as an inflammatory demyelination disease and may occur at any age, but occurs most often between the ages of 20 and 40, and the incidence rate in women is known to be about twice higher than that of men. These neurogenic disorders are caused by damage to the myelin surrounding the axons of neurons due to an inflammatory reaction in the central nervous system. The lesion is a primary inflammatory disease that appears scattered in the white matter of the brain and spinal cord and is a typical phenomenon in the lesion area. Early, inflammation is temporary and a repair of damaged myelin occurs, but does not last. However, as time passes, pathological changes progress due to widespread activation of microglia, which is associated with intensive and chronic neurodegeneration, and clinically disorder symptoms become increasingly severe.

Amyotrophic lateral sclerosis (ALS), also known as Lou Gehrig's disease, is the most lethal progressive neurodegenerative disease characterized by remarked loss of motor neurons in the primary motor cortex, brainstem, and spinal cord. The loss of motor neurons destroys basic and fundamental movements such as breathing, and usually results in the patient's death within 2 to 5 years after diagnosis. The gradual deterioration of the patient's motor function eventually severely impairs the patient's breathing ability to require any form of respiratory assistance for the patient's survival. Other symptoms also include muscle weakness in the hands, arms, legs, or deglutition muscles. Some patients (e.g., frontotemporal dementia (FTD)-ALS) may develop frontotemporal dementia. According to the ALS Association, approximately 5,600 people in the United States are diagnosed with ALS each year. The incidence of ALS is 2 per 100,000 people. Two types of ALS have been described: One type is sporadic ALS (sALS), which is the most common form of ALS in the United States and accounts for 90 to 95% of all diagnosed cases; and the other type is familial ALS (fALS), which mainly occurs in familial lines with dominant inheritance and accounts for only about 5 to 10% of all cases in the United States. sALS and fALS are clinically indistinguishable. Currently, riluzole, a glutamate toxicity inhibitor, and edaravone, an antioxidant, have been prescribed in clinical trials, but these drugs have the effect of slightly lowering the progression of ALS, but may not be expected to have therapeutic effects. Another strategy for treating ALS is stem cell-based therapy. Stem cells have the potential to differentiate into motor neurons and replace degenerative motor neurons in the central nervous system of ALS patients, such as the primary motor cortex, brainstem, and spinal cord. Actually, the stem cells include induced pluripotent stem cells (iPSCs), mesenchymal stem cells (MSCs) (such as bone marrow mesenchymal stem cells (BMSCs) and adipose stem cells (ASCs)) and neural tissue-derived neural stem cells (such as fetal spinal neural stem cells (NSCs), and multipotent neural progenitor cells (NPCs)), so that the possibility of stem cells derived from various sources to replace degenerative motor neurons during ALS progression has been proposed (see e.g., Kim et al., Exp. Neurobiol., 2014, 23(3), 207-214).

Parkinson's disease is a disease first reported by James Parkinson in 1817, and a type of representative degenerative brain disease in which motor ability is lost due to loss of neurons in the substantia nigra (SN), where dopamine, a neurotransmitter that regulates muscles, is secreted, and dopamine deficiency in the striatum due to aging. Parkinson's disease occurs approximately 1% of the population over the age of 65 and approximately 5% of the population over the age of 85 (Twelves et al., Mov Disord 18, 19-31). Characteristic clinical symptoms include resting tremor, bradykinesia, rigidity, and postural instability, and are caused by selective loss of dopaminergic neurons in the substantia nigra, and the presence of intraneural proteinous inclusions known as Lewy body is a typical pathological characteristic (Olanow et al., Annu Rev Neurosci 22,123-44). The occurrence cause of Parkinson's disease is not accurately known. In the case of most sporadic forms of Parkinson's disease, the cause is little known as idiopathic, but the complex interaction between environmental factors and genetic susceptibility, which has not yet been fully identified, is estimated as an important cause (Langston et al., Ann Neurol 44(3 Suppl 1): S45-52). Parkinson's disease pathologically causes behavioral abnormalities due to the specific loss of dopaminergic neurons and neurite fibers containing melanin pigment present in the substantia nigra of the midbrain and dopamine deficiency in the striatum. In addition, Lewy bodies, which are proteinous inclusions, are observed as a marker of the disease in the neurons of patients with Parkinson's disease. Representative pathogenic mechanisms of Parkinson's disease include oxidative stress, mitochondrial dysfunction, ubiquitin-proteasome dysfunction, and accumulation of misfolded proteins.

When spinal cord neurons are damaged due to trauma, etc., paralysis of human body functions occurs. Spinal cord nerve tissue has a simpler structure than the brain, but regeneration is not easy. Pathological phenomena occurring after spinal cord injury are largely divided into primary injury and secondary injury depending on time. Primary injury is a phenomenon that occurs within a few minutes immediately after injury, and mainly involves necrosis of cells at the wound site, and during this period, cells are destroyed so quickly so that it is almost impossible to be treated with pharmacological treatment. Secondary injury following primary injury progresses slowly over several hours to several days, and not only the cells in the injured area degenerate, but also the surrounding undamaged neurons and oligodendrocytes gradually begin to die by apoptosis. Cell death continues to progress around the injured area, and thus eventually, the injured area in the spinal cord is gradually expanded. In addition, degeneration of the axon, which is a moving path for nerve signals, and the myelin sheath that helps the function of the axon occurs to ultimately form a cystic cavity within the spinal cord and prevent further nerve signal transmission, thereby causing a permanent functional loss. For a long time, research has been conducted to suppress or palliate permanent functional paralysis caused by spinal cord injury through research on regeneration and identification of the causes of pathological effects that occur after spinal nerve trauma. Because the initial mechanism of spinal cord injury progresses so rapidly that it is difficult to be treated with pharmacological treatment, it is important to develop therapeutic agents as a pharmacological strategy that addresses secondary mechanisms.

To date, various pharmacological treatments have been attempted, including steroids, antioxidants, glutamate receptor inhibitors, ion channel inhibitors, anti-inflammatory drugs, and nerve growth factors, but only methylprednisolone has been currently used clinically. However, methylprednisolone has the limitation that may expect the effect only when administered within 8 hours after spinal cord injury, and has anti-inflammatory, antioxidant, and anti-apoptotic effects to reduce secondary injury and cause some recovery, but has the risk of infection and side effects capable of causing gastrointestinal complications, so that there is virtually no effective therapeutic agent for spinal cord injury at present.

Until now, the focus has been only on developing treatments to reduce the degree of secondary injury that occurs after injury, not nerve regeneration, but recently, as research on stem cells has been actively conducted in various fields, the possibility of nerve regeneration treatment for irreversible nervous system injury such as spinal cord injury is gradually increasing.

Meanwhile, cellular therapy includes the administration of living cells for any purpose, including, for example, regenerative medicine, organ transplantation, and even for purposes of diagnosis or prevention of any condition such as cancer. Stem cells are evaluated as having great potential in cell therapy. The stem cells are cells that may differentiate into various cells that constitute biological tissues, and are a general term for undifferentiated cells in the pre-differentiation stage that may be obtained from each tissue of the embryo, fetus, and adult. The stem cells differentiate into specific cells depending on a differentiation stimulus (environment), have a characteristic of self-renewing and proliferating (expanding) cells identical to themselves through cell division (self-renewal) and have the characteristic of proliferation (expansion) unlike cells that have completed differentiation and stopped cell division, and is characterized by being differentiated into other cells by different environments or different differentiation stimuli to have plasticity in differentiation. The stem cells are broadly divided into pluripotent embryonic stem cells (ES cells), which are obtained from embryos and have the potential (totipotent) to differentiate into all cells, and multipotent adult stem cells obtained from each tissue. The adult stem cells include hematopoietic stem cells, mesenchymal stem cells (MSC), neural stem cells (NSC), intestinal stem cells, muscle stem cells, hair follicle stem cells, endothelial stem cells, etc. Among these, the mesenchymal stem cells exist in many mature tissues and are well known to have self-renewal ability and the potential to differentiate into various lineages, and may be easily obtained from adult tissues such as bone marrow, adipose tissue, placenta, umbilical cord blood, and Wharton's jelly.

In order to overcome the limitations of differentiation into specific cells using conventional induced pluripotent stem cells and cell therapy therethrough, a lot of studies on transdifferentiation (direct conversion) have been conducted as a technology that 'directly' converts any somatic cells into specific desired cells without going through a process of dedifferentiating the somatic cells into induced pluripotent stem cells capable of differentiating into all cells and then re-differentiating the stem cells to a lower level. However, a conventional transdifferentiation method has several problems. For example, a cross-differentiation using pluripotent stem cells formed during the process of inducing induced pluripotent stem cells may induce various tissue cells, but has a risk of teratoma development, and a differentiation method according to a genetic combination of induced pluripotent stem cell factors and discovered specific tissue cell-specific transcription factors has a low risk of teratoma development, but has difficulty in discovering specific factors that induce differentiation of each tissue cell.

[Disclosure]

[Technical Problem]

An object of the present invention is to provide a composition for inducing oligomerization of C-terminal binding proteins (CtBPs).

In addition, another object of the present invention is to provide a composition for inducing transdifferentiation.

In addition, yet another object of the present invention is to provide a method for inducing oligomerization of CtBPs.

In addition, yet another object of the present invention is to provide a method for inducing transdifferentiation.

In addition, yet another object of the present invention is to provide stem cells treated with at least one selected from the group consisting of a compound of Chemical Formula 1, a compound of Chemical Formula 2, and a compound of Chemical Formula 3.

In addition, yet another object of the present invention is to provide a pharmaceutical composition for preventing or treating nerve injury diseases, including the stem cells as an active ingredient.

In addition, yet another object of the present invention is to provide a use of a compound of Chemical Formula 1, a compound of Chemical Formula 2, or a compound of Chemical Formula 3, for use in inducing oligomerization of CtBPs.

In addition, yet another object of the present invention is to provide a use of a compound of Chemical Formula 1, a compound of Chemical Formula 2, or a compound of Chemical Formula 3, for use in inducing transdifferentiation.

In addition, yet another object of the present invention is to provide a use for preventing or treating nerve injury diseases by stem cells treated with at least one selected from the group consisting of a compound of Chemical Formula 1, a compound of Chemical Formula 2, and a compound of Chemical Formula 3.

In addition, still another object of the present invention is to provide a method for treating nerve injury diseases.

[Technical Solution]

In order to achieve the object, an aspect of the present invention provides a composition for inducing oligomerization of CtBPs, including at least one selected from the group consisting of a compound of Chemical Formula 1, a compound of Chemical Formula 2, and a compound of Chemical Formula 3.

In addition, another aspect of the present invention provides a composition for inducing transdifferentiation of adult stem cells into neural progenitor cells, including at least one selected from the group consisting of a compound of Chemical Formula 1, a compound of Chemical Formula 2, and a compound of Chemical Formula 3.

In addition, yet another aspect of the present invention provides a method for inducing oligomerization of CtBPs.

In addition, yet another aspect of the present invention provides a method for inducing transdifferentiation.

In addition, yet another aspect of the present invention provides stem cells treated with at least one selected from the group consisting of a compound of Chemical Formula 1, a compound of Chemical Formula 2, and a compound of Chemical Formula 3.

In addition, yet another aspect of the present invention provides a pharmaceutical composition for preventing or treating nerve injury diseases, including the stem cells, a stem cell culture, or a suspension culture as an active ingredient.

In addition, yet another aspect of the present invention provides a use of a compound of Chemical Formula 1, a compound of Chemical Formula 2, or a compound of Chemical Formula 3, for use in inducing oligomerization of CtBPs.

In addition, yet another aspect of the present invention provides a use of a compound of Chemical Formula 1, a compound of Chemical Formula 2, or a compound of Chemical Formula 3, for use in inducing transdifferentiation.

In addition, yet another aspect of the present invention provides a use for preventing or treating nerve injury diseases by stem cells treated with at least one selected from the group consisting of a compound of Chemical Formula 1, a compound of Chemical Formula 2, and a compound of Chemical Formula 3.

In addition, yet another aspect of the present invention provides a method for treating nerve injury diseases, including transplanting stem cells treated with at least one selected from the group consisting of a compound of Chemical Formula 1, a compound of Chemical Formula 2, and a compound of Chemical Formula 3 into a subject suffering from a nerve injury disease.

[Advantageous Effects]

According to the present invention, the compounds of the present invention can induce transdifferentiation of adult stem cells into neural progenitor cells by regulating the oligomerization of CtBPs. While ciNSC5 derived from transdifferentiation does not express neural stem cell markers or mature neuronal markers, the expression of intermediate neural progenitors or immature neuronal marker proteins was significantly increased compared to adult stem cells, the secretion of secretory proteins, including growth factors, tissue degradation factors, ligands involved in neural differentiation, neurogenesis, and axon regeneration, and cytokines, was increased, and the expression of enzymes for neurogenesis, neuranagenesis, neuroprotection, glial scar degradation, and neurogenesis-related factors was increased. Since ciNSC5 has the ability to differentiate into dopaminergic neurons or neurons, the compounds of the present invention can be used to induce oligomerization or transdifferentiation of CtBPs, and exhibits remarkable therapeutic effects in an ALS mouse model, an MS mouse model, a PD rat model, and a chronic spinal cord injury rat model, and thus the stem cells treated with the compound of the present invention can be used as a cellular therapeutic agent.

[Description of Drawings]

FIG. 1 is a diagram showing results of Drug Affinity Responsive Target Stability (DARTS) analysis of YJ101, YJ102, and YJ103.

FIG. 2 is a diagram confirming the regulation patterns of CTBP1 oligomerization in YJ102 using immunoprecipitation.

FIG. 3 shows molecular docking results of CtBP1 and YJ101, YJ102, and YJ 103.

FIG. 4 is a diagram confirming target genes of CtBP1-YJ102 by a ChIP method.

FIG. 5 is a diagram confirming CtBP1-mediated ciNSC5 differentiation by YJ102.

FIG. 6 is a diagram confirming transdifferentiation of human UCB-MSC treated with YJ101, YJ102, or YJ103.

FIG. 7 is a diagram confirming whether mesenchymal stem cells derived from various origins are transdifferentiated by YJ102.

FIG. 8 is a diagram confirming the cytotoxicity of YJ101, YJ102, and YJ103 to UCB-MSC.

FIG. 9 is a diagram confirming effects of YJ101, YJ102, and YJ103 on cell proliferation of UCB-MSC.

FIG. 10 is a diagram confirming the expression of neuronal markers in ciNSC5 treated with YJ102 for 5 days by immunofluorescence assay.

FIG. 11 is a diagram confirming the expression of neural progenitor cell marker proteins in ciNSC5 treated with YJ102 for 5 days by Western blot assay.

FIG. 12 is a diagram confirming the expression of MSC cell surface antigen marker proteins in ciNSC5 treated with YJ102 for 5 days by Western blot assay.

FIG. 13 is a diagram summarizing rates of change of the Western blot assay results of FIGS. 11 and 12.

FIG. 14 is a diagram confirming the growth factors, and tissue degradation and neural differentiation/regeneration-related factors increased in ciNSC5 treated with YJ102 for 5 days.

FIG. 15 is a diagram summarizing the results of FIG. 14 by change pattern.

FIG. 16 is a diagram showing growth factors, and tissue degradation and neural differentiation/regeneration-related proteins in a medium by Western blot after subculturing ciNSC5 differentiated from UCB-MSCs by treatment of YJ102 in an YJ102-free medium for 2 days.

FIG. 17 is a diagram summarizing the results of FIG. 16 by change pattern.

FIG. 18 is a diagram showing growth factors, and tissue degradation and neural differentiation/regeneration-related proteins in a medium by Western blot after further culturing ciNSC5 differentiated from UCB-MSCs by treatment of YJ102 in an YJ102-free medium for 4 or 7 days.

FIG. 19 is a diagram summarizing the results of FIG. 18 by change pattern.

FIG. 20 shows results of measuring the amounts of growth factors and six factors related to tissue degradation increased in ciNSC5 in the corresponding medium by ELISA, after culturing ciNSC5 in an YJ102-free medium for 2 days.

FIG. 21 is a diagram showing ELISA result values and increased ratios in a ciNSC5 medium compared to amounts confirmed in an MSC medium.

FIG. 22 is a heat map showing results of analyzing genes expressed in ciNSC5 by RNA-seq.

FIG. 23 is a diagram showing the selection and analysis of genes involved in neurogenesis, synapse, neurotransmitters, nerve growth factors, etc. among factors whose expression changes in the medium on day 5 based on RNA-seq results.

FIG. 24 is a table showing gene ontology analysis results of FIG. 23.

FIG. 25 is a diagram analyzing the expression of enzymes for glial scar degradation in ciNSC5 differentiated from UCB-MSC treated with YJ102 for 5 days by real-time RT-PCR.

FIG. 26 is a diagram analyzing the expression of transcription factors related to neurogenesis in ciNSC5 differentiated from UCB-MSC treated with YJ102 for 5 days by real-time RT-PCR.

FIG. 27 is a diagram analyzing the expression of neuronal markers such as myelination, synapsis, matrix/cell adhesion, and calcium signaling in ciNSC5 differentiated from UCB-MSC treated with YJ102 for 5 days by real-time RT-PCR.

FIG. 28 is a diagram analyzing the expression of neural receptors and channels in ciNSC5 differentiated from UCB-MSC treated with YJ102 for 5 days by real-time RT-PCR.

FIG. 29 is a diagram confirming changes in cell characteristics during subculture of ciNSC5 by doubling time for each passage.

FIG. 30 is a diagram confirming changes in cell characteristics during subculture of ciNSC5 with CPDL values according to the passage number.

FIG. 31 is a diagram confirming Tuj1 expression of ciNSC5 during subculture.

FIG. 32 is a diagram confirming chromosomal abnormalities during subculture of ciNSC5 through karyotyping.

FIG. 33 is a diagram confirming the differentiation of ciNSC5 into neurons.

FIG. 34 is a diagram confirming adipogenesis during MDI treatment by the expression of its markers, C/EBP$\alpha$ and PPAR$\gamma$.

FIG. 35 is a diagram confirming the lifespan of an ALS mouse model after ciNSC5 transplantation.

FIG. 36 is a diagram showing behavioral analysis (rota rod, motor score, hanging wire test, and balance beam test) of an ALS mouse model after ciNSC5 transplantation.

FIG. 37 is a diagram confirming lumbal neuron loss in an ALS mouse model after ciNSC5 transplantation.

FIG. 38 is a diagram showing behavior analysis of an EAE mouse model after ciNSC5 transplantation.

FIG. 39 is a diagram confirming the range and degree of demyelination in an EAE mouse model after ciNSC5 transplantation.

FIG. 40 is a diagram confirming mononuclear cells infiltrated around the spinal cord white matter of an EAE mouse model after ciNSC5 transplantation.

FIG. 41 is a diagram confirming macrophages infiltrated around the spinal cord white matter of an EAE mouse model after ciNSC5 transplantation.

FIGS. 42 and 43 are diagrams confirming the activation of microglia and astrocytes in the spinal cord of an EAE mouse model after ciNSC5 transplantation.

FIG. 44 shows a process of fabricating a PD rat model through 6-OHDA injection and a location of stereotaxic (Partial injury: AP +0.7, ML +2.6, DV –4.5 from bregma; and Complete injury: AP −2.2 , ML+1.5, DV –8.0 from bregma).

FIG. 45 is a diagram confirming motor function recovery in a PD rat model after ciNSC5 transplantation by behavioral analysis (rotation test and stepping test).

FIG. 46 is a diagram confirming the loss of dopaminergic neurons in a PD rat model after ciNSC5 transplantation.

FIG. 47 is a diagram confirming transplantation with a human-specific antibody (STEM121) in a PD rat model after ciNSC5 transplantation.

FIG. 48 is a diagram confirming that ciNSCs are differentiated into human dopaminergic neurons after transplantation by confirming human-specific antibody (STEM121) and dopaminergic neurons (TH-positive cells) in an area where cells are transplanted into the PD rat model after ciNSC5 transplantation.

FIG. 49 is a diagram confirming motor function recovery in a chronic spinal cord injury rat model after ciNSC5 transplantation through behavioral analysis (BBB, grid walk, and footprint).

FIG. 50 is a diagram analyzing motor function recovery in a chronic spinal cord injury rat model after ciNSC5 transplantation by FRI.

FIG. 51 is a diagram confirming lesion volume and myelin loss in a chronic spinal cord injury rat model after ciNSC5 transplantation.

FIG. 52 is a diagram confirming the degree of gliotic scar formation in the lesion site in a chronic spinal cord injury rat model after ciNSC5 transplantation.

FIG. 53 is a diagram confirming axon regeneration in a chronic spinal cord injury rat model after ciNSC5 transplantation by BDA anterograde axon tracing.

FIG. 54 is a diagram confirming axon regeneration in a chronic spinal cord injury rat model after ciNSC5 transplantation by Fluorogold retrograde axon tracing.

FIG. 55 is a diagram confirming the expression pattern of an axon regeneration factor GAP43 in a chronic spinal cord injury rat model after ciNSC5 transplantation by Western blot.

FIG. 56 is a diagram showing immunohistochemical staining using a human-specific antibody STEM121 and a neuronal marker, performed to confirm whether cells have been differentiated into mature neurons 6 weeks after transplantation into a chronic spinal cord injury rat model after ciNSC5 transplantation.

[Best Mode of the Invention]

Hereinafter, embodiments of the present invention will be described in detail with reference to the accompanying drawings. However, the following embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Further, terminologies used herein are terminologies used to properly express preferred examples of the present invention, which may vary according to a user, an operator's intention, or customs in the art to which the present invention pertains. Therefore, these terminologies used herein will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present invention, unless otherwise defined, have the meaning as commonly understood by those skilled in the related art of the present invention. In addition, although preferred methods and samples are described in the present specification, similar or equivalent methods and samples thereto are also included in the scope of the present invention. The contents of all publications disclosed as references in this specification are incorporated in the present invention.

Throughout this specification, '%' used to indicate the concentration of a specific material is solid/solid (w/w)%, solid/liquid (w/v)%, and liquid/liquid (v/v)%, unless otherwise stated.

In one aspect, the present invention relates to a benzoxazole derivative represented by Chemical Formula 4 below, a stereoisomer thereof, a pharmaceutically acceptable salt thereof, or a solvate or hydrate thereof:

【Chemical Formula 4】

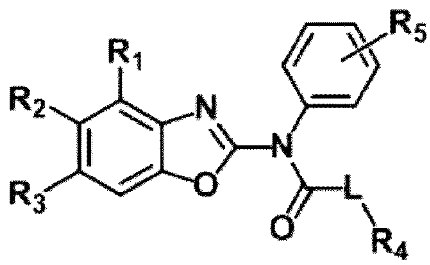

In Chemical Formula 4,

$R_1$ to $R_3$ are each independently hydrogen; a halogen group; a $C_1$-$C_4$ straight or branched alkyl group; a $C_1$-$C_4$ straight or branched alkoxy group; a nitro group ($-NO_2$); or an amino group ($-NH_2$),
L is a $C_1$-$C_4$ alkylene group,
$R_4$ is a $C_1$-$C_4$ straight or branched alkoxy group; $-C(=O)OR_6$; or $-OC(=O)R_7$,
$R_5$ is hydrogen; a halogen group; a straight or branched C1-C4 alkyl group; or a straight or branched C1-C4 alkoxy group, and
$R_6$ and $R_7$ are each independently a straight or branched C1-C4 alkyl group.

In the present invention, the stereoisomer is an isomer that occurs when the arrangement of atoms or atomic groups within the molecule is spatially varied, and includes both enantiomer and geometric isomer.

In the present invention, the hydrate is produced when a benzoxazole derivative, a pharmaceutically acceptable salt thereof, or a stereoisomer thereof is bound with water by non-covalent intermolecular forces, and may include a stoichiometric or non-stoichiometric amount of water. Specifically, the hydrate may include water in a ratio of about 0.25 mole to about 10 mole based on 1 mole of the active ingredient, and more specifically, about 0.5 mole, about 1 mole, about 1.5 mole, about 2 mole, about 2.5 moles, about 3 moles, about 5 moles, etc.

In the present specification, the 'solvate' is produced when a benzoxazole derivative, a pharmaceutically acceptable salt thereof, or a stereoisomer thereof is bound with a solvent other than water by intermolecular forces, and may include a stoichiometric or non-stoichiometric amount of solvent. Specifically, the solvate may include solvent molecules in a ratio of about 0.25 mole to about 10 mole based on 1 mole of the active ingredient, and more specifically, about 0.5 mole, about 1 mole, about 1.5 mole, about 2 mole, about 2.5 moles, about 3 moles, about 5 moles, etc.

In one embodiment, the benzoxazole derivative represented by Chemical Formula 4 may be a benzoxazole derivative represented by Chemical Formula 5 below, a stereoisomer thereof, a pharmaceutically acceptable salt thereof, or a solvent or hydrate thereof:

【Chemical Formula 5】

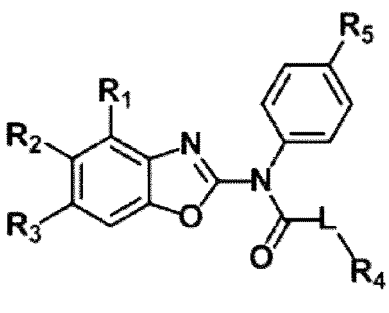

In Chemical Formula 5,
The definition of L and $R_1$ to $R_5$ are the same as those defined in Chemical Formula 4 above.

In one embodiment, two of $R_1$ to $R_3$ are hydrogen, and the other one may be hydrogen; a halogen group; a straight or branched $C_1$-$C_4$ alkyl group; a straight or branched C1-C4 alkoxy group; a nitro group ($-NO_2$); or an amino group ($-NH_2$).

In examples of the present invention, the benzoxazole derivative represented by Chemical Formula 4 may be any one of the compounds of Chemical Formula 1, Chemical Formula 2, or Chemical Formula 3 below:

【Chemical Formula 1】

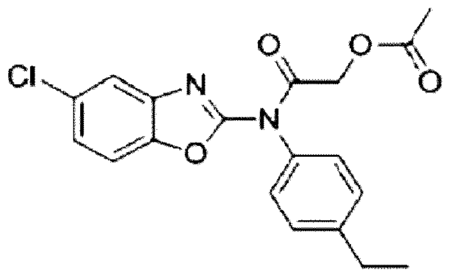

;

【Chemical Formula 2】

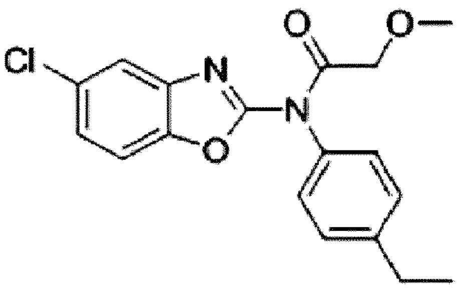

; and

【Chemical Formula 3】

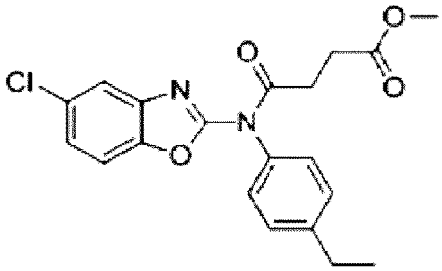

.

In an aspect, the present invention provides a composition for inducing oligomerization of C-terminal Binding Proteins (CtBPs), including at least one selected from the group consisting of a compound of Chemical Formula 1, a compound of Chemical Formula 2, and a compound of Chemical Formula 3.

In one embodiment, the CtBPs may be CtBP1 or CtBP2.

In one embodiment, the composition may protect CtBPs from enzymes to induce oligomerization.

In one embodiment, the oligomerization may be homooligomerization of CtBP1-CtBP1 or CtBP2-CtBP2.

In one embodiment, the compound of Chemical Formula 1, the compound of Chemical Formula 2, and the compound of Chemical Formula 3 may directly bind to CtBP1, and the compound of Chemical Formula 2 may also directly bind to CtBP2.

In one embodiment, the compound of Chemical Formula 1 may bind to Ser100 of CtBP1, the compound of

Chemical Formula 2 may bind to Ser100 and Arg266 of CtBP1, and the compound of Chemical Formula 3 may bind to Phe102, Arg184, and His236 of CtBP1.

In one embodiment, the composition may decrease the expression of HES1.

In one embodiment, the composition may increase the expression of Sox2.

In one embodiment, the composition may induce the expression of OCT4.

In one embodiment, the composition may differentiate adult stem cells into neural progenitor cells through CtBPs-mediation.

In one embodiment, the adult stem cells may be mesenchymal stem cells (MSC), and the mesenchymal stem cells may be umbilical cord blood-derived mesenchymal stem cells (UCB-MSC), umbilical cord-derived mesenchymal stem cells (UC-MSC), adipose-derived mesenchymal stem cells (AD-MSC) or bone marrow-derived mesenchymal stem cells (BM-MSC).

In one embodiment, the neural progenitor cell may be chemical induced neural stem cell 5 (ciNSC5).

In one embodiment, the composition may be a reagent composition or a medium composition.

In another aspect, the present invention provides a composition for inducing transdifferentiation of adult stem cells into neural progenitor cells, including at least one selected from the group consisting of a compound of Chemical Formula 1, a compound of Chemical Formula 2, and a compound of Chemical Formula 3.

In one embodiment, the composition may induce transdifferentiation into neural progenitor cells by CtBPs-mediation by inducing oligomers of CtBPs in adult stem cells.

In one embodiment, the adult stem cells may be mesenchymal stem cells, and the mesenchymal stem cells may be umbilical cord blood-derived mesenchymal stem cells, adipose-derived mesenchymal stem cells, or bone marrow-derived mesenchymal stem cells, and any adult stem cells may be applied regardless of specific tissue cells, but are not limited thereto. In many specific examples of the present invention, the umbilical cord blood-derived mesenchymal stem cells were used, but it was confirmed that transdifferentiation from adipose-derived mesenchymal stem cells and bone marrow-derived mesenchymal stem cells to neural progenitor cells was possible.

In one embodiment, the neural progenitor cells may be ciNSC5.

In one embodiment, the composition may increase the expression of a neuronal marker, and the neuronal marker may be Tuj1, TBR2, MASH1, GAP-43, or p75.

In one embodiment, the composition may increase the expression of neural progenitor cell marker proteins, and the neural progenitor cell marker protein may be Tuj 1 protein, CD325 (N-cadherin) protein, p75 protein, GAP-43 protein, CD54 protein, CD309 protein, CD56 (NCAM) protein, PSA-NCAM protein, CD29 protein, MASH1 protein or TBR2 protein. After induction of transdifferentiation compared to before induction of transdifferentiation, the composition may increase the expression of Tuj 1 protein 6-fold or more, preferably 6 to 8-fold, CD325 (N-cadherin) protein 7-fold or more, preferably 7 to 10-fold, p75 protein 4-fold or more, preferably 4 to 8-fold, GAP-43 protein 4-fold or more, preferably 4 to 7-fold, CD54 protein 2-fold or more, preferably 2 to 5-fold, CD309 protein 3-fold or more, preferably 3 to 7-fold, CD56 (NCAM) protein 3.5-fold or more, preferably 3.5 to 6-fold, PSA-NCAM protein 4-fold or more, preferably 4 to 6-fold, CD29 protein 4.5-fold or more, preferably 4.5 to 8-fold, MASH1 protein 4-fold or more, preferably 4 to 8-fold or TBR2 protein 3-fold or more, preferably 3 to 7-fold.

In one embodiment, the composition may decrease the expression of adult stem cell marker proteins, and the adult stem cell marker protein may be CD44, CD73, or CD105, and after induction of transdifferentiation compared to before induction of transdifferentiation, the composition may decrease the expression of CD44 protein by 50 to 90%, CD73 protein by 30 to 70%, or CD105 protein by 50 to 90%.

In one embodiment, the composition may increase the secretion of secretory proteins, and the secretory protein may be a growth factor, a tissue degradation factor, a ligand, or a cytokine.

In one embodiment, the growth factor may be PIGF, NGF, BDNF, or VEGFA, and after induction of transdifferentiation compared to before induction of transdifferentiation, the composition may increase secretion of PIGF 1.5 to 4-fold, NGF 1.5 to 6-fold, BDNF 1.5 to 3-fold or VEGFA 1.5 to 5-fold.

In one embodiment, the tissue degradation factor may be MMP1, MMP2, MMP7, or TIMP2, and after induction of transdifferentiation compared to before induction of transdifferentiation, the composition may increase secretion of MMP1 1.5 to 3.5-fold or MMP2 2 to 6-fold.

In one embodiment, the ligand may be SHH or Notch 1.

In one embodiment, the cytokine may be TNFSF12 or IL-16, and after induction of transdifferentiation compared to before induction of transdifferentiation, the composition may increase secretion of TNFSF12 1.5 to 4-fold.

In one embodiment, the composition may be a reagent composition or a medium composition.

In the present invention, the expression level of mRNA of the marker gene may be measured by polymerase chain reaction (PCR), real-time RT-PCR, reverse transcription PCR, competitive RT-PCR, nuclease protection analysis (RNase, S1 nuclease assay), in situ hybridization, nucleic acid microarray, Northern blot, or DNA chip method using a primer pair, a probe, or a primer pair and a probe that specifically recognizes a nucleic acid sequence of the marker, a nucleic acid sequence complementary to the nucleic acid sequence, and the nucleic acid sequence and a fragment of

the complementary sequence. The protein expression level of the marker may be measured by Western blot, enzyme linked immunosorbent assay (ELISA), radioimmunoassay (RIA), radioimmunodiffusion, immunoelectrophoresis, tissue immunostaining, immunoprecipitation assay, complement fixation assay, FACS, mass spectrometry, or protein microarray using antibodies, antibody fragments, aptamers, avidity multimers, or peptidomimetics that specifically recognize a full-length protein of the marker or fragments thereof.

As used in the present invention, the term "transdifferentiation (direct reprogramming, direct conversion) is a process of inducing conversion between mature (completely differentiated) cells having completely different cell types in higher organisms and a technology of converting 'directly' a desired specific cell (cell type B) without a process of dedifferentiating and then re-differentiating any somatic cells (cell type A) into induced pluripotent stem cells capable of differentiating into all cells. Currently, transdifferentiation is recognized for its potential use in disease modeling and new drug discovery, and is expected to be applied to gene therapy, regenerative medicine, etc. in the future.

In an aspect, the present invention provides a method for inducing oligomerization of CtBPs, including treating a sample *in vitro* with at least one selected from the group consisting of a compound of Chemical Formula 1, a compound of Chemical Formula 2, and a compound of Chemical Formula 3.

In one embodiment, the compound may be treated for 3 to 7 days, more preferably for 5 days.

In one embodiment, the CtBPs may be CtBP1 or CtBP2.

In one embodiment, the oligomerization may be homooligomerization of CtBP1-CtBP1 or CtBP2-CtBP2.

In one embodiment, the sample may be adult stem cells.

In one embodiment, the adult stem cells may be mesenchymal stem cells, and the mesenchymal stem cells may be umbilical cord blood-derived mesenchymal stem cells (UCB-MSC), umbilical cord-derived mesenchymal stem cells, adipose-derived mesenchymal stem cells, or bone marrow-derived mesenchymal stem cells.

In one embodiment, the method may increase the expression of Tuj1, CD325 (N-cadherin), p75, GAP-43, CD309, CD56 (NCAM), PSA-NCAM, CD29, MASH1 or TBR2 in the sample after treatment compared to before treatment with the compound, and may increase the expression of Tuj 1 6-fold or more, preferably 6 to 8-fold, CD325 (N-cadherin) 7-fold or more, preferably 7 to 10-fold, p75 4-fold or more, preferably 4 to 8-fold, GAP-43 4-fold or more, preferably 4 to 7-fold, CD54 2-fold or more, preferably 2 to 5-fold, CD309 3-fold or more, preferably 3 to 7-fold, CD56 (NCAM) 3.5-fold or more, preferably 3.5 to 6-fold, PSA-NCAM 4-fold or more, preferably 4 to 6-fold, CD29 4.5-fold or more, preferably 4.5 to 8-fold, MASH1 4-fold or more, preferably 4 to 8-fold or TBR2 3-fold or more, preferably 3 to 7-fold.

In one embodiment, the method may increase the secretion of growth factors, tissue degradation factors, ligands or cytokines in the sample after treatment compared to before treatment with the compound, increase the secretion of PIGF, NGF, BDNF, VEGFA, MMP1, MMP2, MMP7, TIMP2, SHH, Notch1, TNFSF12 or IL-16 in the sample after treatment compared to before treatment with the compound, and increase the secretion of PIGF 1.5 to 4-fold, NGF 1.5 to 6-fold, BDNF 1.5 to 3-fold, VEGFA 1.5 to 5-fold, MMP1 1.5 to 3.5-fold, MMP2 2 to 6-fold, or TNFSF12 1.5 to 4-fold. The method may decrease the expression of CD44, CD73 or CD 105 in the sample after treatment compared to before treatment with the compound, and may decrease the expression of CD44 by 50 to 90%, the expression of CD73 by 30 to 70%, or the expression of CD105 by 50 to 90%.

In an aspect, the present invention provides a method for inducing transdifferentiation of adult stem cells into neural progenitor cells, including treating adult stem cells with at least one selected from the group consisting of a compound of Chemical Formula 1, a compound of Chemical Formula 2, and a compound of Chemical Formula 3 *in vitro*.

In one embodiment, the compound may be treated for 3 to 7 days, most preferably for 5 days.

In one embodiment, transdifferentiation may be induced by CtBPs-mediation, and more preferably, transdifferentiation is induced through oligomerization of CtBPs.

In one embodiment, the method may increase the expression of Tuj1, CD325 (N-cadherin), p75, GAP-43, CD309, CD56 (NCAM), PSA-NCAM, CD29, MASH1 or TBR2 in cells after treatment compared to before treatment with the compound, and may increase the expression of Tuj1 2 to 5-fold, CD325 (N-cadherin) 3 to 6-fold, p75 1.5 to 4-fold, GAP43 2 to 6-fold, CD309 1.5 to 3-fold, CD56 (NCAM) 1.5 to 4.5-fold, PSA-NCAM 1.5 to 4-fold, CD29 1.5 to 4-fold, MASH 2 to 4-fold, or TBR2 5 to 9-fold.

In one embodiment, the method may increase the secretion of growth factors, tissue degradation factors, ligands, or cytokines from cells after treatment compared to before treatment with the compound.

In one embodiment, the method may increase the secretion of PIGF, NGF, BDNF, VEGFA, MMP1, MMP2, MMP7, TIMP2, SHH, Notch1, TNFSF12 or IL-16 from the cells after treatment compared to before treatment with the compound, and increase the secretion of PIGF 1.5 to 4-fold, NGF 1.5 to 6-fold, BDNF 1.5 to 3-fold, VEGFA 1.5 to 5-fold, MMP1 1.5 to 3.5-fold, MMP2 2 to 6-fold, or TNFSF12 1.5 to 4-fold.

In an embodiment, the method may decrease the expression of CD44, CD73, or CD105 from the cells after treatment compared to before treatment with the compound, and decrease the expression of CD44 by 50 to 90%, the expression of CD73 by 30 to 70%, or the expression of CD105 by 40 to 80%.

By transdifferentiation induced by the composition for inducing transdifferentiation and the transdifferentiation method of the present invention, direct differentiation of adult stem cells into neural progenitor cells may be induced, in

which the neural progenitor cells may be differentiated again into neurons and/or dopaminergic neurons. In the case, the transdifferentiation method may further include differentiating neural progenitor cells obtained by introducing adult stem cells with at least one selected from the group consisting of the compound of Chemical Formula 1, the compound of Chemical Formula 2, and the compound of Chemical Formula 3 of the present invention described above, or transdifferentiated composition containing the same into neurons or dopaminergic neurons, and at this time, the differentiating of mesenchymal stem cells into neurons or dopaminergic neurons may be performed by applying general differentiation techniques.

In an aspect, the present invention provides stem cells treated with at least one compound selected from the group consisting of a compound of Chemical Formula 1, a compound of Chemical Formula 2, and a compound of Chemical Formula 3.

In one embodiment, the stem cells treated with the compound may be adult stem cells, and the adult stem cells may be mesenchymal stem cells (MSC), and the mesenchymal stem cells may be umbilical cord blood-derived mesenchymal stem cells (UCB-MSC), umbilical cord-derived mesenchymal stem cells (UC-MSC), adipose-derived mesenchymal stem cells (AD-MSC) or bone marrow-derived mesenchymal stem cells (BM-MSC).

In one embodiment, the stem cells of the present invention may be stem cells treated with at least one selected from the group consisting of a compound of Chemical Formula 1, a compound of Chemical Formula 2, and a compound of Chemical Formula *3 in vitro* for 3 to 7 days, and stem cells prepared by the method including treating the stem cells with at least one compound selected from the group consisting of a compound of Chemical Formula 1, a compound of Chemical Formula 2, and a compound of Chemical Formula 3; and culturing the stem cells treated with the compound for 3 to 7 days.

In one embodiment, the stem cells of the present invention may be stem cells treated with 30 mM of the compound dissolved in DMSO at a concentration of 5 to 100 $\mu$M.

In one embodiment, the stem cells of the present invention may be neural progenitor cells that transdifferentiate (directly convert) from adult stem cells by treatment with the compound, and the neural progenitor cell may be chemical induced neural stem cell 5 (ciNSC5).

In one embodiment, the stem cells of the present invention may increase the expression of a neuronal marker compared to stem cells untreated with the compound, and the neuronal marker may be Tuj1, TBR2, MASH1, GAP-43, or p75.

In one embodiment, the stem cells of the present invention may increase the expression of neural progenitor cell marker proteins compared to stem cells untreated with the compound, and the neural progenitor cell marker protein may be Tuj 1 protein, CD325 (N-cadherin) protein, p75 protein, GAP-43 protein, CD54 protein, CD309 protein, CD56 (NCAM) protein, PSA-NCAM protein, CD29 protein, MASH1 protein or TBR2 protein.

In one embodiment, compared to stem cells untreated with the compound, the stem cells of the present invention may increase the expression of Tuj1 protein 6-fold or more, preferably 6 to 8-fold, CD325 (N-cadherin) protein 7-fold or more, preferably 7 to 10-fold, p75 protein 4-fold or more, preferably 4 to 8-fold, GAP-43 protein 4-fold or more, preferably 4 to 7-fold, CD54 protein 2-fold or more, preferably 2 to 5-fold, CD309 protein 3-fold or more, preferably 3 to 7-fold, CD56 (NCAM) protein 3.5-fold or more, preferably 3.5 to 6-fold, PSA-NCAM protein 4-fold or more, preferably 4 to 6-fold, CD29 protein 4.5-fold or more, preferably 4.5 to 8-fold, MASH1 protein 4-fold or more, preferably 4 to 8-fold or TBR2 protein 3-fold or more, preferably 3 to 7-fold.

In one embodiment, compared to stem cells untreated with the compound, the stem cells of the present invention may decrease the expression of CD44, CD73 or CD105, and may decrease the expression of CD44 protein by 50 to 90%, the expression of CD73 protein by 30 to 70%, or the expression of CD105 protein by 50 to 90%.

In one embodiment, compared to stem cells untreated with the compound, the stem cells of the present invention may increase the secretion of secretory proteins, and the secretory protein may be a growth factor, a tissue degradation factor, a ligand, or a cytokine.

In one embodiment, the growth factor may be PIGF, NGF, BDNF, or VEGFA, and the stem cells of the present invention may increase the secretion of PIGF 1.5 to 4-fold, the secretion of NGF 1.5 to 6-fold, the secretion of BDNF 1.5 to 3-fold or the secretion of VEGFA 1.5 to 5-fold, compared to stem cells untreated with the compound.

In one embodiment, the tissue degradation factor may be MMP1, MMP2, MMP7, or TIMP2, and the stem cells of the present invention may increase the secretion of MMP1 1.5 to 3.5-fold or secretion of MMP2 2 to 6-fold compared to stem cells untreated with the compound.

In one embodiment, the ligand may be SHH or Notch1.

In one embodiment, the cytokine may be TNFSF12 or IL-16, and the stem cells of the present invention may increase the secretion of TNFSF12 1.5 to 4-fold compared to stem cells untreated with the compound.

In one embodiment, the stem cells of the present invention may increase the expression of HES1, Sox2, or OCT4 compared to stem cells untreated with the compound.

In one embodiment, the stem cells of the present invention may induce oligomerization of C-terminal Binding Proteins (CtBPs) by treatment with the compound, and the compound may protect CtBPs from enzymes to induce

oligomerization.

In one embodiment, the CtBPs may be CtBP1 or CtBP2.

In one embodiment, the oligomerization may be homooligomerization of CtBP1-CtBP1 or CtBP2-CtBP2.

In one embodiment, the compound of Chemical Formula 1, the compound of Chemical Formula 2, and the compound of Chemical Formula 3 may directly bind to CtBP1, and the compound of Chemical Formula 2 may also directly bind to CtBP2.

In one embodiment, the compound of Chemical Formula 1 may bind to Ser100 of CtBP1, the compound of Chemical Formula 2 may bind to Ser100 and Arg266 of CtBP1, and the compound of Chemical Formula 3 may bind to Phe102, Arg184, and His236 of CtBP1.

In one embodiment, the compound may differentiate adult stem cells into neural progenitor cells through CtBPs-mediation.

In one embodiment, the compound may induce transdifferentiation into neural progenitor cells by CtBPs-mediation by inducing oligomers of CtBPs in adult stem cells.

In one embodiment, the adult stem cells may be mesenchymal stem cells, and the mesenchymal stem cells may be umbilical cord blood-derived mesenchymal stem cells (UCB-MSC), umbilical cord-derived mesenchymal stem cells, adipose-derived mesenchymal stem cells, or bone marrow-derived mesenchymal stem cells, and any adult stem cells may be applied regardless of specific tissue cells, but it is not limited thereto. In many specific examples of the present invention, the umbilical cord blood-derived mesenchymal stem cells were used, but it was confirmed that transdifferentiation from adipose-derived mesenchymal stem cells and bone marrow-derived mesenchymal stem cells to neural progenitor cells was possible.

In one embodiment, the stem cells of the present invention may not express MAP2, NSE, NeuN, doublecortin, Neurogenic differentiation 1 (NeuroD1), Nestin, Mussashi 1 or GFAP.

In one embodiment, as compared to stem cells untreated with the compound, the stem cells of the present invention may increase the expression of neurogenesisinducing genes, inducible neurotrophic factor secretion genes, neurogenesis-related genes, neuronal marker genes, neuron receptor genes, and channel-related genes.

In one embodiment, the stem cells of the present invention may increase the expression of enzymes for glial scar degradation as compared to stem cells untreated with the compound.

In one embodiment, the stem cells of the present invention may be capable of differentiating into dopaminergic neurons, neurons, or mature neurons.

In one embodiment, the stem cells of the present invention may not induce adipogenesis.

As used herein, the term "expression" refers to a process in which a nucleic acid is transcribed from a DNA template (e.g., into mRNA or other RNA transcripts) and/or a process in which transcribed mRNA is subsequently translated into a peptide, polypeptide, or protein.

In the present invention, the expression level of mRNA of the gene may be measured by polymerase chain reaction (PCR), real-time RT-PCR, reverse transcription PCR, competitive RT-PCR, nuclease protection analysis (RNase, S1 nuclease assay), in situ hybridization, nucleic acid microarray, Northern blot, or DNA chip method using a primer pair, a probe, or a primer pair and a probe that specifically recognizes a nucleic acid sequence of the marker, a nucleic acid sequence complementary to the nucleic acid sequence, and the nucleic acid sequence and a fragment of the complementary sequence. The protein expression level of the gene may be measured by Western blot, enzyme linked immunosorbent assay (ELISA), radioimmunoassay (RIA), radioimmunodiffusion, immunoelectrophoresis, tissue immunostaining, immunoprecipitation assay, complement fixation assay, FACS, mass spectrometry, or protein microarray using antibodies, antibody fragments, aptamers, avidity multimers, or peptidomimetics that specifically recognize a full-length protein or fragments thereof.

As used in the present invention, the term "transdifferentiation (direct reprogramming, direct conversion) is a process of inducing conversion between mature (completely differentiated) cells having completely different cell types in higher organisms and a technology of converting 'directly' a desired specific cell (cell type B) without a process of dedifferentiating and then re-differentiating any somatic cells (cell type A) into induced pluripotent stem cells capable of differentiating into all cells.

The stem cells treated with the compound of the present invention transdifferentiate into neural progenitor cells, which may again differentiate into mature neurons and/or dopaminergic neurons. In this case, the present invention may additionally include a component for differentiating stem cells treated with the compound into neurons or dopaminergic neurons, and in this case, the differentiating of the mesenchymal stem cells into neurons or dopaminergic neurons may be performed by applying general differentiation techniques.

In an aspect, the present invention provides a pharmaceutical composition for preventing or treating nerve injury diseases, including the stem cells of the present invention, a stem cell culture, or a suspension culture as an active ingredient.

In one embodiment, the nerve injury disease may be injury to the central or peripheral nervous system, or a neurodegenerative disease, or may be a central nervous system (CNS) injury disease.

In one embodiment, the injury to the central or peripheral nervous system may be spinal cord injury (SCI), traumatic brain injury (TBI), peripheral nerve injury, stroke, or brain cancer.

In one embodiment, the spinal cord injury may be caused by trauma or inflammation, and may be caused by at least one selected from the group consisting of acute transverse myelitis, acute disseminated myelitis, myelopathy, non-Hodgkin's lymphoma, hydrocephalus, hereditary ataxia, neurosyphilis, Minamata disease, Lou Gehrig's disease, and multiple sclerosis.

In one embodiment, the neurodegenerative disease may be Alzheimer's disease, Parkinson's disease, amyotrophic lateral sclerosis (ALS), Huntington's disease (HD), multiple sclerosis (MS), or multiple system atrophy, and more preferably Parkinson's disease, amyotrophic lateral sclerosis (ALS) or multiple sclerosis (MS).

In one embodiment, the composition of the present invention may achieve the effect of preventing or treating Lou Gehrig's disease by significantly inhibiting the death of motor neurons in the spinal cord, delaying the progression of Lou Gehrig's disease symptoms accompanied by motor function impairment, and extending lifespan.

In one embodiment, the composition of the present invention may achieve the effect of preventing or treating multiple sclerosis (MS) by inhibiting demyelination of CNS, inhibiting the infiltration of monocytes or macrophages into the spinal cord white matter, and inhibiting the activation of microglia or astrocytes.

In one embodiment, the composition of the present invention may achieve the effect of preventing or treating Parkinson's disease (PD) by inhibiting the loss of dopaminergic neurons and differentiating the transplanted stem cells of the present invention into dopaminergic neurons.

In one embodiment, the composition of the present invention may achieve the effect of preventing or treating chronic spinal cord injury by reducing myelin loss, reducing gliotic scar formation, increasing axons, and differentiating the transplanted stem cells of the present invention into mature nerve cells.

As used in the present invention, the term "prevention" refers to all actions that inhibit or delay the occurrence, development, and recurrence of the nerve injury disease by administration of the composition according to the present invention.

As used in the present invention, the term "treatment" means all actions that improve or beneficially change the symptoms of the nerve injury disease and its complications by administration of the composition according to the present invention. Those skilled in the art to which the present invention pertains will be able to determine the degree of improvement, enhancement and treatment by knowing the exact criteria of a disease for which the composition of the present invention is effective by referring to data presented by the Korean Academy of Medical Sciences, etc.

As used in the present specification, the term "treatment" refers to an approach for obtaining beneficial or desirable clinical results. For the purposes of the present invention, beneficial or desirable clinical results include non-restrictively palliation of symptoms, reduction of a disease range, stabilization (i.e., not worsening) of a disease condition, delay or reduced rate of disease progression, improvement or temporary palliation and reduction (partially or entirely) of a disease condition, and whether it is detectable or undetectable.

In addition, the "treatment" may also mean increasing survival rate compared to expected survival rate without treatment. The "treatment" refers to all therapeutic treatment and prophylactic or preventive measures. The treatments include the treatment required for disorders that have already occurred as well as disorders to be prevented. The "palliating" of the disease means that the range of the disease condition and/or undesirable clinical symptoms are reduced and/or a time course of progression is delayed or lengthened, compared to no treatment.

The therapeutically effective amount of the composition of the present invention may vary depending on many factors, for example, an administration method, a target site, a condition of a subject, and the like.

The pharmaceutical composition of the present invention is administered in a pharmaceutically effective amount. As used in the present invention, the term "pharmaceutically effective amount" refers to an amount enough to treat the disease at a reasonable benefit/risk ratio applicable to medical treatment and enough not to cause side effects. The effective dose level may be determined according to factors including the health condition of a subject, the type and severity of nerve injury diseases, the activity of a drug, the sensitivity to a drug, an administration method, a time of administration, a route of administration, an excretion rate, duration of treatment, and drugs used in combination or simultaneously, and other factors well-known in the medical field. The composition of the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

Unless otherwise stated, the 'treatment' means reversing, palliating, inhibiting the progression of, or preventing the disease or disorder to which the term is applied, or one or more symptoms of the disease or disorder, and as used herein, the term 'treatment' refers to any treating action when 'treating' is defined as above. Accordingly, treatment or therapy for autoimmune diseases in mammals may include one or more of the following:

(1) inhibiting the growth of nerve injury diseases, that is, arresting development thereof;

(2) preventing the spread of nerve injury diseases, i.e., preventing metastasis;
(3) alleviating nerve injury diseases;
(4) preventing recurrence of nerve injury diseases; and
(5) palliating symptoms of nerve injury diseases.

If a recipient animal is able to tolerate administration of the composition, or administration of the composition to the animal is suitable, it is indicated that the composition is "pharmaceutically or physiologically acceptable". It may be said that the agent has been administered in a "therapeutically effective amount" when the administered amount is physiologically significant. If the presence of the agent results in a physiologically detectable change in the recipient patient, the agent is physiologically significant.

As used in the present specification, the "effective amount" is an appropriate amount that affects a beneficial or desirable clinical or biochemical result. The effective amount may be administered once or more times. For the purposes of the present invention, an effective amount of an accelerator is an amount appropriate to temporarily palliate, ameliorate, stabilize, reverse, slow or delay the progression of the associated disease condition.

The therapeutically effective amount of the composition of the present invention may vary depending on many factors, for example, an administration method, a target site, a condition of a patient, and the like. Accordingly, when used in the human body, the dose should be determined as an appropriate amount in consideration of both safety and efficiency. It is also possible to estimate the amount used in humans from the effective amount determined through animal experiments. These matters to be considered when determining the effective amount are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective amount. As used in the present invention, the "pharmaceutically effective amount" refers to an amount enough to treat the disease at a reasonable benefit/risk ratio applicable to medical treatment and not to cause side effects. The effective dose level may be determined according to factors including the health condition of a patient, the type and severity of a disease, the activity of a drug, the sensitivity to a drug, an administration method, a time of administration, a route of administration, an excretion rate, duration of treatment, and drugs used in combination or simultaneously, and other factors well-known in the medical field. The composition of the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may include a carrier, diluent, excipient, or a combination of two or more thereof, which are commonly used in biological agents. As used in the present invention, the term "pharmaceutically acceptable" means that the composition exhibits non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these components, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

In one embodiment, the pharmaceutical composition may be one or more formulations selected from the group including oral formulations, external formulations, suppositories, sterile injection solutions and sprays.

The composition of the present invention may include a carrier, a diluent, an excipient, or a combination of two or more thereof, which are commonly used in biological agents. The pharmaceutically acceptable carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and one or more of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using a suitable method in the art or a method disclosed in Remington's Pharmaceutical Science (Mack Publishing Company, Easton PA, 18th, 1990).

The composition of the present invention may further contain one or more active ingredients exhibiting the

same or similar functions.

The pharmaceutical composition of the present invention may further include a pharmaceutically acceptable additive. At this time, the pharmaceutically acceptable additive may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc and the like. The pharmaceutically acceptable additive according to the present invention is preferably included in an amount of 0.1 part by weight to 90 parts by weight based on the composition, but is not limited thereto.

The composition of the present invention may be administered parenterally (e.g., applied intravenously, subcutaneously, intraperitoneally or topically) or orally according to a desired method, and the dose may vary depending on the weight, age, sex, and health condition of a patient, a diet, an administration time, an administration method, an excretion rate, the severity of a disease, etc. A daily dose of the composition according to the present invention is 0.0001 to 10 mg/ml, preferably 0.0001 to 5 mg/ml, and more preferably administered once to several times a day.

Liquid formulations for oral administration of the composition of the present invention correspond to suspensions, internal solutions, emulsions, syrups, etc., and may include various excipients, such as wetting agents, sweeteners, fragrances, preservatives, and the like, in addition to water and liquid paraffin, which are commonly used simple diluents. Formulations for parenteral administration include sterilized aqueous solutions, non-aqueous solvents, suspensions, emulsions, lyophilized agents, suppositories, and the like.

In an aspect, the present invention provides a use of a compound of Chemical Formula 1, a compound of Chemical Formula 2, or a compound of Chemical Formula 3, for use in inducing oligomerization of CtBPs.

In an aspect, the present invention provides a use of a compound of Chemical Formula 1, a compound of Chemical Formula 2, or a compound of Chemical Formula 3, for use in inducing transdifferentiation.

In an aspect, the present invention is to provide a use for preventing or treating nerve injury diseases by stem cells treated with at least one compound selected from the group consisting of a compound of Chemical Formula 1, a compound of Chemical Formula 2, and a compound of Chemical Formula 3.

In an aspect, the present invention provides a method for treating nerve injury diseases, including transplanting stem cells treated with at least one compound selected from the group consisting of a compound of Chemical Formula 1, a compound of Chemical Formula 2, and a compound of Chemical Formula 3 into a subject suffering from a nerve injury disease.

[Modes of the Invention]

Hereinafter, the present invention will be described in more detail with reference to the following Examples. However, the following Examples are only intended to embody the contents of the present invention, and the present invention is not limited thereto.

**Example 1. Regulation of CTBP1 oligomerization of compounds of the present invention**

1-1. Identification of YJ101, YJ102 and YJ103 binding proteins through DART assay

To identify proteins binding to compounds of the present invention, YJ101 (Chemical Formula 1), YJ102 (Chemical Formula 2), and YJ103 (Chemical Formula 3), in human umbilical cord blood-derived mesenchymal stem cells (UCB-MSCs), DART assay was performed. Specifically, UCB-MSCs (Kang Stem Cell, Passage # 6) were dispensed into a 100 mm culture dish and incubated in a KSB-3 medium (Cat. K3901, Kang Stem Cell) containing 10% FBS (Cat. 16000044), and then collected and washed with PBS. Thereafter, the cells were added with an IP50 lysis buffer [50 mM Tris-HCl (pH 7.4), 150 mM NaCl, 2 mM $MgCl_2$, and 0.1% NP40], sonicated, and centrifuged at 13,000 × g for 10 minutes at 4°C to obtain the supernatant. The supernatant was added with 1/10 volume of 10X TNC buffer [500 mM Tris-HCl (pH 8.0), 500 mM NaCl, and 100 mM $CaCl_2$], and protein concentration was measured using a Bradford method. Thereafter, 9 μl of a cell lysate and 1 μl of YJ101 (20 or 40 μM), YJ102 (20 or 30 μM), or YJ103 (20 or 40 μM) were mixed in a microcentrifuge tube and incubated at room temperature for 1 hour. A control group was treated with 1 μl of DMSO. After incubation, 1 μl (1 mg/ml) of Pronase (Cat. 10 165 921 001, Roche) diluted to 1 mg/ml using a 1xTNC buffer was added to the microcentrifuge tube and incubated at 37°C for 15 minutes. After incubation, 1/5 volume of a 5X SDS-PAGE loading buffer was added, heated at 100°C for 5 minutes, and then SDS-PAGE and Coomassie blue staining were performed. In addition, UCB-MSCs were dispensed at 4 × $10^5$ cells/ml in a 60 mm culture dish, and after 24 hours, 4 μg of FLAG-CtBP1 or FLAG-CtBP2 DNA was transfected using 4 μl of jetPRIME. After 48 hours, the cells were collected, and proteins were extracted and quantified using the same method as above. DARTS assay was performed with the cell lysate of CtBP1 and Y102 labeled with FLAG, and Western blot assay was performed with a flag

antibody (Cat. F3165, Sigma).

As a result, after DARTS assay, a band (*) protected by YJ102 was confirmed and identified as C-terminal Binding Proteins (CtBP1) (FIG. 1A). It was confirmed that CtBP1 was protected by YJ102 and was not degraded by pronase (FIG. 1B), overexpressed CtBP1 was protected by YJ102 and was not degraded by pronase (FIG. 1C), and overexpressed CtBP1 (flag-CtBP1) was protected by YJ101, YJ102, and YJ103 and was not degraded by pronase (FIG. 1D). In addition, it was confirmed that CtBP2 was also protected by YJ102 and was not degraded by pronase (FIG. 1E), and overexpressed CtBP2 was also protected by YJ102 and was not degraded by pronase (FIG. 1F).

As a result, it was confirmed that YJ101, YJ102, and YJ103 all bound to CtBP1, and YJ102 bound to CtBP1 and CtBP2 in human UCB-MSC.

1-2. Confirmation of regulation of CTBP1 oligomerization by YJ102

To determine the effect of YJ102 on the regulation of oligomerization of C-terminal Binding Proteins (CtBPs) in human UCB-MSCs, immunoprecipitation analysis was performed. Specifically, UCB-MSCs were dispensed at $4 \times 10^5$ cells/ml in a 60 mm culture dish, and the next day, FLAG-CtBP1, HA-CtBP1, FLAG-CtBP2, and HA-CtBP1 were transfected into cells using 4 µl jetPRIME. Thereafter, the cells were treated with YJ102 (stock 30 mM in DMSO) and MTOB (stock 300 mM in DMSO) at 20 µM and 300 µM, respectively (Table 1). After 48 hours of transfection, proteins were extracted in the same manner as in Example 1-1. Protein G agarose slurry was washed with PBS and blocked with 1 ml of 1% BSA, washed with PBS, and added with 1 µg of anti-HA antibody diluted with 1.5 ml of PBS and incubated at 4°C for 2 hours. Thereafter, the cells were centrifuged at 3,000 × g for 2 minutes to remove the supernatant, washed with an IP150 buffer two times, added with 1 µg of the protein extracted above, and incubated at 4°C overnight. After incubation, the cells were centrifuged at 3,000 × g for 2 minutes to remove the supernatant, washed with PBS three times, added with 50 µl of 1 mg/ml HA peptide diluted with PBS, and incubated at room temperature for 10 minutes. After the cells were centrifuged at 3,000 × g for 2 minutes, the supernatant was transferred to a new microcentrifuge tube, added with 10 µl of a 5X sample buffer, and then heated at 100°C for 5 minutes, and then Western blot assay was performed with FLAG antibody or HA antibody.

[Table 1]

| Group | FLAG-CtBP1 | HA-CtBP1 | FLAG-CtBP2 | HA-CtBP1 | Treatment |
|---|---|---|---|---|---|
| 1 | 2 µg | 2 µg | | | - |
| 2 | 2 µg | 2 µg | | | YJ102 (20 µM) |
| 3 | 2 µg | 2 µg | | | MTOB (300 µM) |
| 4 | | | 2 µg | 2 µg | - |
| 5 | | | 2 µg | 2 µg | YJ102 (20 µM) |
| 6 | | | 2 µg | 2 µg | MTOB (300 µM) |

As a result, it was shown that YJ102 induced oligomerization of CtBP1, and such oligomerization was inhibited by MTOB (FIG. 2). On the other hand, it was shown that YJ102 had no effect on CtBP1-CtBP2 heteromerization. It was known that MTOB was a substrate for dehydrogenease of CtBP and served as an inhibitor of inhibiting the transcriptional activity of CtBP at high concentration to inhibit oligomerization.

1-3. Identification of interaction sites between YJ101, YJ102 and YJ103 and CtBP1

Molecular docking was performed to identify sites where the compounds YJ101, YJ102 and YJ103 of the present invention interacted with CtBP1. Specifically, protein-ligand docking was performed using Glide (Grid based LIgand Docking with Energetics) docking application of the Schrodinger suites rogram Schrodinger LC, Y, SA), and the three-dimensional structures of CtBP1 protein (PDB ID: 6CDF) and YJ102 were prepared using Schrodinger sites. In addition, a receptor grid for CtBP1 was generated by specifying a binding (active) site residue and identified with SiteMap. After generating the receptor grid, the ligand was docked to CtBP1 using the Glide docking protocol, and a docked conformer was evaluated using the Glide (G) Score. G Score was calculated using Equation 1 below.

[Equation 1]

$$G\ Score = a \times vdW + b \times Coul + Lipo + Hbond + Metal + BuryP + RotB + Site$$

(vdW: van der Waals energy, Coul: Coulomb energy, Lipo: lipophilic contact, HBond: hydrogen-bonding, Metal: metal-binding, BuryP: penalty for buried polar groups, RotB: penalty for freezing rotatable bonds, Site: polar interactions in the active site, a = 0.065: coefficient of vdW, and b = 0.130: coefficient of Coul)

As a result, it was shown that YJ101, YJ102, and YJ103 all interacted with an NAD(H) binding domain of CtBP1, and ΔG values were - 4.980 kcal/mol for YJ101, - 5.458 kcal/mol for YJ102, and - 3.767 kcal/mol for YJ103 (FIG. 3A). In addition, it was expected that Ser100 of CTBP1 was important for the interaction between YJ101 and CTBP1, and Ser100 and Arg266 of CTBP1 were important for the interaction between YJ102 and CtBP1, and it was expected that Phe102, Arg184, and His236 of CTBP1 played an important role in the interaction between YJ103 and CTBP1 (FIG. 3B).

1-4. Identification of target gene of CTBP-YJ102

To identify a target gene of CtBP1-YJ102 in human UCB-MSC, chromatin immunoprecipitation (ChIP) assay was performed. Specifically, $2 \times 10^6$ human UCB-MSCs were dispensed into a 100 mm culture dish, treated with 10 μl of YJ102 (30mM) or DMSO (control) the next day, and incubated for 2 days. For cross-linking and DNA sharing, the cells were added with formaldehyde to be 1% to the culture dish in which UCB-MSCs were incubated and incubated at 37°C for 10 minutes to be fixed. The culture medium was removed and washed twice with PBS containing a protease inhibitor to collect the cells. The cells were centrifuged at 3000 rpm at 4°C for 5 minutes to remove the supernatant, and the cells were added with an SDS lysis buffer containing a protease inhibitor, incubated on ice for 10 minutes, and lysed by ultrasound. The cells were centrifuged at 13,000 rpm at 4°C for 10 minutes, the supernatant was transferred to a new microcentrifuge tube, and added with 1/10 volume of ChIP Dilution Buffer. Thereafter, DNA was purified and subjected to agarose gel electrophoresis to confirm that the DNA was shared at 200 to 1000 bp. In addition, for chromatin immunoprecipitation, the Protein G agarose slurry was washed with PBS, blocked with 1 ml of 1% BSA, and then washed with PBS, added with a cell lysate lysed by ultrasound and primary antibodies included anti-CtBP1 antibody (Cat. 07-306, Millipore), anti-LSD1 antibody (Cat. A1156, Abclonal), and Normal rabbit IgG (Cat. 12-370, Merck Millipore) (control group) at 1 μg each, and then incubated using a rotator overnight at 4°C. The next day, the supernatant was removed by centrifugation, and DNA-antibody-agarose beads were washed once with Low Salt Immune Complex Wash Buffer; High Salt Immune Complex Wash Buffer; and LiCl Immune Complex Wash Buffer in sequence, and then washed twice with 1X TE. The DNA-antibody-agarose beads were added with 250 μl of elution buffer (1% SDS, 0.1M NaHCOs), incubated at room temperature for 15 minutes using a rotator, and centrifuged, and the supernatant was transferred to a new microcentrifuge tube. The elution process was repeated one more time and the eluate was mixed. The eluate was added with 20 μl of 5 M NaCl, heated at 65°C for 4 hours, and then added with 10 μl of 0.5 M EDTA, 20 μl of 1 M Tris-HCl (pH 6.5), and 2 μl of proteinase K (10 mg /ml) and incubated at 45°C for 1 hour. Phenol/chloroform extraction and ethanol precipitation were performed, the DNA pellet was resuspended in DW, and then real-time quantitative PCR was performed using primers shown in Table 2 below.

[Table 2]

| Gene | Forward Primer | Reverse Primer |
|---|---|---|
| hHES1 | CTCCTCCCATTGGCTGAAAGTT | ATATCTGGGACTGCACGCGAAC |
| hOCT4 (-1kb) | TGTGCTTATGGCTGTTGATG | CCACTGTGCCCTGTTAGTTT |
| hOCT4 (-2kb) | GCATTCCGTTGGCTATTCTC | GATGTGCTTTGTTTAGTGGG |
| hSOX2 (-4Kb) | CCC CAA TAC TGG TGG TCG TCA AAC | GCAGTAATTAGGTGAGAACTAGCCAAGCATC |

As a result, it was shown that CtBP1 binding to a HES1 gene promoter increased by YJ102 to decrease HES1 expression (FIG. 4A), and a CtBP1-LSD1 complex bound to a Sox2 gene promoter by YJ102 to increase Sox2 expression (FIG. 4B). In addition, it was shown that the amount of CtBP1-LSD1 complex binding to a OCT4 gene promoter was increased by YJ102 to induce the expression of OCT4 (FIG. 4C), and CtBP1 binding to an HES1 gene promoter was increased by YJ102 to increase the CtBP1-LSD1 complex binding to Sox2 and Oct4 gene promoters (FIG. 4D).

**Example 2. Transdifferentiation into ciNSC5 by compounds of the present invention**

2-1. Confirmation of differentiation of UCB-MSCs into ciNSC5 by YJ102

To determine CtBP1-mediated ciNSC5 differentiation by YJ102 in human UCB-MSCs, a CTBP1 mechanism by treatment with NSC95397 or 4-methylthio-2-oxobutanoate (MTOB) was analyzed by immunochemical assay. Specifically, UCB-MSCs were dispensed at $2.1 \times 10^4$ cells/well in a 12-well plate containing an 18 mm cover glass (circular) (a DMSO control group was dispensed at $3.5 \times 10^3$ cells/well). The next day after dispensing, 30 μM of YJ102, 10 μM NSC95397 (Cat. 93718-83-3, Merck), or 300 μM of MTOB (Cat. K6000, Merck) was treated alone or in combination (the control group was treated with DMSO) and incubated at 37°C for 5 days. The culture medium was removed, washed with PBS, and fixed with 4% paraformaldehyde at room temperature for 10 minutes. After washed three times with PBS, the cells were permeabilized with PBS-T containing 0.5% Triton X-100 for 10 minutes, washed with PBS-T three times for 10 minutes each, and then blocked with a blocking solution (PBS-T containing 5% BSA and 5% goat serum) for 1 hour. Thereafter, the cells were treated with anti-Tuj1 antibody (Cat. 801201, BioLegend) diluted 1/5000 in a blocking solution and incubated at 4°C overnight. The next day, the cells were washed 10 times for 10 minutes each with PBS-T, and then treated with anti-mouse IgG-cy3 (Cat. 111-165-003, Jackson ImmunoResearch) diluted 1/2000 in a blocking solution, and incubated for 1 hour in dark conditions. Next, the cells were washed 10 times for 10 minutes each with PBS-T, mounted with a mounting solution containing DAPI, and then observed with a confocal microscope.

As a result, it was shown that the expression of Tuj 1 in YJ102-treated ciNSC5 differentiated from UCB-MSCs by YJ102 was reduced by NSC95397, which inhibited the binding of CtBP to a protein with a PxDLS motif (FIG. 5A), and the expression of Tuj 1 was reduced by MTOB, which inhibited the transcriptional activity of CtBP (FIG. 5B).

Through this, it was confirmed that YJ102 differentiated UCB-MSCs into ciNSC5, neural progenitor cells, through CtBP1 regulation.

2-2. Confirmation of transdifferentiation of UCB-MSCs into ciNSC5 by YJ101, YJ102 or YJ103

Immunochemical assay was performed to confirm whether UCB-MSCs were transdifferentiated into ciNSC5, Tuj1-positive neural progenitor cells, by the compounds of the present invention, YJ101, YJ102, and YJ103. Specifically, UCB-MSCs were dispensed at $2.1 \times 10^4$ cells/well in a 12-well plate containing an 18 mm cover glass (a DMSO control group was dispensed at $3.5 \times 10^3$ cells/well). YJ101, YJ102, and YJ103 were each dissolved in DMSO at 30 mM, and then treated in cells at a concentration of 30 μM each (the control group treated with DMSO) and incubated at 37°C for 5 days. The culture medium was removed, washed with PBS, and then fixed with 4% paraformaldehyde at room temperature for 10 minutes. After washed three times with PBS, the cells were permeabilized with PBS-T containing 0.5% Triton X-100 for 10 minutes, washed with PBS-T three times for 10 minutes each, and then blocked with a blocking solution (PBS-T containing 5% BSA and 5% goat serum) for 1 hour. Thereafter, the cells were treated with anti-Tuj 1 antibody (Cat. 801201, BioLegend) diluted 1/5000 in a blocking solution and incubated at 4°C overnight. The next day, the cells were washed 10 times for 10 minutes each with PBS-T, and then treated with anti-mouse IgG-FITC diluted 1/500 in a blocking solution, and incubated for 1 hour in dark conditions. Next, the cells were washed 10 times for 10 minutes each with PBS-T, mounted with a mounting solution containing DAPI, and then observed with a confocal microscope.

As a result, it was confirmed that the expression of Tuj1 was increased in ciNSC5 differentiated from UCB-MSCs by YJ101, YJ102, and YJ103 (FIG. 6), and YJ101, YJ102, and YJ103 all induced transdifferentiation from UCB-MSCs to ciNSC5.

2-3. Confirmation of transdifferentiation by YJ101, YJ102 or YJ103 of mesenchymal stem cells of different origins

To determine whether transdifferentiation into ciNSC5, neural progenitor cells, occurred similarly even in MSCs of different origins when human UCB-MSCs were treated with YJ101, YJ102, or YJ103 for 5 days, the expression of neuronal markers in differentiated ciNSC5 was confirmed by immunochemical assay using MSCs from human adipose-derived mesenchymal stem cells (AD-MSCs), human bone marrow-derived mesenchymal stem cells (ATCCs) and human umbilical cord blood-derived mesenchymal stem cells (UCB-MSCs; Promo Cells). Specifically, AD-MSC (StemPro™ Human Adipose-Derived Stem Cell, Gibco, Passage #6) was incubated in a MesenPRO RS™ medium, BM-MSC (ATCC, Passage #6) was incubated in a Mesenchymal Stem Cell Basal Medium for Adipose, Umbilical and Bone Marrow-derived MSC (Cat. PCS-500-030, ATCC) medium, and UCB-MSC (Promo Cell, Passage # 6) was incubated in a CellCor™ CD MSC (Cat. YSP001, Xell Therapeutics) medium, and then dispensed at $2.1 \times 10^4$ cells/well in a 12-well plate with an 18 mm cover glass (circular) (DMSO control group was dispensed at $3.5 \times 10^3$ cells/well). YJ101, YJ102, and YJ103 were each dissolved in DMSO at 30 mM, and then treated in cells at a concentration of 10, 20 or 30 μM each (the control group was UCB-MSC treated with DMSO) and incubated at 37°C for 5 days. The culture medium was removed, washed

with PBS, and then fixed with 4% paraformaldehyde at room temperature for 10 minutes. After washed three times with PBS, the cells were permeabilized with PBS-T containing 0.5% Triton X-100 for 10 minutes, washed with PBS-T three times for 10 minutes each, and then blocked with a blocking solution (PBS-T containing 5% BSA and 5% goat serum) for 1 hour. Thereafter, the cells were treated with anti-Tuj1 antibody (Cat. 801201, BioLegend) diluted 1/5000 in a blocking solution and incubated at 4°C overnight. The next day, the cells were washed 10 times for 10 minutes each with PBS-T, and then treated with anti-mouse IgG-cy3 diluted 1/2000 in a blocking solution, and incubated for 1 hour in dark conditions. Next, the cells were washed 10 times for 10 minutes each with PBS-T, mounted with a mounting solution containing DAPI, and then observed with a confocal microscope.

As a result, it was shown that the expression of Tuj1 was increased in ciNSC5 differentiated from AD-MSC by YJ101, YJ102, and YJ103 (FIG. 7A), the expression of Tuj1 was also increased in ciNSC5 differentiated from BM-MSC by YJ101, YJ102, and YJ103 (FIG. 7B), and the expression of Tuj1 was also increased in ciNSC5 differentiated from UCB-MSC by YJ101, YJ102, and YJ103 (FIG. 7C).

2-4, Confirmation of cytotoxicity of compounds

The cytotoxicity of YJ101, YJ102, and YJ103 was confirmed in human UCB-MSC using the Caspase-Glo 3/7 Assay System (Cat. G8091, Promega). Specifically, 100 μl (6,000 cells) of UCB-MSCs were dispensed into each well of a 96-well plate, and YJ101, YJ102, and YJ103 were each dissolved in DMSO at 30 mM, and then treated to the cells at a concentration of 10, 20, or 40 μM (the control group was treated with DMSO) and incubated at 37°C for 3 or 5 days. The compound plate was prepared by serial dilution from 40 μM in a 96-well plate. The highest dose was plated in rows 4, 8 and 12 and DMSO (excipient control) was plated in rows 1, 5 and 9 (Table 3). The compound plate was mixed in a shaker for 1 minute, the medium of the 96-well plate in which UCB-MSCs were dispensed was removed, and then 100 μl of the compound solution was treated and then incubated at 37°C for 3 and 5 days. Thereafter, 100 μl of a Caspase-Glo 3/7 reagent prepared by mixing a Place Caspase-Glo 3/7 Buffer with a Caspase-Glo 3/7 substrate was added to each well of the 96 well plate, covered with a plate cover, and then shaking-mixed at 300 to 500 rpm for 30 seconds. Thereafter, the cells were incubated at room temperature for 30 minutes, and luminescence was measured using a plate-reading luminometer, calculated using Equation 2 below, and plotted.

[Table 3]

| | 1 | 2 | 3 | 4 | 5 | 6 | 7 | 8 | 9 | 10 | 11 | 12 |
|---|---|---|---|---|---|---|---|---|---|---|---|---|
| A | DMSO | 10μM | 20μM | L1 40μM | DMSO | 10μM | 20μM | L1 40μM | DMSO | 10μM | 20μM | L1 40μM |
| B | DMSO | 10μM | 20μM | L2 40μM | DMSO | 10μM | 20μM | L2 40μM | DMSO | 10μM | 20μM | L2 40μM |
| C | DMSO | 10μM | 20μM | L3 40μM | DMSO | 10μM | 20μM | L3 40μM | DMSO | 10μM | 20μM | L3 40μM |
| D | | | | | | | | | | | | |
| E | | | | | | | | | | | | |
| F | | | | | | | | | | | | |
| G | | | | | | | | | | | | |
| H | | | | | | | | | | | | |

$$\text{Relative apoptosis (\%)} = \text{luminescence of test compound/DMSO luminescence} \times 100 \qquad \text{[Equation 2]}$$

As a result, it was shown that the compounds YJ101, YJ102 and YJ103 of the present invention did not induce cell apoptosis of UCB-MSCs (FIG. 8).

2-5. Confirmation of effect of compounds on MSC proliferation

To confirm the effect of YJ101, YJ102 and YJ103 on the proliferation of MSCs, 100 μl (6,000 cells) of UCB-MSCs were dispensed into each well of a 96-well plate, and YJ101, YJ102, and YJ103 were each dissolved in DMSO at 30 mM, and then treated to the cells at a concentration of 10, 20, or 40 μM (the control group was treated with DMSO) and incubated at 37°C for 3 or 5 days. The compound plate was prepared in the same manner as Example 2-4, the medium of the 96-well plate in which UCB-MSCs were dispensed was removed, and then 100 μl of the compound solution was treated and then incubated at 37°C for 3 and 5 days. Thereafter, 100 μl of a CellTiter-Glo reagent prepared

by mixing a CellTiter-Glo Buffer with a CellTiter-Glo substrate was added to each well of a 96-well plate, covered with a plate cover, and incubated at room temperature for 30 minutes. Then, the luminescence was measured using a plate-reading luminometer, calculated using Equation 3 below, and plotted.

$$\text{Relative proliferation (\%)} = \text{luminescence of test compound/DMSO luminescence} \times 100 \qquad \text{[Equation 3]}$$

As a result, the compounds YJ101, YJ102, and YJ103 of the present invention all reduced cell proliferation in a concentration-dependent manner (FIG. 9). Generally, the cell proliferation was reduced during cell differentiation.

**Example 3. Characteristics of ciNSC5 transdifferentiated by compounds of the present invention**

3-1. Analysis of neuronal marker expression in ciNSC5

The expression of neuronal markers in chemically induced neural stem cell 5 (ciNSC5), which was transdifferentiated from human UCB-MSC by treating YJ102 for 5 days by the method of Example 2, was confirmed by immunochemical assay and Western blot assay. Specifically, for immunochemical assay, UCB-MSCs were dispensed at $2.1 \times 10^4$ cells/well in a 12-well plate containing an 18 mm cover glass (a DMSO control group was dispensed at $3.5 \times 10^3$ cells/well). The next day of dispensing, YJ102 was dissolved in DMSO at 30 mM, and then treated in cells at a concentration of 30 μM (the control group treated with DMSO) and incubated at 37°C for 5 days. The culture medium was removed, washed with PBS, and then fixed with 4% paraformaldehyde at room temperature for 10 minutes. After washed three times with PBS, the cells were permeabilized with PBS-T containing 0.5% Triton X-100 for 10 minutes, washed with PBS-T three times for 10 minutes each, and then blocked with a blocking solution (PBS-T containing 5% BSA and 5% goat serum) for 1 hour. Thereafter, the cells were treated with primary antibodies diluted with the blocking solution, anti-Tuj1 antibody, anti-TBR2 antibody (Cat. Ab23345, abcam), anti-MASH1 antibody (Cat. Ab74065, abcam), anti-GAP43 antibody (Cat. Ab16053, abcam), anti-p75 antibody (Cat. G323A, Promega), anti-MAP2 antibody (Cat. 4542, Cell signaling), anti-NSE antibody (Cat. AB951, Chemicon), anti-NeuN antibody (Cat. MAB377, Chemicon), anti-Calretinin antibody (Cat. 180211, Invitrogen), anti-Doublecortin antibody (Cat. Ab18723, abcam), anti-NeuroD1 antibody (Cat. Ab213725, abcam), anti-Nestin antibody (Cat. Ab18102, abcam), anti-Musashi 1 antibody (Cat. AB5977, Chemicon) and anti-GFAP antibody (Cat. G3893, Sigma), respectively, and incubated at 4°C overnight. The next day, the cells were washed 10 times for 10 minutes each with PBS-T, and then treated with anti-mouse IgG-cy3 or anti-rabbit IgG-cy3 (Cat. 111-165-003, Jackson ImmunoResearch) diluted in a blocking solution, and incubated for 1 hour in dark conditions. Next, the cells were washed 10 times for 10 minutes each with PBS-T, mounted with a mounting solution containing DAPI, and then observed with a confocal microscope. In addition, for Western blot assay, the culture medium was removed and the cells were washed with PBS and added with a lysis buffer (1% Nonidet P-40, 20 mM Tris (pH 8.0), 137 mM NaCl, 0.5 mM EDTA, 10% glycerol, 10 mM $Na_2P_2O_7$, 10 mM NaF, 1 μg/ml aprotinin, 10 μg/ml leupeptin, 1 mM vanadate and 1 mM PMSF), and then the cells were collected using a scraper, rocked at 4°C for 20 minutes, and then centrifuged at 12,000 rpm for 30 minutes. After centrifugation, the supernatant was transferred to another e-tube, the proteins were quantified using a BCA assay kit, and the same amount of proteins was loaded on SDS-PAGE and electrophoresis was performed. Thereafter, the proteins were transferred to a nitrocellulose membrane, and subjected to Western blot according to protocol in the antibody data sheet of each of primary antibodies, anti-CD325 antibody (Cat. 10-0224, Invitrogen), anti-p75 antibody, anti-GAP43 antibody, anti-CD54 antibody (Cat. 67836, Cell signaling), anti-CD309 antibody (Cat. MA5-15157, Invitrogen), anti-CD56 antibody (Cat. A7913, Abclonal), anti-PSA-NCAM antibody (Cat. 5324, Millipore), anti-CD29 antibody (Cat. Ab183666, abcam), anti-MASH1 antibody, anti-TBR2 antibody, anti-CD44 antibody, anti-CD73 antibody, and anti-CD105 antibody.

As the immunoassay results, it was shown that ciNSC5 transdifferentiated from human UCB-MSC by treating YJ102 for 5 days expressed neural progenitor markers Tuj1, TBR2, MASH1, GAP-43, and p75, did not express mature neuronal markers MAP2, NSE and NeuN, did not express doublecortin and Neurogenic differentiation 1 (NeuroD1), which were known to be expressed during the early differentiation of neurons, and did also not express early neural stem cell markers Nestin, Mussashi 1 and GFAP (FIG. 10). In addition, as Western blot assay results of neural progenitor cell marker proteins, as compared to MSCs, the ciNSC5 increased Tuj1 protein 6-fold or more (about 6 to 8-fold), CD325 (N-cadherin) protein 7-fold or more (about 7 to 10-fold), p75 protein 4-fold or more (about 4 to 8-fold), GAP-43 protein 4-fold or more (about 4 to 7-fold), CD54 protein 2-fold or more (about 2 to 5-fold), CD309 protein 3-fold or more (about 3 to 7-fold), CD56 (NCAM) protein 3.5-fold or more (about 3.5 to 6-fold), PSA-NCAM protein 4-fold or more (about 4 to 6-fold), CD29 protein 4.5-fold or more (about 4.5 to 8-fold), MASH1 protein 4-fold or more (about 4 to 8-fold) or TBR2 protein 3-fold or more (about 3 to 7-fold) (FIGS. 11 and 13). On the other hand, it was shown that the protein expression of MSC markers CD44, CD73, and CD105 was reduced by 90%, 50%, and 80% or more in ciNSC5 compared to MSC, respectively (FIGS. 12 and 13).

3-2. Secretory protein analysis of ciNSC5

Western blot and ELISA assay were performed to confirm therapeutic effects of ciNSC5 transdifferentiated from human UCB-MSC by treating YJ102 for 5 days by the method of Example 2 through secretion of growth factors, and tissue degradation, and neural differentiation/regeneration-related factors. Specifically, UCB-MSCs were dispensed in a 100 mm culture dish at $3 \times 10^5$ cells/well (DMSO control group was dispensed at $5 \times 10^4$ cells/well), and the next day after dispensing, YJ102 was dissolved in DMSO at 30 mM, treated in the cells at a concentration of 30 $\mu$M (control group was treated with DMSO), and incubated at 37°C for 5 days to obtain a cell lysate of ciNSC5 differentiated from UCB-MSCs. In addition, the culture medium was removed from ciNSC5 differentiated from UCB-MSCs, washed with PBS, subcultured, added with a new YJ102-free culture medium, and incubated at 37°C for 2 days, or 4 and 7 days. Thereafter, the cells were lysed and Western blot assay was performed. Then, Western blot assay was performed on the cell lysate of ciNSC5 and the culture medium. Antibody information used during analysis was shown in Table 4.

[Table 4]

| antibody | catalog # | dilution | company |
|---|---|---|---|
| Shh | A7726 | 1/1000 | Abclonal |
| PGF | A 1727 | 1/1000 | Abclonal |
| HGF | A1193 | 1/1000 | Abclonal |
| MMP1 | A1191 | 1/1000 | Abclonal |
| MMP2 | 87809 | 1/1000 | Cell signaling |
| MMP9 | 13667 | 1/1000 | Cell signaling |
| MMP13 | A11148 | 1/1000 | Abclonal |
| TIMP1 | A4959 | 1/1000 | Abclonal |
| TIMP2 | 5738 | 1/1000 | Cell signaling |
| IGFBP1 | sc-25257 | 1/500 | Santacruz |
| IGFBP2 | sc-6001 | 1/500 | Santacruz |
| HB-EGF | A1695 | 1/1000 | Abclonal |
| ARSB | A14231 | 1/1000 | Abclonal |
| SEMA3C | A15386 | 1/1000 | Abclonal |
| NTNG2 | ab220615 | 1/1000 | Abcam |
| BMP2 | A0231 | 1/1000 | Abclonal |
| Pro-NGF | ANT-005 | 1/1000 | alomone labs |
| mNGF | SC-548 | 1/500 | Santacruz |
| GDNF | A14734 | 1/1000 | Abclonal |
| C3 | A11196 | 1/1000 | Abclonal |
| IYJ1033 | A8096 | 1/1000 | Abclonal |
| NXPH4 | A18190 | 1/1000 | Abclonal |
| IYJ101b | sc-1252 | 1/500 | Santacruz |
| DKK1 | sc-25516 | 1/500 | Santacruz |
| LIF | A1288 | 1/1000 | Abclonal |
| TGFb3 | A8460 | 1/1000 | Abclonal |
| NRG1 | A0687 | 1/1000 | Abclonal |
| FGF2 | SAB2100814 | 1/1000 | Sigma |
| BDNF | AB1534 | 1/1000 | Chemicon |

(continued)

| antibody | catalog # | dilution | company |
|---|---|---|---|
| VEGFA | ab46154 | 1/1000 | Abcam |
| TNFSF12 | A5659 | 1/1000 | Abclonal |
| ADAMTS4 | A2525 | 1/1000 | Abclonal |
| IYJ1016 | A1764 | 1/1000 | Abclonal |
| IGF-1 | 05-172 | 1/1000 | Millipore |
| NDNF | A14930 | 1/1000 | Abclonal |
| BMP4 | ab39973 | 1/1000 | Abcam |
| NRG2 | ab220615 | 1/1000 | Abcam |
| Anti-mouse IgG-HRP | 115-035-003 | 1/5000 | Jackson Immuno Resarch |
| Anti-rabbit IgG-HRP | 111-005-003 | 1/5000 | Jackson Immuno Resarch |
| Anti-goat IgG-HRP | A50-201P | 1/5000 | BETHYL |

As a result, it was shown that in the case of ciNSC5 differentiated from UCB-MSCs by YJ102 treatment, growth factors PlGF and VEGFA (VEGF-A) were increased and tissue degradation factors MMP1, MMP2, MMP7, and TIMP2 were increased in ciNSC5 compared to MSC, and among ligands involved in neural differentiation, neurogenesis, and axon regeneration, SHH and Notch1 were increased (FIGS. 14 and 15). In addition, when ciNSC5 differentiated from UCB-MSCs by YJ102 treatment were passaged and incubated in a YJ102-free medium for 2 days, growth factors increased in a ciNSC5 culture medium compared to MSC were PlGF, NGF, BDNF, and VEGFA (PlGF: 2.5-fold, NGF: 3.8-fold, BDNF: 2-fold, and VEGFA: 2.1-fold), and cytokines TNFSF12 and IL-16 were increased (FIGS. 16 and 17). In addition, it was shown that tissue degradation factors MMP1 and MMP2 were increased, and MMP7 and TIMP2 were increased, and among the ligands involved in neural differentiation, neurogenesis, and axon regeneration, SHH and Notch1 were increased (FIGS. 16 and 17). In addition, as a result of further incubating ciNSC5 in the YJ102-free medium for 4 or 7 days and then confirming the proteins in the medium by Western blot, as shown in FIGS. 18 and 19, it was shown that the secretion of growth factors, and tissue degradation, and neural differentiation/regeneration-related proteins was changed. Further, as a result of confirming the amounts of VEGFA, MMP-2, NGF, MMP1, PlGF and TNFSF12 among growth factors and tissue degradation-related factors which were increased in ciNSC5 in the Western blot assay result, in the medium in which ciNSC5 was incubated in the YJ102-free medium for 2 days, it was shown that the amounts of VEGFA, MMP-2, NGF, MMP1, PlGF, and TNFSF12 secreted in the ciNSC5 medium were significantly increased compared to the amounts confirmed in the MSC medium (FIGS. 20 and 21).

3-3. Analysis of genes expressed in ciNSC5 using RNA-seq

The expression gene profile of ciNSCS, which was transdifferentiated from human UCB-MSCs by treating YJ102 for 5 days, was analyzed using genome-wide RNA-seq. Specifically, $1 \times 10^5$ human UCB-MSCs were dispensed in a 60 mm culture dish (a control group dispensed at $1.67 \times 10^4$), the cells were treated with YJ102 dissolved at 30 mM in DMSO at a concentration of 30 μM (the control group treated with DMSO), and incubated at 37°C for 2, 3, or 5 days. Thereafter, the culture medium was removed, washed with PBS, and the cells were collected with a scraper and transferred to a microcentrifuge tube. PBS was removed by centrifugation, and the cells were added with 500 μl of a Tri-RNA Reagent and mixed well by pipetting. The mixture was added with 100 μl of chloroform, the tube was mixed by vortexing for 15 seconds, and then centrifuged at 4°C at 14,000 rpm for 20 minutes. Thereafter, the upper aqueous phase was transferred to a new microcentrifuge tube using a pipette, and added and mixed with 550 μl of isopropanol. The mixture was centrifuged at 14,000 rpm at 4°C for 20 minutes, the supernatant was discarded, and the pellet was washed with 1 ml of 70% ethanol. After centrifugation at 14,000 rpm for 2 minutes, the supernatant was discarded. The pellet was air-dried, and then RNA was lysed with 50 μl RNase-free DIW. The pellet was added with 1 μl DNase and incubated at 37°C for 30 minutes. Then, the pellet was added with an equal volume of 8 M LiCl, placed on ice for 1 hour, centrifuged at 14,000 rpm for 10 minutes at 4°C, washed with 70% ethanol, and air dried. The pellet was resuspended in 100 μl RNase-free DIW, centrifuged at 14,000 rpm for 10 minutes at 4°C to remove the supernatant, air-dried, and then the RNA concentration was quantified using nanodrop. Thereafter, RNAseq was performed at Theragen Bio, and the expression levels of genes between samples were compared using Fragments Per Kilobase of transcript per Million

(FPKM). To verify accuracy, a significant gene ontology classification was selected based on genes with *p-values* of less than 0.05 using a Fisher's method, and genes that increased 2-fold or more or decreased 0.5-fold or less compared to UCB-MSCs treated with DMSO were selected to perform the function analysis. The gene expression profile was shown as a heat map with three colors of RGB (red, green, blue) according to an expression value (FIG. 22), and Gene Ontology (Gene Ontology) was confirmed using Reactome, a gene function database and tendency analysis was performed.

As a result of RNA-seq, it was shown that the expression of cell divisionrelated genes decreased and the expression of nervous system-related genes increased in UCB-MSCs treated with YJ102 for 3 days. Based on this, as a result of selecting and analyzing genes involved in neurogenesis, synapses, neurotransmitters, and neural growth factors among the factors of which the expression was changed in the medium on day 5, the expression of genes (transcription factors, ligands, and receptors) that induced neurogenesis in ciNSCS, and the expression of genes involved in the secretion of brain-derived neurotrophic factors for neural regeneration and neuroprotection were prominent (FIGS. 23 and 24).

3-4. Analysis of genes expressed in ciNSC5 using real-time RT-PCR

The expression of genes selected based on the RNA-seq analysis results of Example 3-3 was confirmed by real-time RT-PCR to confirm the expression patterns of genes in MSC and ciNSC5. Specifically, $1 \times 10^5$ human UCB-MSCs were dispensed in a 60 mm culture dish (a control group dispensed at $1.67 \times 10^4$), the cells were treated with YJ102 dissolved at 30 mM in DMSO at a concentration of 30 $\mu$M (the control group treated with DMSO), and incubated at 37°C for 5 days. Thereafter, RNA was extracted and quantified using the same method as in Example above.

As a result, it was shown that the expression of enzymes for glial scar degradation in ciNSC5 differentiated from UCB-MSCs treated with YJ102 for 5 days was significantly increased compared to the control group (FIG. 25), and the expression of transcription factors related to neurogenesis was also increased compared to the control group (FIG. 26). In addition, it was shown that the expression of most neuronal markers such as myelination, synapsis, matrix/cell adhesion, and calcium signaling was increased compared to the control group (FIG. 27), and the expression of neuronal receptors and channels was also mostly increased compared to the control group (FIG. 28).

Through this, it was found that YJ102 treatment induced various neurogenic gene programs and also induced secretory molecules related to neurogenesis and axon regeneration. In addition, it was confirmed that YJ102 may also increase the expression of enzymes that enable glial scar degradation in ciNSC5.

3-5. Confirmation of changes in cell characteristics due to passage of ciNSC5

Changes in cell characteristics due to passage of ciNSC5 differentiated from human UCB-MSCs by YJ102 were confirmed by immunochemical assay for a Tuj1 antibody. Specifically, MSC and ciNSC5 were dispensed in a 100 mm culture dish at $2 \times 10^6$ each, passaged every 2 days, and incubated for a total of 6 passages, and then the culture medium was removed, washed with PBS, and fixed with 4% paraformaldehyde at room temperature for 10 minutes. After washed three times with PBS, the cells were permeabilized with PBS-T containing 0.5% Triton X-100 for 10 minutes, washed with PBS-T three times for 10 minutes each, and then blocked with a blocking solution (PBS-T containing 5% BSA and 5% goat serum) for 1 hour. Thereafter, the cells were treated with anti-Tuj 1 antibody diluted 1/5000 in a blocking solution and incubated at 4°C overnight. The next day, the cells were washed 10 times for 10 minutes each with PBS-T, and then treated with anti-mouse IgG-cy3 diluted 1/2000 in a blocking solution, and incubated for 1 hour in dark conditions. Next, the cells were washed 10 times for 10 minutes each with PBS-T, mounted with a mounting solution containing DAPI, and then observed with a confocal microscope.

As a result, it was shown that the doubling time was maintained even if ciNSC5 was passaged (FIG. 29), and the cumulative population doubling level (CPDL) value was also maintained depending on the passage number (FIG. 30). In addition, the expression of Tuj1 was maintained even when ciNSC5 was passaged (FIG. 31), and ciNSC5 differentiated by YJ102 showed no chromosomal abnormalities (FIG. 32).

Through this, it was found that cell characteristics were maintained even when ciNSC5 was subcultured.

3-6. Confirmation of differentiation of ciNSC5 into neurons

It was confirmed whether ciNSC5 differentiated from human UCB-MSCs by YJ102 could differentiate into neurons, and it was confirmed whether adipogenesis occurred by treatment with methylisobutylxanthine (MDI, Dexamethasone, Insulin) to investigate the differentiation stability of ciNSC5. Specifically, UCB-MSCs were dispensed in a 100 mm culture dish at $3 \times 10^5$ cells/well (DMSO control group was dispensed at $5 \times 10^4$ cells/well), and the next day after dispensing, YJ102 was dissolved in DMSO at 30 mM, treated in the cells at a concentration of 30 $\mu$M (a control group was UCB-MSC treated with DMSO), and incubated at 37°C for 5 days to obtain ciNSC5 differentiated from UCB-MSCs.

Thereafter, in order to differentiate into dopaminergic neurons or neurons, the ciNSC5 and MSCs as the control group were dispensed at $5 \times 10^5$ cells/dish in a 60 mm culture dish, respectively, and changes in morphology were observed while replacing the medium every 3 days. In addition, to confirm whether ciNSC5 could differentiate into adipocytes, MSCs and ciNSC5 were dispensed at $5 \times 10^5$ cells/dish each on a 60 mm culture dish, respectively, and incubated in a medium added with MDI (0.5 mM of 3-isobutyl-1-methylxanthine, 1 μM dexamethasone, and 10 μg/ml insulin) for 3 days and then recovered. For immunochemical assay, the culture medium was removed, washed with PBS, and fixed with 4% paraformaldehyde for 10 minutes at room temperature. After washed three times with PBS, the cells were permeabilized with PBS-T containing 0.5% Triton X-100 for 10 minutes, washed with PBS-T three times for 10 minutes each, and then blocked with a blocking solution (PBS-T containing 5% BSA and 5% goat serum) for 1 hour. Thereafter, the cells were treated with anti-TH antibody (Cat. p40101-150, Pel-Freez) and anti-MAP2 antibody (Cat. 4542, cell signaling) diluted in a blocking solution and incubated at 4°C overnight. The next day, the cells were washed 10 times for 10 minutes each with PBS-T, and then treated with anti-rabbit IgG-cy3 diluted in a blocking solution, and incubated for 1 hour in dark conditions. Next, the cells were washed 10 times for 10 minutes each with PBS-T, mounted with a mounting solution containing DAPI, and then observed with a confocal microscope. In addition, in order to extract RNA, the culture medium was removed, washed with PBS, and the cells were collected with a scraper and transferred to a microcentrifuge tube. PBS was removed by centrifugation, and the cells were added with 500 μl of a Tri-RNA Reagent and mixed well by pipetting. The mixture was added with 100 μl of chloroform, the tube was mixed by vortexing for 15 seconds, and then centrifuged at 4°C at 14,000 rpm for 20 minutes. Thereafter, the upper aqueous phase was transferred to a new microcentrifuge tube using a pipette, and added and mixed with 550 μl of isopropanol. The mixture was centrifuged at 14,000 rpm at 4°C for 20 minutes, the supernatant was discarded, and the pellet was washed with 1 ml of 70% ethanol. After centrifugation at 14,000 rpm for 2 minutes, the supernatant was discarded. The pellet was air-dried, and then RNA was lysed with 50 μl RNase free DIW. The pellet was added with 1 μl DNase and incubated at 37°C for 30 minutes. Then, the pellet was added with an equal volume of 8 M LiCl, placed on ice for 1 hour, centrifuged at 14,000 rpm for 10 minutes at 4°C, washed with 70% ethanol, and air-dried. The pellet was resuspended in 100 μl RNase-free DIW, centrifuged at 14,000 rpm for 10 minutes at 4°C to remove the supernatant, air-dried, and then the RNA concentration was quantified using nanodrop. Thereafter, 500 ng of RNA was added with 2 μl of PrimeScript RT Master Mix, and the total volume was adjusted to 10 μl with DW. cDNA was synthesized by reverse transcription at 37°C for 15 minutes, and reacted at 85°C for 5 seconds to inactivate reverse transcriptase. The expression of C/EBPα and PPARγ, adipogenesis markers, was confirmed by real-time PCR using the synthesized cDNA.

As a result, it was confirmed that ciNSC5 differentiated into neurons when incubated in a differentiation medium (FIG. 33), and unlike MSC, ciNSC5 did not respond to the induction of adipogenesis (FIG. 34).

**Example 4. Confirmation of therapeutic effect on Lou Gehrig's disease of ciNSC5 transdifferentiated by compounds of the present invention**

4-1. Confirmation of increased lifespan of ALS model mouse

To confirm the effectiveness of ciNSC5 on Lou Gehrig's disease, an ALS model mouse was fabricated, ciNSC5 was transplanted into the ALS model mouse, and the lifespan of the mouse was confirmed. Specifically, transgenic ALS mice (B6SJL-Tg(SOD1-G93A)1Gur/J over-expressing human SOD1 containing the Gly93→Ala mutation, The Jackson Laboratory, Bar Harbor, ME, USA) were transformed into female mice (B6/SJLF1) with similar backgrounds to fabricate a $SOD1^{G93A}$ transgenic ALS mouse model. For their progenies, genotypes for tail DNA were analyzed using a PCR assay. At 60 days after birth, mice were randomly grouped, anesthetized with chloral hydrate (500 mg/kg, intraperitoneally), and ciNSC5 ($1 \times 10^6$ cells) differentiated from human UCB-MSCs treated with 10 μl of MSC or YJ102 suspended in saline for 5 days were injected into the cisterna magna. In a control group, the same amount of saline was injected into the cisterna magna. Thereafter, the lifespan of each group was confirmed.

As a result, the lifespan of both a MSC-transplanted group and a ciNSC5-transplanted group increased compared to a saline-treated group, and particularly, the lifespan of the ciNSC5-transplanted group was further extended 12 days compared to the saline-transplanted group and 9 days compared to the MSC-transplanted group (FIG. 35).

4-2. Confirmation of ALS onset and delayed progression of motor symptoms

After transplanting ciNSC5 into the ALS model mouse, behavioral tests were performed every 5 days from day 75 after birth to confirm the motor function of the mouse. The behavioral tests were performed by a Rotarod test, a motor score, a hanging wire test, and a balance beam test. For the rotarod test to assess general motor coordination and strength and balance in the mouse, the mouse was trained on a rotarod device (4-40 rpm. 180 sec) from 3 days before testing, each mouse exercised on the rotarod for 180 seconds during the test, and the time when the mouse fell from the rotarod in the middle of the exercise was recorded. A total of three tests were conducted per mouse, with a rest

interval of at least 10 minutes between tests. The average value of three recordings was determined as the score for each mouse. In addition, the motor score was determined by checking the condition of the hind limbs when the tail of the mouse was lifted and scoring 0 to 4 points based on the criteria [4 points = normal; 3.5 points = onset of hindlimb abduction defect; 3 points = abnormal gait; 2 points = partial hind limb paralysis (first sign of leg dragging); 1 point = hindlimb paralysis and forelimb weakness; 0 point = paralysis of the forelimbs similar to the hindlimbs]. In addition, in order to perform the hanging wire test to evaluate the neuromuscular strength of the leg held against gravity, each mouse was placed on a wire lead in a conventional housing case and the lead turned back and forth. The test was performed for a total of 180 seconds, and the time until the mouse hanging on the wire lead fell to the floor was measured. A total of three tests were conducted per mouse, with a rest interval of at least 10 minutes between tests. The average value of three recordings was determined as the score for each mouse. Further, the balance beam test was performed by placing the mouse on a round wooden beam (0.5 cm diameter, 100 cm long, and 60 cm high) and scoring 0 to 6 points of the ability to maintain balance based on the criteria [0 point = mouse may not stand on beam for 30 seconds; 1 point = mouse may stand on beam for 30 seconds; 2 points = mouse may not walk but may turn left or right on beam; 3 points = mouse may turn left or right on beam and may take at least one step; 4 points = mouse may traverse beam with paw slipping of 50% or more of the affected hindlimb; 5 points: mouse may traverse the beam with paw slipping of 50% or less of the affected hindlimb; 6 points = mouse may traverse beam with paw slipping of one step or less].

As a result of the rotarod test, there was no significant difference between the saline-treated group and the MSC-transplanted group, but in the ciNSC5-transplanted group showed a significantly delayed decline in motor capacity compared to both the saline-transplanted group and the MSC-transplanted group (12.8 $\pm$ 5.8 in the saline group, 21.8 $\pm$ 9.0 in the MSC group, and 107.4 $\pm$ 22.7 in the ciNSC5 group for 120 days) (FIG. 36A). In addition, as a result of confirming the motor score, the motor score in SOD1$^{G93A}$ transgenic mice began to decrease from day 90 and continued to decrease until the end point, MSC-transplanted mice showed somewhat higher scores than saline-treated mice, but had no significant difference, whereas the ciNSCS-transplanted mouse group showed a decrease in motor scores after 120 days, and motor scores during the experimental period were significantly higher than both the saline group and the MSC group (2.1 $\pm$ 0.4 in the saline group, 2.3 $\pm$ 0.4 in the MSC group, and 3.7 $\pm$ 0.2 in the ciNSC5 group for 120 days) (FIG. 36B). In addition, as a result of the hanging wire test, mice in the saline-transplanted group and the MSC-transplanted group began to fall off the wire from day 80, but the onset of the disease was delayed to the extent that some mice transplanted with ciNSC5 were observed to fall after 95 days. In addition, the ciNSC5-transplanted group showed significantly higher scores than the other two groups throughout the entire process of the hanging wire test (6.4 $\pm$ 2.8 in the saline group, 22.9 $\pm$ 15.14 in the MSC group, and 113.1 $\pm$ 18.2 in the ciNSC5 group for 120 days) (FIG. 36C). Further, as a result of the balance beam test, the ciNSC5-transplanted group showed significantly higher scores than the other two groups from 85 days after birth to the end of the experiment (1.5 $\pm$ 0.32 in the saline group, 2.3 $\pm$ 0.5 in the MSC group, and 4.0 $\pm$ 0.5 in the ciNSC5 group for 120 days) (FIG. 36D).

4-3. Confirmation of inhibitory effect on motor neuron loss in spinal cord

To determine whether ciNSC5 transplantation palliated the lumbar spinal cord nerve loss accompanied with the clinical symptoms of ALS, Cresyl violet staining was performed on day 110 after transplantation, and the number of motor neurons in the lumbar spinal cord was counted.

As a result, compared to the average of 7711.3 $\pm$ 193.8 motor neurons in wildtype mice, the number of motor neurons in saline-treated SOD1$^{G93A}$ transgenic mice was significantly reduced (3875.1 $\pm$ 465), and MSC-transplanted mice showed a slight increase in the number of motor neurons compared to saline-treated mice, but had no significant difference. However, it was shown that the number of motor neurons in the ciNSC5-transplanted mouse group was significantly increased compared to the saline-treated or MSC-transplanted group (FIG. 37).

Through this, it was confirmed that ciNSC5 significantly inhibited death of motor neurons in the spinal cord compared to both saline- and MSC-treated groups in the SOD1$^{G93A}$ transgenic ALS mouse model, delayed the progression of Lou Gehrig's disease symptoms accompanied by motor dysfunction and extended the lifespan of ALS mice.

**Example 5. Confirmation of therapeutic effect on multiple sclerosis of ciNSC5 transdifferentiated by compounds of the present invention**

5-1. Confirmation of improvement in behavioral symptoms of EAE mice

The effectiveness of ciNSC5 was confirmed in an experimental autoimmune encephalomyelitis (EAE) mouse model, which was an orthodox model of multiple sclerosis (MS). Specifically, female adult C57BL/6NTac mice (8 to 9 weeks old; 19 to 21 g) were purchased from Narbiotec Co., Ltd. (Seoul, Republic of Korea), and subcutaneously immunized with 200 ng MOG35-55 peptide (Sigma-Aldrich, St. Louis, MO, USA) in Complete Freund's Adjuvant (Sigma-Aldrich) containing 4 mg/ml sterile *Mycobacterium tuberculosis* (Difco) to induce EAE. On days 0 and 2, 200 ng pertussis

toxin (Sigma-Aldrich), which played a key role in stimulating the immune response, was injected intraperitoneally (FIG. 38A). On day 8 after MOG injection, mice were anesthetized with chloral hydrate (500 mg/kg, intraperitoneally) and 10 μl of MSC or ciNSC5 ($1\times10^6$ cells) suspended in saline were injected into the cisterna magna. The control group was injected with the same amount of saline at the same time. Thereafter, the behavioral symptoms of the EAE mouse model were assessed daily according to a standard score ranging from 0 to 7 below: Score 0 = no symptoms; Score 1 = partial tail sagging; Score 2 = moderate hindlimb waddling gait; Score 3 = moderately severe hindlimb waddling gait; Score 4 = paraplegia; Score 5 = paraplegia with moderate forelimb waddling gait; Score 6 = quadriplegia, moribund; Score 7 = death. The behavior analysis results were analyzed by repeated evaluation of ANOVA (time vs treatment).

As a result, mice in the saline-treated control group began to exhibit typical behavioral symptoms, including tail sagging and leg paralysis, demonstrating that paralysis was progressing, at 8 to 9 days (onset stage) after EAE induction, peaking at 18 to 20 days, and continued until the end of the experiment (FIG. 38B). On the other hand, in the group transplanted with MSC or ciNSC5, the behavioral symptoms of EAE were significantly palliated even at the onset stage, and particularly, the clinical scores in the ciNSC5 transplanted group were significantly lower than those in the MSC transplanted group ($2.7 \pm 0.2$ in the saline group, $2.1 \pm 0.2$ in the MSC group, and $1.5 \pm 0.2$ in the ciNSC5 group at 9 days after transplantation) (FIGS. 38B and 38D). There were no significant differences in body weight between experimental groups during the experiment period (FIG. 38C).

5-2. Confirmation of CNS demyelination inhibition effect

On day 30 after MOG injection, mice were anesthetized with chloral hydrate (500 mg/kg) and perfused via cardiopuncture with 0.1 M PBS (pH 7.4) followed by 4% paraformaldehyde in 0.1 M PBS (pH 7.4). The lumbar segments of the spinal cord were dissected, post-fixed for 6 hours in the same fixing solution, and placed in 30% sucrose in 0.1 M PBS (pH 7.4). The segments were placed in OCT to make frozen sections, and cross-sectional sections were cut at 16 μm using a cryostat (Leica, Germany). For molecular studies, animals were perfused with PBS and sections of the lumbar spinal cord (L4-L5) were isolated and frozen at - 80°C until use. The frozen sections were analyzed by immunohistochemistry using antibodies against GFAP (DAKO, Santa Clara, CA, USA), CD11b (Millipore, Billerica, MA), and CD68 (Millipore). For double labeling, FITC or cy3-conjugated secondary antibodies were used (Jackson ImmunoResearch, West Grove, PA). In addition, nuclei were labeled with DAPI (Molecular Probes, Eugene, OR). Immunostaining control studies were performed by replacing the primary antibody with a non-immune, control antibody and preadsorbing the antibody with an excess (10 μg/ml) of each antigen to remove the primary antibody. Fluorescence intensities of a titer or more were quantified and averaged using MetaMorph software (Molecular devices, Sunnyvale, CA). Titer values were at least three backgrounds and the backgrounds were quantified and averaged for the primary antibody missing control. In addition, for Western blot assay, frozen spinal cord tissue was added with a lysis buffer (1% Nonidet P-40, 20 mM Tris (pH 8.0), 137 mM NaCl, 0.5 mM EDTA, 10% glycerol, 10 mM $Na_2P_2O_7$, 10 mM NaF, 1 μg/ml aprotinin, 10 μg/ml leupeptin, 1 mM vanadate and 1 mM PMSF) and homogenized. The mixture was collected in an e-tube, rocked at 4°C for 20 minutes and centrifuged at 12,000 rpm for 30 minutes, and then the supernatant was transferred to another e-tube, protein quantification was performed using a BCA assay kit, and the same amount of proteins was loaded on SDS-PAGE and subjected to electrophoresis. Thereafter, the proteins were transferred to a nitrocellulose membrane, and Western blot assay was performed according to the protocol from each antibody data sheet. The obtained data were expressed as mean ± SEM, and multiple comparisons between groups were performed using one-way ANOVA. Tukey's multiple comparison was used as post hoc analysis, $p < 0.05$ was considered statistically significant. All statistical analyses were performed using SPSS 15.0 (SPSS Science, Chicago, IL).

As a result, demyelination assessed by luxol fast blue staining was significantly increased in the white matter of the spinal cord of EAE mice at 30 days after immunization (FIG. 39A, Saline), but demyelination of the spinal cord was significantly decreased in ciNSC5-transplanted EAE mice (FIGS. 38A and 38B). In addition, ciNSC5-transplanted EAE mice showed a significantly decreased demyelination effect compared to MSC-transplanted EAE mice. As a result of confirming the expression of myelin basic protein (MBP) expression by immunostaining and Western blot to re-verify the demyelination inhibition effect of ciNSC5, the expression of MBP was significantly decreased in EAE mice administered with saline compared to Sham, and the decrease in MBP was significantly suppressed in the spinal cord of mice transplanted with ciNSC5 compared to mice transplanted with saline or MSC (FIGS. 39C and 39D). Western blot results also showed that MBP expression in ciNSC5-transplanted mice was significantly higher than that of other two groups (saline-transplanted group or MSC-transplanted group) (FIGS. 39E and 39F). Through this, it was confirmed that ciNSC5 inhibited CNS demyelination, which was known as a typical pathological characteristic of MS patients, thereby palliating the scope and degree thereof.

5-3. Confirmation of inhibitory effect of monocyte and macrophage infiltration into spinal cord white matter

On day 30 after MOG immunization, infiltration of monocytes around small blood vessels of the spinal cord

was detected by cresyl violet (Cat. C5042, Sigma) staining. In addition, CD68-positive macrophages were confirmed by immunohistochemical staining on day 30 after MOG immunization.

As a result, it was confirmed that EAE mice showed intensive infiltration of monocytes around the white matter of the spinal cord (FIGS. 40A and 40B, saline), whereas the MSC or ciNSC5 treated group showed reduced infiltration of monocytes into the spinal cord white matter in both thoracic (A) and lumbar (B) slices (FIG. 40), and ciNSC5 transplantation palliated EAE-induced monocyte infiltration into the spinal cord white matter. In addition, while D68-positive macrophages were observed in the white matter of saline-treated EAE mice, the number of infiltrated macrophages was significantly reduced in the MSC or ciNSC5 transplanted group (FIG. 41).

5-4. Confirmation of inhibitory effect on activation of microglia and astrocytes

To determine the effect of ciNSC5 on the activation of glial cells such as microglia and astrocytes in the spinal cord, immunohistochemical assay for CD11b (microglial marker) or GFAP (astrocyte marker) antibody was performed at day 30 after immunization.

As a result, it was confirmed that very strong GFAP immunoreactivity appeared in the spinal cord of EAE mice treated with saline, and thus the activation of astrocytes in the spinal cord occurred (FIG. 42). On the other hand, in EAE mice transplanted with MSC or ciNSC5, the activation of astrocytes in the spinal cord was significantly reduced, and as a result of quantifying GFAP fluorescence intensity, GFAP intensity was significantly decreased in both the MSC-transplanted group and the ciNSC5-transplanted group. In particular, ciNSC5 showed a significant inhibitory effect on GFAP expression compared to MSC (FIG. 42D). It was shown that ciNSC5 inhibited the activation of astrocytes in EAE mice. In addition, as a result of immunostaining with CD11b, microglia in the spinal cord of EAE mice showed typical activation patterns such as enlarged cytoplasm and shortened branches (FIG. 43A), whereas the activation pattern of microglia in EAE mice transplanted with MSC or ciNSC5 was significantly reduced. In particular, in ciNSC5-transplanted mice, many resting forms of microglia with long and thin branches were also observed (FIG. 43). These results indicated that the microglia activation was inhibited by ciNSC5 transplantation in EAE mice.

**Example 6. Confirmation of therapeutic effect on Parkinson's disease of ciNSC5 transdifferentiated by compounds of the present invention**

6-1. Confirmation of effect of improving motor function recovery

To confirm the effect of ciNSC5 on functional recovery in the chronic stage of a PD animal model through 6-OHDA injection, adult Sprague-Dawley male rats (230 to 250 g, Sam:TacN(SD) BR, Samtako, Osan, Korea) were weighed and anesthetized with chloral hydrate (500 mg/kg, intraperitoneal injection), and fixed to Stereotaxic, and 8 μg of 6-OHDA was dissolved in 4 μl of saline and injected at a predetermined location (Partial injury model: AP +0.7, ML +2.6, DV -4.5 from bregma; and Complete injury model: AP -2.2, ML +1.5, DV -8.0 from bregma) using a 33 gauge Hamilton syringe (FIG. 44). At week 4 after 6-OHDA injection, the rats were anesthetized with chloral hydrate (500 mg/kg, i.p.), fixed in a stereotaxic, and the right striatum (AP: +1.0, ML: -3.0, DV: -5.0) was transplanted with 10 μl of MSCs or ciNSC5 ($1 \times 10^6$ cells) suspended in saline. Thereafter, the rats were injected subcutaneously with apomorphine (0.05 mg/kg) and placed in a transparent cylinder with a diameter of 40 cm, and after 10 minutes of adaptation, the rats were determined as rats in which the Parkinson's disease model was completed when the rats were rotated 4 times or more per minute for 10 minutes. To evaluate the efficacy of improving the ciNSC5 motor function in the partial injury model, a rotation test was performed every two weeks after cell transplantation. In addition, in the case of the Complete injury model, a stepping test was performed once a week from week 2 after transplantation to confirm the recovery of motor function. Each front paw of the rat was placed on the treadmill, and observed by recording a video for 1 minute, and a stepping ratio (%) was calculated by counting a contralateral step in which walking disturbance in a side opposite to the side injected with 6-OHDA was injected occurred, and a ipsilateral step where normal walking was possible, and dividing the contralateral steps by the total steps. Normal rats showed a stepping ratio of 50%, and rats with Parkinson's disease showed a stepping ratio of 10% or less.

As a result, in the partial injury model, the number of rotations decreased in rats transplanted with MSC and ciNSC5 as the time passed after transplantation, and in ciNSC5-transplanted rats, a significant motor function recovery effect was shown compared to the saline-transplanted group from 2 to 6 weeks after transplantation (FIG. 45A). In addition, in the complete injury model, the saline administered group showed no change at all compared to before transplantation, while the ciNSC5 transplanted group significantly increased the stepping ratio compared to the saline group from week 2 after transplantation, and the effect thereof lasted until 6 weeks after transplantation (FIG. 45B).

6-2. Confirmation of effect of inhibiting dopaminergic neuron loss

Cells were transplanted into a 6-OHDA-injected PD animal model, and after 4 or 6 weeks, rats were anesthetized with chloral hydrate (500 mg/kg) and perfused via cardiopuncture with 0.1 M PBS (pH 7.4) followed by 4% paraformaldehyde in 0.1 M PBS (pH 7.4). The brain was extracted and immersed and post-fixed in the same fixing solution for 24 hours, and placed in 30% sucrose in 0.1 M PBS (pH 7.4). Immunohistochemical staining was performed by cutting the tissue embedded with an OCT compound at 40 μm using a cryostat (Leica, Germany) and immunostaining the tissue containing Substantia nigra with an antibody against TH, a dopaminergic neuronal marker.

As a result, it was found that the number of dopaminergic neurons remained significantly higher in the tissue transplanted with ciNSC5 than in the tissue transplanted with saline or MSC (FIG. 46).

6-3. Confirmation of differentiation effect into TH-positive dopaminergic neurons

As a result of immunostaining the striatum of rats 6 weeks after transplantation in a partial injury model using a human-specific antibody, STEM121, No STEM121-positive cells were observed in the MSC transplanted group (data not shown), but STEM121-positive cells were identified in the ciNSC5 transplanted group (FIG. 47). This means that the transplanted ciNSC5 is surviving in the tissue even 6 weeks after transplantation. In addition, as a result of double immunostaining using a STEM121 antibody and a TH antibody, it was confirmed that the STEM121-positive human cells in the ciNSC5-transplanted tissue were TH-positive dopaminergic neurons (FIG. 48), and thus it was shown that the transplanted ciNSC5 differentiated into dopaminergic neurons in the striatum.

Through this, ciNSC5 not only significantly improved behavioral symptoms in the PD model induced by 6-OHDA, but also showed the effect of inhibiting the death of dopaminergic neurons and remarkable therapeutic efficacy compared to MSC. In particular, since ciNSCS, transplanted neural progenitor cells, differentiated into dopaminergic neurons in the striatum, the transplant site, it may be seen that ciNSC5 may exhibit therapeutic efficacy by replacing dopaminergic neurons decreased due to PD when transplanted into neural tissue.

**Example 7. Confirmation of therapeutic effect on chronic spinal cord injury of ciNSC5 transdifferentiated by compounds of the present invention**

7-1. Confirmation of effect of improving motor function recovery

To determine the effect of ciNSC5 on functional recovery in a chronic stage after spinal cord injury, after ciNSC5 transplantation, behavioral tests including a BBB open field test, a grid walk test, and a foot print analysis were performed, and functional recovery index (FRI) analysis was performed by integrating the behavioral tests. Specifically, to fabricate a chronic spinal cord injury rat model, before surgery, adult Sprague-Dawley male and female rats (230 to 250 g, Sam:TacN(SD) BR, Samtako, Osan, Korea) were weighed and anesthetized with chloral hydrate (500 mg/kg, intraperitoneal injection), and the back and neck were shaved and laminectomy was performed at T9-T10 levels to expose the lower part of the spinal cord without damaging the dura. Thereafter, the spinous processes of T8 and T11 were fixed with clamps to stabilize the spine, and a moderate contusion injury (10 g × 25 mm) was applied to the exposed back surface of the spinal cord to fabricate a chronic spinal cord injury rat model. After injury, the muscle and skin were covered and the rats were placed in a temperature and humidity controlled chamber overnight. After surgery, the rats were injected subcutaneously with supplementary fluid (5 ml, lactated Ringer) and injected with an antibiotic (gentamicin, 5 mg/kg, intramuscular injection) once daily for 5 days after surgery. After injury, the rats were bred one per cage and easily accessed water and feed. Body weights and remaining food and water weights were checked and recorded for all animals. The bladder was passively emptied three times per day until reflex voiding was established. At week 6 after spinal cord injury, rats were anesthetized with chloral hydrate (500 mg/kg, i.p.), and 10 μl of MSCs or ciNSC5 ($1 \times 10^6$ cells) suspended in saline were injected into the lesion space. The control group was injected with the same amount of saline at the corresponding time. The behavioral analysis of the BBB open field test, the grid walk test, and the footprint analysis was performed once a week for 8 weeks after transplantation. The BBB open field test used the BBB score for functional recovery and mobility tests in chronic SCI studies, and the recovery after contusion injury in the rat spinal cord was evaluated by testing the locomotion function using the following open field locomotor scores (Table 5) according to the Basso, Beattie, and Bresnahan (BBB) criteria ranging from complete paralysis (score 0) to normal locomotion (score 21). In addition, in the grid walk test, animals walked on a horizontal runway of a 1 m-long metal grid bar placed 30 cm from the floor, and 10 defined bar sectors were selected for analysis. To prevent habituation to fixed bar spacing, the bars in these sectors were arranged irregularly (1 to 4 cm intervals) and changed every test sections. The analysis was performed by counting the number of errors at the foot position, and if the animal did not walk on the hind legs, two errors were made per bar, resulting in a total of 20 errors, and the percentage of tripping per step was calculated. In addition, the foot print analysis was performed by soaking the animals' front and hind paws in non-toxic red and blue

dyes and then walking across a narrow box (1 m long and 7 cm wide), scanning the footprints, and analyzing the digitized images. In addition, by integrating the three behavioral tests and comparing the behavior at the time of cell transplantation and 8 weeks after transplantation, the locomotion recovery delta values for 8 weeks were calculated to quantify the stage of motor function recovery to derive the Functional Recovery Index (FRI). The standards for the points given in each behavior experiment were shown in Table 6 below.

[Table 5]

0. No observable hindlimb( HL) movement.
1. Slight (45° 이하) movement of one or two joint , usually the hip and/or knee.
2 Extensive movement of one joint Or extensive movement of one joint and slight movement of one other joint .(rare case)
3. Extensive movement of two joints
4 Slight movement of all three joint of HL
5. Slight movement of two joint and extensive movement of the third
6. Extensive movement of two joints and slight movement of the third
7. Extensive movement of all three joint of the HL
8. Sweeping with no weight support Or plantar placement of the paw with no weight support
9. Plantar placement of the paw with weight support in stance only (i.e., when stationary) Or occasional, frequent or consistent weight supported dorsal stepping and no plantar stepping
10. Occasional (50% 이하) weight supported plantar step, no forelimb (FL)-HL coordination
11. Frequent to consistent (95%-100%) weight supported plantar steps and occasional FL-HL coordination
12. Consistent weight supported plantar steps and occasional FL-HL coordination
13. Consistent weight supported plantar steps and frequent FL-HL coordination
14. Consistent weight supported plantar steps, consistent FL-HL coordination ; and predominant paw position during locomotion is rotated (internally or externally) when it makes initial contact with the surface as well as just before it is lifted off at the end of stance Or frequent plantar stepping, consistent FL-HL coordination , and occasional dorsal stepping
15. Consistent plantar stepping and consistent FL-HL coordination ; and no toe clearance or occasional toe clearance during forward limb advancement, predominant paw position is parallel to the body at initial contact
16. Consistent plantar stepping and consistent FL-HL coordination during gait; toe clearance occurs frequently during forward limb advancement ; predominant paw position is parallel at initial contact and rotated at lift off
17. Consistent plantar stepping and consistent FL-HL coordination during gait; toe clearance occurs frequently during forward limb advancement ; predominant paw position is parallel at initial contact and lift off
18. Consistent plantar stepping and consistent FL-HL coordination during gait; toe clearance occurs consistently during forward limb advancement ; predominant paw position is parallel at initial contact and lift off
19. Consistent plantar stepping and consistent FL-HL coordination during gait; toe clearance occurs consistently during forward limb advancement ; predominant paw position is parallel at initial contact and lift off; and tail is down part or all of the time
20. Consistent plantar stepping and consistent coordinated gait; consistent toe clearance; predominant paw position is parallel at initial contact and lift off; tail consistently up ; and trunk instability
21. Consistent plantar stepping and consistent coordinated gait; consistent toe clearance; predominant paw position is parallel throughout stance, consistent trunk stability, tail consistently up

[Table 6]

| Behavioral test | Score criteria |
|---|---|
| BBB Open Field Test | Points equal to BBB scores increased for 8 weeks |
| Grid walk test | 1 point per 20% reduction compared to average delta value in saline-treated group |
| Foot print test | 1 point for each improvement in relative position and toe dragging |

As a result, it was shown that both the ciNSC5 transplanted group and the MSC transplanted group significantly increased the BBB migration score after the spinal cord injury (FIGS. 49A and 49D). In addition, the grid error rate (tripping per step) of both ciNSC5 and MSC was lower than that in the saline-treated group, and the rate of tripping in the ciNSC5 transplanted group was significantly reduced compared to the MSC transplanted group in both male and female rats (FIGS. 49B and 49E). At week 14 after injury (week 8 after transplantation), footprints in both ciNSCS-transplanted and MSC-transplanted rats showed fairly consistent forelimb-hindlimb coordination and very little toe dragging. On the other hand, footprints from the saline-treated group showed inconsistent forelimb-hindlimb coordination and a lot of dragging, with ink marks extending from both hindlimbs. In addition, it was shown that toe dragging in the ciNSC5 transplanted group was significantly reduced compared to the MSC transplanted group (FIGS. 49C and 49F). Further, as a result of functional recovery index (FRI) analysis, the FRI scores of ciNSC5 and MSC significantly increased compared to the saline transplanted group, and in particular, the FRI score of the ciNSC5 transplanted group was significantly improved compared to the MSC transplanted group (FIGS. 50A, 50B, 50D and 50E). Abnormal body weight changes were not observed according to transplantation of ciNSC5 and MSC while the behavioral experiment was performed (FIGS. 50C and 50F).

7-2. Confirmation of effect of reducing lesion volume and myelin loss

On week 8 after MSC or ciNSC5 transplantation, animals were anesthetized with chloral hydrate (500 mg/kg) and perfused via cardiopuncture with 0.1 M PBS (pH 7.4) followed by 4% paraformaldehyde in 0.1 M PBS (pH 7.4). A spinal cord section (1.5 cm) focused on the lesion site was cut and immersed and post-fixed in the same fixing solution for 6 hours, and placed in 30% sucrose in 0.1 M PBS (pH 7.4). The segment was placed in OCT to make frozen sections, and longitudinal or transverse sections were cut at 10 or 16 μm. To determine the lesion volume using serial longitudinal sections (10 μm) through the dorsoventral axis of the spinal cord, sections every 50 μm were stained with Cresyl violet acetate and observed under a light microscope. The lesion area was determined by MetaMorph software (Molecular devices) using a low-power lens (1.25 X), and the area at each longitudinal level was determined, and the total lesion volume was inferred as the average of the numerical integration of the contiguous areas. In addition, to determine myelin loss after injury, serial transverse cryosections (16 μm thick) were mounted on glass slides. Selected slides were incubated overnight at 60°C with 0.1% Luxol fast blue (Solvent Blue 38; Sigma) acidified with 95% ethanol. Differentiation was performed with 0.05% lithium carbonate.

To confirm tissue loss after spinal cord injury, serial longitudinal sections were stained with Cresyl violet and the lesion volume was measured, and as a result, at week 8 after transplantation, the total lesion volume was significantly reduced in both the ciNSC5 and MSC-treated groups compared to the saline-injected group, and the lesion volume was significantly reduced in the ciNSC5 transplanted group compared to the MSC transplanted group (FIGS. 51A and 51B). In addition, as a result of confirming myelin loss after injury by Luxol-fast blue staining, both the ciNSC5 and MSC-treated groups showed significantly suppressed myelin loss compared to the saline-injected group, and the ciNSC5 transplanted group showed significantly further reduced myelin loss than the MSC transplanted group (FIGS. 51C and 51D).

7-3. Confirmation of effect of reducing gliotic scar formation

To confirm the effect of ciNSC5 on gliotic scar formation, the effect was evaluated by double immunostaining for GFAP and CSPG, and CSPG, which was secreted from the scar densely formed within the lesion area after spinal cord injury, was considered as a chemical obstacle to axon regeneration and functional recovery to confirm the presence of CSPG by staining with CS-56. Specifically, the frozen sections were analyzed by immunohistochemistry using antibodies against GFAP (DAKO, Santa Clara, CA, USA) and CS-56 (CSPG, Millipore, Billerica, MA). For double labeling, FITC or cy3-conjugated secondary antibodies were used (Jackson ImmunoResearch, West Grove, PA), and nuclei were labeled with DAPI (Molecular Probes, Eugene, OR). Immunostaining control studies were performed by replacing the primary antibody with a non-immune, control antibody and preadsorbing the antibody with an excess (10 μg/ml) of each antigen to remove the primary antibody. Fluorescence intensities of a titer or more were quantified and averaged using MetaMorph software (Molecular devices, Sunnyvale, CA). Titer values were at least three backgrounds and the backgrounds were quantified and averaged for the primary antibody missing control.

As a result of confirming the presence of CSPG by staining with CS-56, the lesion area of the spinal cord in the saline-treated group was richly filled with CS-56 and GFAP-positive staining signals, while both CSPG and GFAP intensities were significantly reduced in the MSC and ciNSC5-transplanted groups compared to the saline-treated group (FIGS. 52A to 52C). Even in the results of confirming the GFAP level by Western blot, it was shown that similarly to the immunostaining results, the GFAP level in the ciNSC5-transplanted group was significantly reduced compared to not only the saline group but also the MSC-transplanted group (FIG. 52D). Through this, it was confirmed that ciNSC5 inhibited the gliotic scar formation.

7-4. Confirmation of effect of improving axon regeneration

To determine the effect of ciNSC5 on the growth of corticospinal tract axons, anterograde axonal tracing was performed using BDA, and to determine the scale of extra descending axon pathways leading to the injury site, fluorogold (FG) was injected into the lesion site through the tail at 14 weeks after injury and FG-labeled neurons selected in the supraspinal nuclei were counted. Specifically, on week 8 after MSC or ciNSC5 transplantation, 12 rats (3 rats from the

saline injected group, 5 rats from the MSC transplanted group, and 5 rats from the ciNSC5 transplanted group) were anesthetized with chloral hydrate (500 mg/kg) and the bone above the motor cortex was removed. 10% fluorescent-labeled biotinylated dextran amine (BDA; Molecular Probes, Eugene, OR) was injected into the motor cortex at eight sites (4 sites on each side) in both directions, and at week 2 after BDA injection, rats were perfused with 4% paraformaldehyde in 0.1 M phosphate buffer (pH 7.4). Spinal cords were collected and longitudinally sectioned at 20 mm thickness on a cryomicrotome, and axonal regeneration was quantified under a 20x lens under a fluorescence microscope. Each section was adjusted to a grid with lines spaced at 0, 500, 1000, 1500, 2000, 2500, and 3000 μm, the number of axons was counted at each distance from the rostral edge of the lesion space, and BDA-positive axons were counted in four selected tissues at 100 μm intervals containing the corticospinal tract. The total number of axons in each animal was averaged per group. The total number of axons present in four sections per animal was counted and averaged. In addition, to determine the extent to which extra descending axons reached the rostral lumbar enlargement by retrograde tracing using Fluorogold (FG; Invitrogen, Grand Island, NY, USA), at week 4 after injury, 4% FG was injected into the YJ101 spinal cord fraction and injected into 4 sites (0.5 μl per injection) on each side according to the following stereotaxic coordinates: (1) 0.5 mm medial-lateral (M-L), 0.5 mm dorsalventral (D-V); (2) 1.0 mm M-L, 0.5 mm D-V; (3) 0.5 mm M-L, 2.0 mm D-V; and (4) 1.0 mm M-L, 2.0 mm D-V. The dye was dispersed for at least 2 minutes after each injection, and the animals were sacrificed after one week and histological analysis was performed. It was confirmed that transverse sections of 5-mm spinal cord sections containing the injection site were serially cut to confirm the range of bulk injection including the white matter and the dye did not spread beyond the injected spinal cord sections. Cases that did not meet these criteria were excluded from additional studies. Transverse brain sections (40 μm thick) containing the sensorimotor cortex were cut on a cryostat, and every third section was mounted on a glass slide. Ten sections per rat were analyzed and labeled neurons were counted for each rat and averaged through the entire rats. In addition, the expression pattern of GAP43, known as an axon regeneration factor, was confirmed by Western blot.

As a result of BDA anterograde axon tracing, the saline-transplanted group and the MSC-transplanted group showed very little regeneration, whereas the ciNSC5 transplanted group showed vigorous CST axon regeneration through and beyond the lesion space (FIG. 53). As a result of counting the number of axons, no axons were observed at 1000 μm point from the starting point of the lesion space in the saline transplanted group and the MSC transplanted group, but axons were observed even at 2000 μm point in the ciNSC5 transplanted group. In addition, as the result of Fluorogold retrograde axon tracing, it was shown that the number of FG-labeled neurons in RN, PnC, LVe, MdV, and MdD was significantly higher in the ciNSC5-transplanted group compared to the saline-treated control group, and in the MSC transplanted group, FG-labeled neurons increased only in LVe, and had no effect in other regions (FIG. 54). In addition, as a result of Western blot assay, GAP43 expression in the spinal cord of rats in the ciNSC5 transplanted group was significantly increased compared to the saline transplanted group or the MSC transplanted group (FIG. 55).

### 7-5. Confirmation of differentiation into mature neurons

Six weeks after transplanting ciNSC5 into a chronic spinal cord injury model, immunohistochemical staining was performed using a human-specific STEM121 antibody.

As a result, STEM121-positive cells were observed near the transplant site, which meant that the transplanted ciNSC5 remained in the tissue. As a result of performing double immunostaining to confirm the cell type, it was shown that the transplanted ciNSC5 was double-stained with neuronal markers NeuN, MAP2, and Tuj1 (FIG. 56). Through this, it was suggested that the transplanted ciNSC5 differentiated into mature neurons in spinal cord tissue.

## Claims

1. A composition for inducing oligomerization of C-terminal Binding Proteins (CtBPs), comprising at least one selected from the group consisting of a compound of Chemical Formula 1, a compound of Chemical Formula 2, and a compound of Chemical Formula 3:

[Chemical Formula 1]

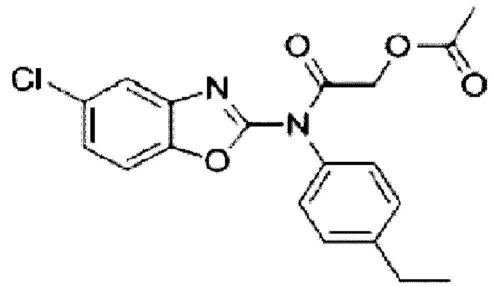

;

[Chemical Formula 2]

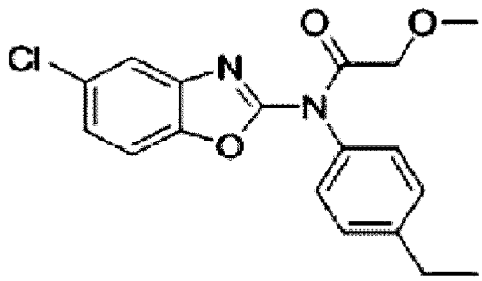

; and

[Chemical Formula 3]

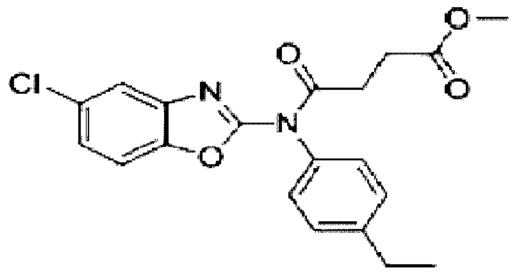

2. The composition for inducing oligomerization of CtBPs of claim 1, wherein the CtBPs is CtBP1 or CtBP2.

3. The composition for inducing oligomerization of CtBPs of claim 1, wherein the compound in Chemical Formula 1 binds to Ser100 of CtBP1.

4. The composition for inducing oligomerization of CtBPs of claim 1, wherein the compound in Chemical Formula 2 binds to Ser100 and Arg266 of CtBP1.

5. The composition for inducing oligomerization of CtBPs of claim 1, wherein the compound in Chemical Formula 3 binds to Phe102, Arg184 and His236 of CtBP1.

6. The composition for inducing oligomerization of CtBPs of claim 1, wherein adult stem cells are differentiated into neural progenitor cells by CtBPs-mediation.

7. A composition for inducing transdifferentiation (direct conversion) of adult stem cells into neural progenitor cells,

comprising at least one compound selected from the group consisting of a compound of Chemical Formula 1, a compound of Chemical Formula 2, and a compound of Chemical Formula 3.

**8.** The composition for inducing transdifferentiation of claim 7, wherein the adult stem cells are mesenchymal stem cells (MSCs).

**9.** The composition for inducing transdifferentiation of claim 8, wherein the mesenchymal stem cells are umbilical cord blood-derived mesenchymal stem cells (UCB-MSC), umbilical cord-derived mesenchymal stem cells (UC-MSC), adipose-derived mesenchymal stem cells (AD-MSC) or bone marrow-derived mesenchymal stem cells (BM-MSC).

**10.** The composition for inducing transdifferentiation of claim 7, wherein the neural progenitor cells are chemical induced neural stem cell 5 (ciNSC5).

**11.** The composition for inducing transdifferentiation of claim 7, wherein the composition increases an expression of Tuj 1, TBR2, MASH1, GAP-43 or p75.

**12.** The composition for inducing transdifferentiation of claim 7, wherein the composition increases an expression of Tuj 1 protein, CD325 (N-cadherin) protein, p75 protein, GAP-43 protein, CD54 protein, CD309 protein, CD56 (NCAM) protein, PSA-NCAM protein, CD29 protein, MASH1 protein or TBR2 protein.

**13.** The composition for inducing transdifferentiation of claim 7, wherein the composition decreases an expression of CD44, CD73 or CD105.

**14.** The composition for inducing transdifferentiation of claim 7, wherein the composition increases a secretion of PIGF, NGF, BDNF, VEGFA, MMP1, MMP2, MMP7, TIMP2, SHH, Notch1, TNFSF12 or IL-16.

**15.** A method for inducing oligomerization of CtBPs, comprising treating a sample *in vitro* with at least one selected from the group consisting of a compound of Chemical Formula 1, a compound of Chemical Formula 2, and a compound of Chemical Formula 3.

**16.** The method for inducing oligomerization of CtBPs of claim 15, wherein the sample is an adult stem cell.

**17.** The method for inducing oligomerization of CtBPs of claim 15, wherein an expression of Tuj1, CD325 (N-cadherin), p75, GAP-43, CD309, CD56 (NCAM), PSA-NCAM, CD29, MASH1 or TBR2 is increased in the sample after treatment compared to before treatment with the compound.

**18.** The method for inducing oligomerization of CtBPs of claim 15, wherein a secretion of PIGF, NGF, BDNF, VEGFA, MMP1, MMP2, MMP7, TIMP2, SHH, Notch1, TNFSF12 or IL-16 is increased in the sample after treatment compared to before treatment with the compound.

**19.** The method for inducing oligomerization of CtBPs of claim 15, wherein an expression of CD44, CD73 or CD105 is decreased in the sample after treatment compared to before treatment with the compound.

**20.** A method for inducing transdifferentiation of adult stem cells into neural progenitor cells, comprising treating adult stem cells with at least one selected from the group consisting of a compound of Chemical Formula 1, a compound of Chemical Formula 2, and a compound of Chemical Formula 3 *in vitro.*

**21.** The method for inducing transdifferentiation of claim 20, wherein an expression of Tuj1, CD325 (N-cadherin), p75, GAP-43, CD54, CD309, CD56(NCAM), PSA-NCAM, CD29, MASH1 or TBR2 is increased in the cells after treatment compared to before treatment with the compound.

**22.** The method for inducing transdifferentiation of claim 20, wherein a secretion of PIGF, NGF, BDNF, VEGFA, MMP1, MMP2, MMP7, TIMP2, SHH, Notch1, TNFSF12 or IL-16 is increased in the cells after treatment compared to before treatment with the compound.

**23.** The method for inducing transdifferentiation of claim 20, wherein an expression of CD44, CD73 or CD105 is decreased in the cells after treatment compared to before treatment with the compound.

**24.** Stem cells treated with at least one compound selected from the group consisting of a compound of Chemical Formula 1, a compound of Chemical Formula 2, and a compound of Chemical Formula 3 below:

[Chemical Formula 1]

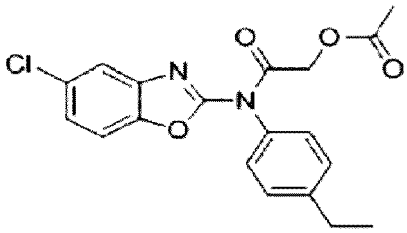

;

[Chemical Formula 2]

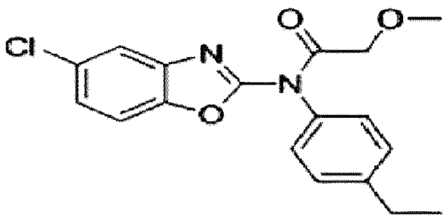

; and

[Chemical Formula 3]

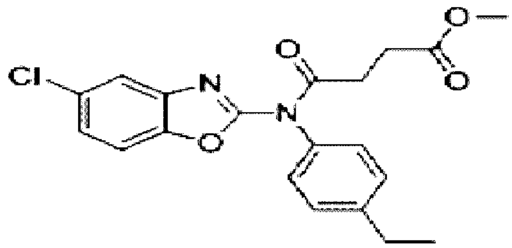

**25.** The stem cells of claim 24, wherein the stem cells treated with the compound are adult stem cells.

**26.** The stem cells of claim 25, wherein the adult stem cells are umbilical cord blood-derived mesenchymal stem cells (UCB-MSC), umbilical cord-derived mesenchymal stem cells (UC-MSC), adipose-derived mesenchymal stem cells (AD-MSC) or bone marrow-derived mesenchymal stem cells (BM-MSC).

**27.** The stem cells of claim 24, wherein at least one selected from the group consisting of the compound of Chemical Formula 1, the compound of Chemical Formula 2, and the compound of Chemical Formula 3 is treated *in vitro* for 3 to 7 days.

**28.** The stem cells of claim 24, wherein the adult stem cells are transdifferentiated (direct converted) into neural progenitor cells by treatment with the compound.

**29.** The stem cells of claim 28, wherein the neural progenitor cells are chemical induced neural stem cell 5 (ciNSC5).

**30.** The stem cells of claim 24, wherein an expression of Tuj1, TBR2, MASH1, GAP-43 or p75 is increased compared

to stem cells untreated with the compound.

**31.** The stem cells of claim 24, wherein an expression of Tuj1 protein, CD325 (N-cadherin) protein, p75 protein, GAP-43 protein, CD54 protein, CD309 protein, CD56 (NCAM) protein, PSA-NCAM protein, CD29 protein, MASH1 protein or TBR2 protein is increased compared to stem cells untreated with the compound.

**32.** The stem cells of claim 24, wherein an expression of CD44, CD73 or CD105 is decreased compared to stem cells untreated with the compound.

**33.** The stem cells of claim 24, wherein a secretion of PlGF, NGF, BDNF, VEGFA, MMP1, MMP2, MMP7, TIMP2, SHH, Notch1, TNFSF12 or IL-16 is increased compared to stem cells untreated with the compound.

**34.** The stem cells of claim 24, wherein the expression of HES1, Sox2 or OCT4 is increased compared to stem cells untreated with the compound.

**35.** The stem cells of claim 24, wherein oligomerization of C-terminal Binding Proteins (CtBPs) is induced by treatment with the compound.

**36.** The stem cells of claim 24, wherein MAP2, NSE, NeuN, doublecortin, Neurogenic differentiation 1 (NeuroD1), Nestin, Mussashi 1 or GFAP is not expressed.

**37.** The stem cells of claim 24, wherein the stem cells differentiate into dopaminergic neurons, neurons, or mature neurons.

**38.** A pharmaceutical composition for preventing or treating nerve injury diseases, comprising the stem cells of claim 24, a stem cell culture or a suspension culture as an active ingredient.

**39.** The pharmaceutical composition for preventing or treating nerve injury diseases of claim 38, wherein the nerve injury disease is injury to the central or peripheral nervous system, or a neurodegenerative disease.

**40.** The pharmaceutical composition for preventing or treating nerve injury diseases of claim 39, wherein the injury to the central or peripheral nervous system is spinal cord injury (SCI), traumatic brain injury (TBI), peripheral nerve injury, stroke, or brain cancer.

**41.** The pharmaceutical composition for preventing or treating nerve injury diseases of claim 39, wherein the neurodegenerative disease is Alzheimer's disease, Parkinson's disease, amyotrophic lateral sclerosis (ALS), Huntington's disease (HD), multiple sclerosis (MS), or multiple system atrophy.

**42.** A use of a compound of Chemical Formula 1, a compound of Chemical Formula 2, or a compound of Chemical Formula 3, for use in inducing oligomerization of CtBPs.

**43.** A use of a compound of Chemical Formula 1, a compound of Chemical Formula 2, or a compound of Chemical Formula 3, for use in inducing transdifferentiation.

**44.** A use for preventing or treating nerve injury diseases by stem cells treated with at least one compound selected from the group consisting of a compound of Chemical Formula 1, a compound of Chemical Formula 2, and a compound of Chemical Formula 3.

**45.** A method for treating nerve injury diseases, comprising transplanting stem cells treated with at least one compound selected from the group consisting of a compound of Chemical Formula 1, a compound of Chemical Formula 2, and a compound of Chemical Formula 3 into a subject suffering from a nerve injury disease.

[Fig.1]

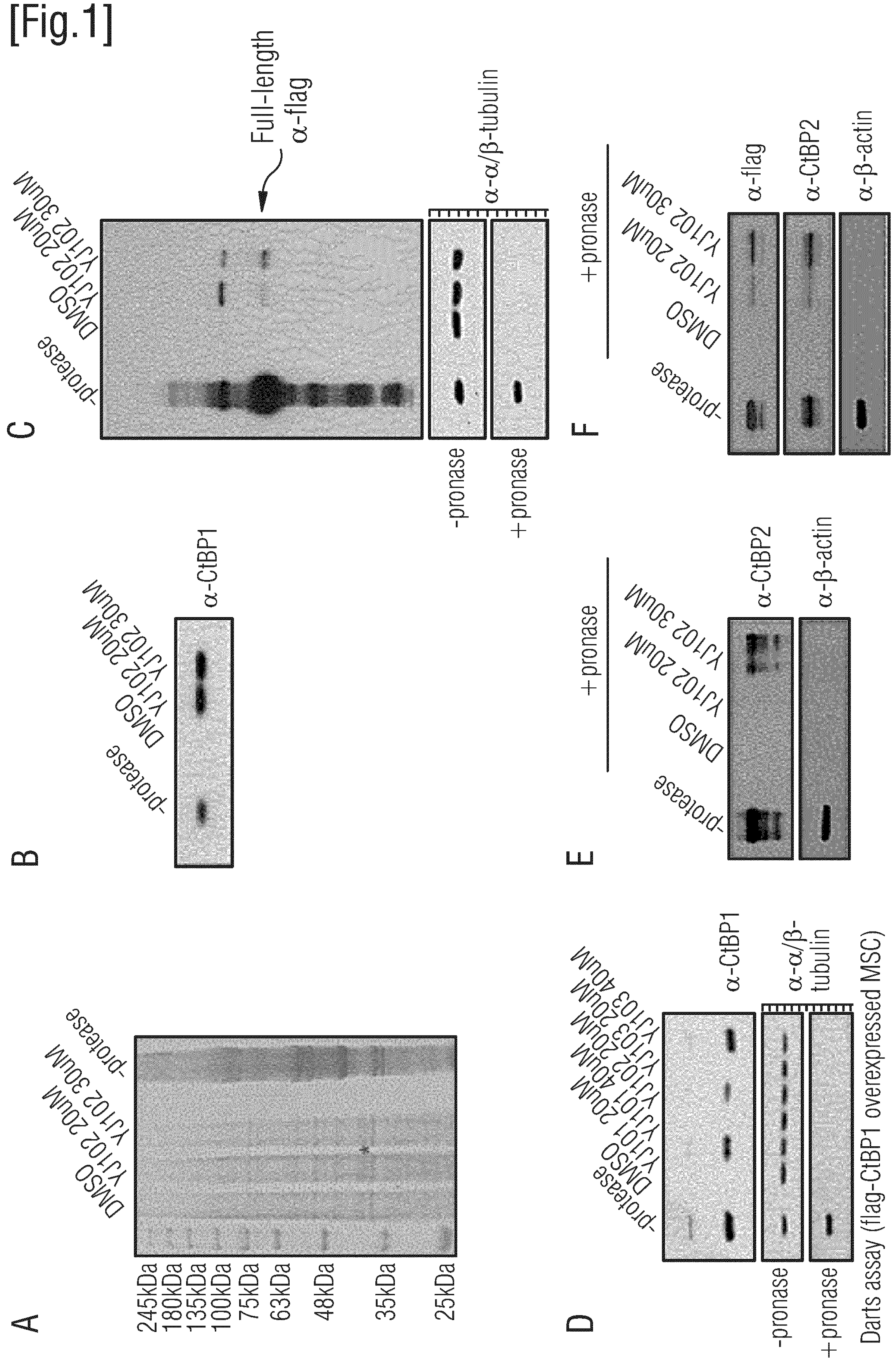
A
245kDa
180kDa
135kDa
100kDa
75kDa
63kDa
48kDa
35kDa
25kDa
-protease
DMSO
YJ102 20uM
YJ102 30uM
B
-protease
DMSO
YJ102 20uM
YJ102 30uM
α-CtBP1
C
-protease
DMSO
YJ102 20uM
YJ102 30uM
Full-length
α-flag
α-α/β-tubulin
-pronase
+pronase
D
-protease
DMSO
YJ101 20uM
YJ101 40uM
YJ102 20uM
YJ103 20uM
YJ103 40uM
α-CtBP1
α-α/β-
tubulin
-pronase
+pronase
Darts assay (flag-CtBP1 overexpressed MSC)
E
+pronase
-protease
DMSO
YJ102 20uM
YJ102 30uM
α-CtBP2
α-β-actin
F
+pronase
-protease
DMSO
YJ102 20uM
YJ102 30uM
α-flag
α-CtBP2
α-β-actin

FIG. 2

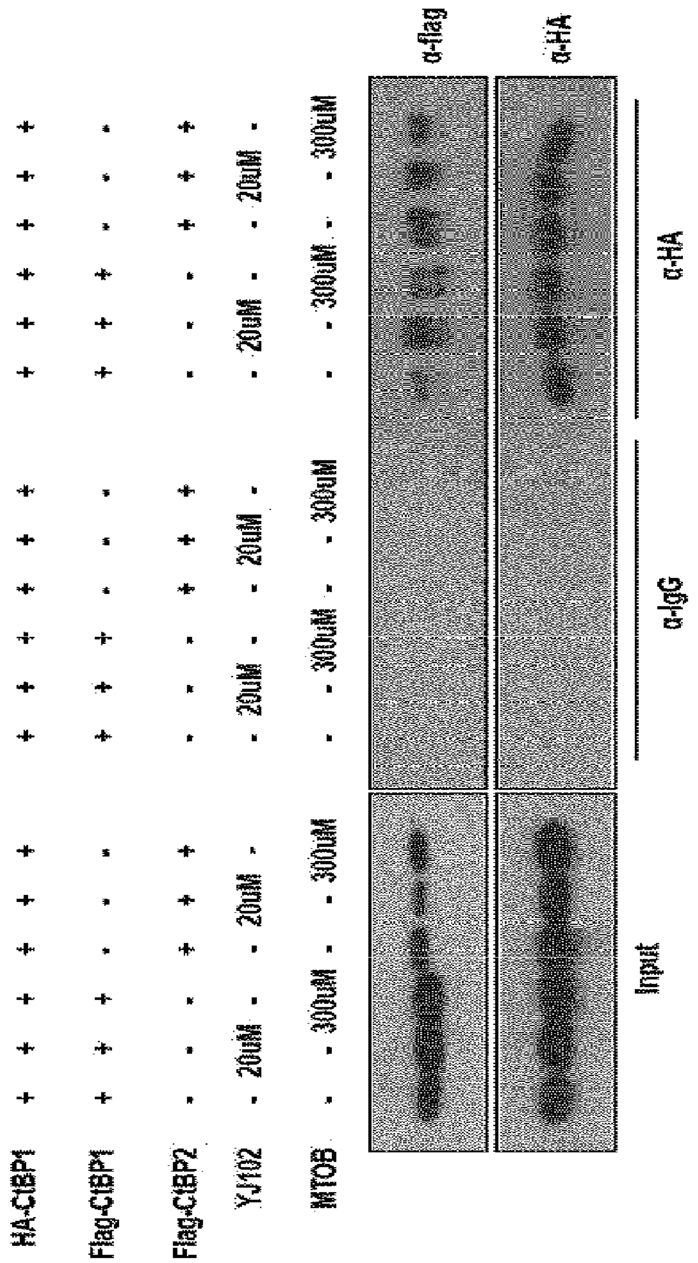

HA-CtBP1
Flag-CtBP1
Flag-CtBP2
YJ102
MTOB
20uM
300uM
Input
α-IgG
α-HA
α-flag

# [FIG.3]

A

YJ101-CTBP1

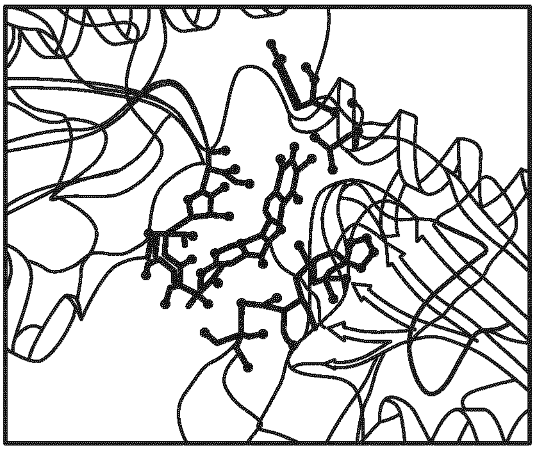

Docking score (ΔG)=4.980 kcal/ mol

YJ102-CTBP1

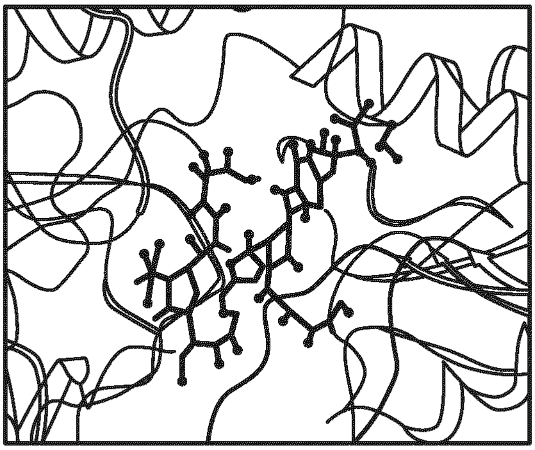

Docking score (ΔG)=-5.458 kcal/ mol
NADH Docking score (ΔG) =-8 kcal/ mol

YJ103-CTBP1

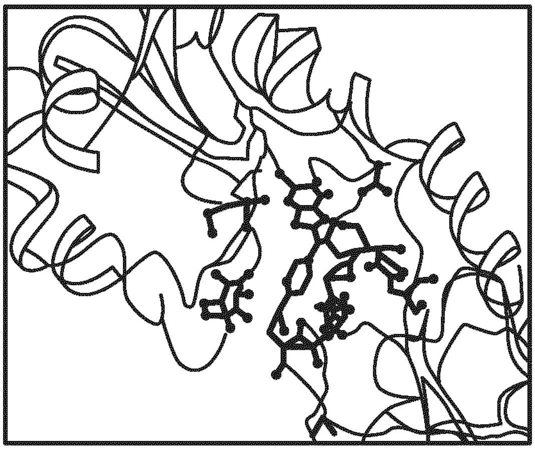

Docking score (ΔG)=3.767 kcal/ mol

| Ligand | Docking score (kcal/mol) |
|---|---|
| YJ101 | -4.980 |
| YJ102 | -5.458 |
| YJ103 | -3.767 |

[FIG.3]

B

| | | |
|---|---|---|
| BA | BB | BC |

[FIG.3]

BA

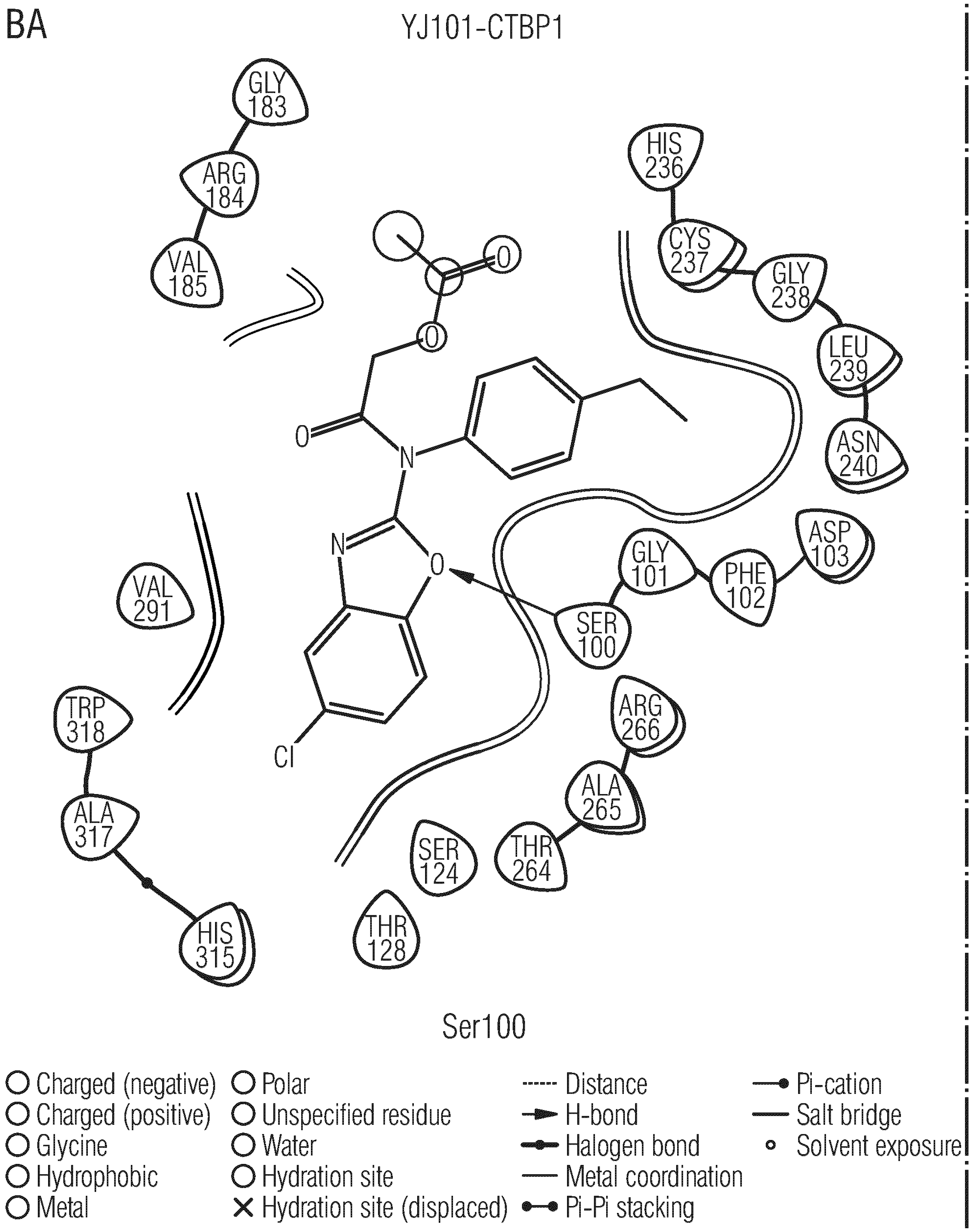

[FIG.3]

BB

YJ102-CTBP1

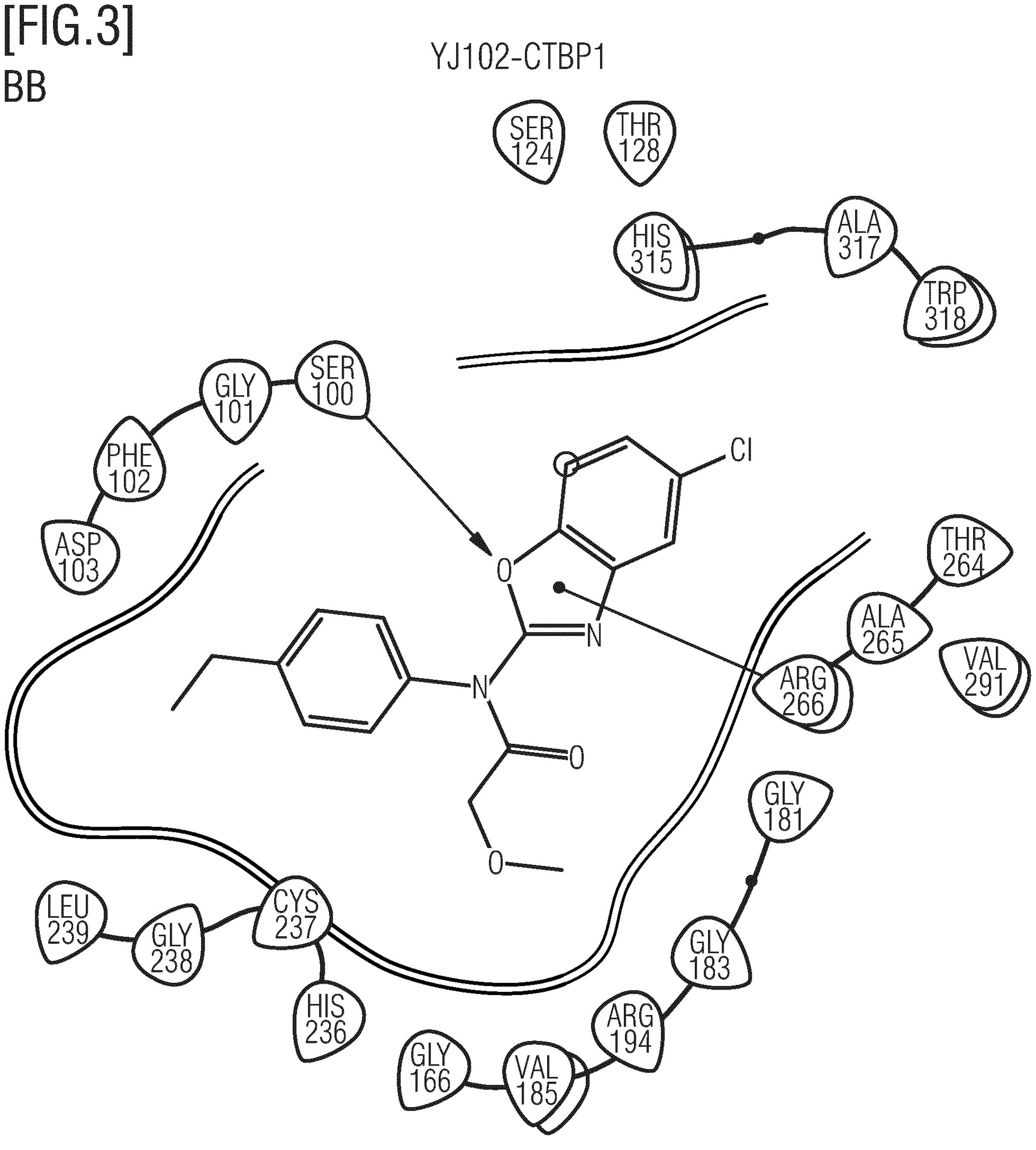


Ser110, Arg266

| | | | |
|---|---|---|---|
| Charged (negative) | Polar | Distance | Pi-cation |
| Charged (positive) | Unspecified residue | H-bond | Salt bridge |
| Glycine | Water | Halogen bond | Solvent exposure |
| Hydrophobic | Hydration site | Metal coordination | |
| Metal | Hydration site (displaced) | Pi-Pi stacking | |

[FIG.3]

BC

YJ103-CTBP1

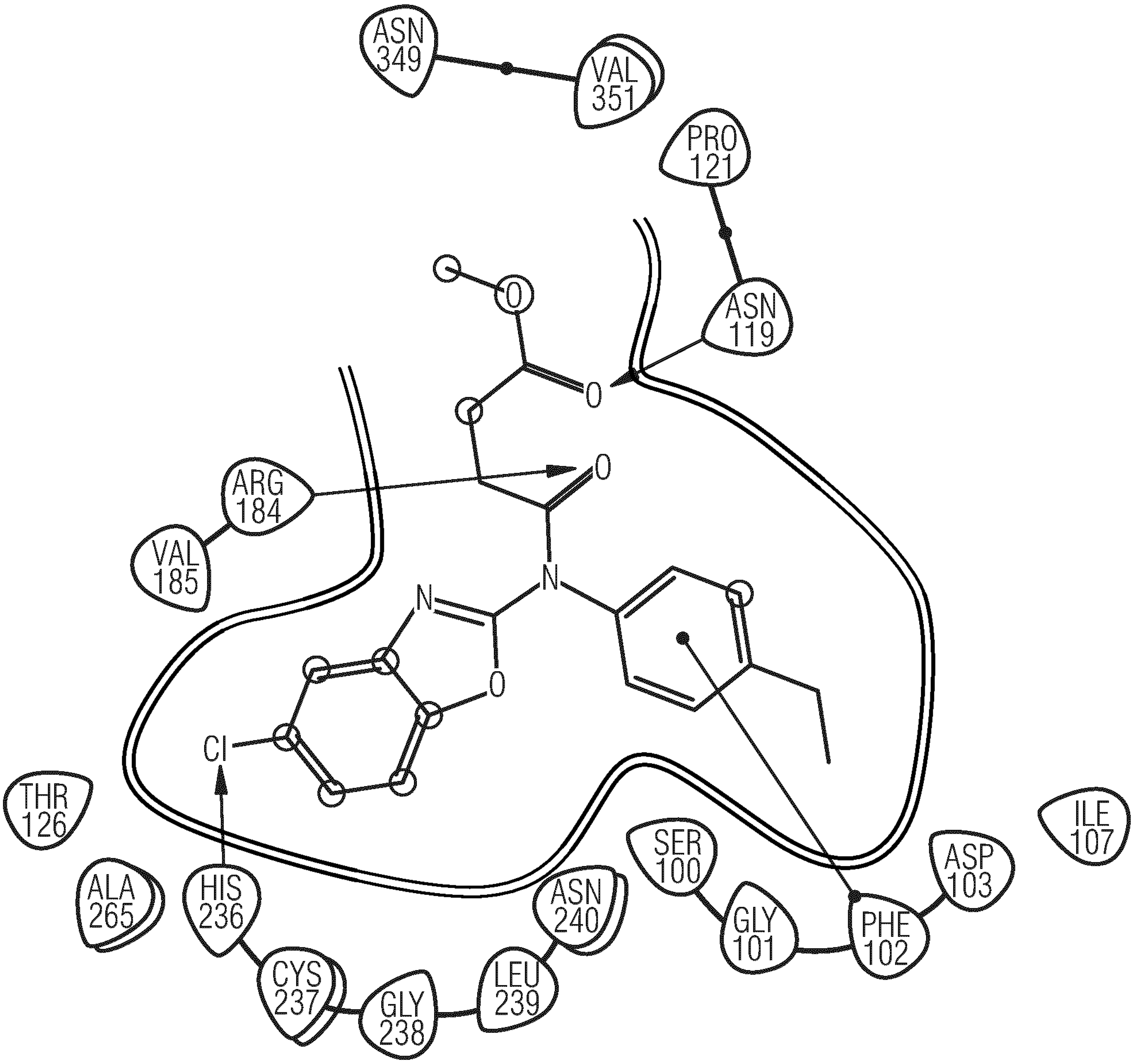


Phe102, Arg184, His236

Charged (negative)
Charged (positive)
Glycine
Hydrophobic
Metal
Polar
Unspecified residue
Water
Hydration site
Hydration site (displaced)
Distance
H-bond
Halogen bond
Metal coordination
Pi-Pi stacking
Pi-cation
Salt bridge
Solvent exposure

FIG. 4

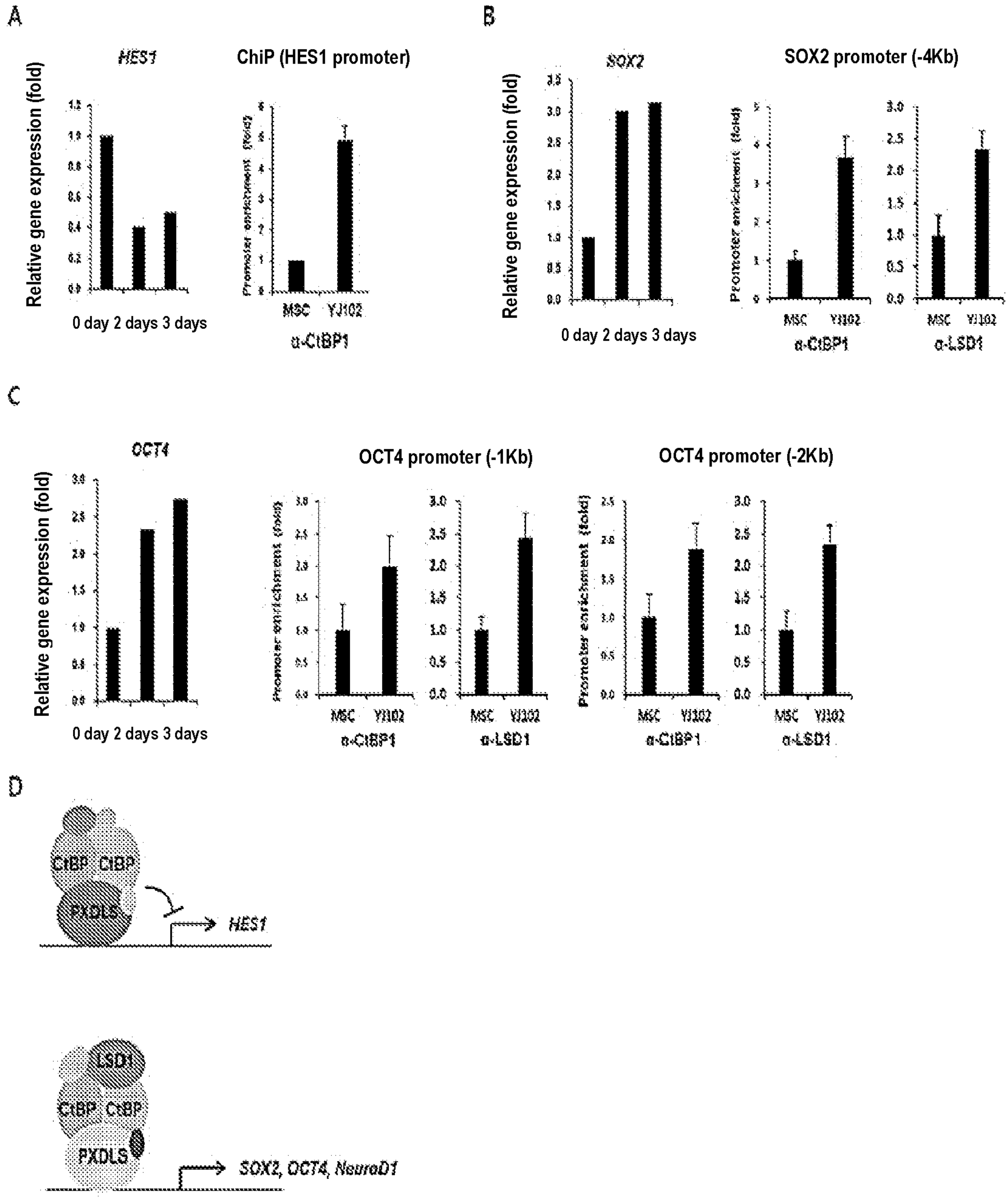
A
HES1
ChiP (HES1 promoter)
Relative gene expression (fold)
0 day 2 days 3 days
MSC YJ102
α-CtBP1
B
SOX2
SOX2 promoter (-4Kb)
Relative gene expression (fold)
0 day 2 days 3 days
Promoter enrichment (fold)
MSC YJ102
α-CtBP1
α-LSD1
C
OCT4
OCT4 promoter (-1Kb)
OCT4 promoter (-2Kb)
Relative gene expression (fold)
0 day 2 days 3 days
α-CtBP1
α-LSD1
D
CtBP CtBP
PXDLS
HES1
LSD1
SOX2, OCT4, NeuroD1

FIG. 5

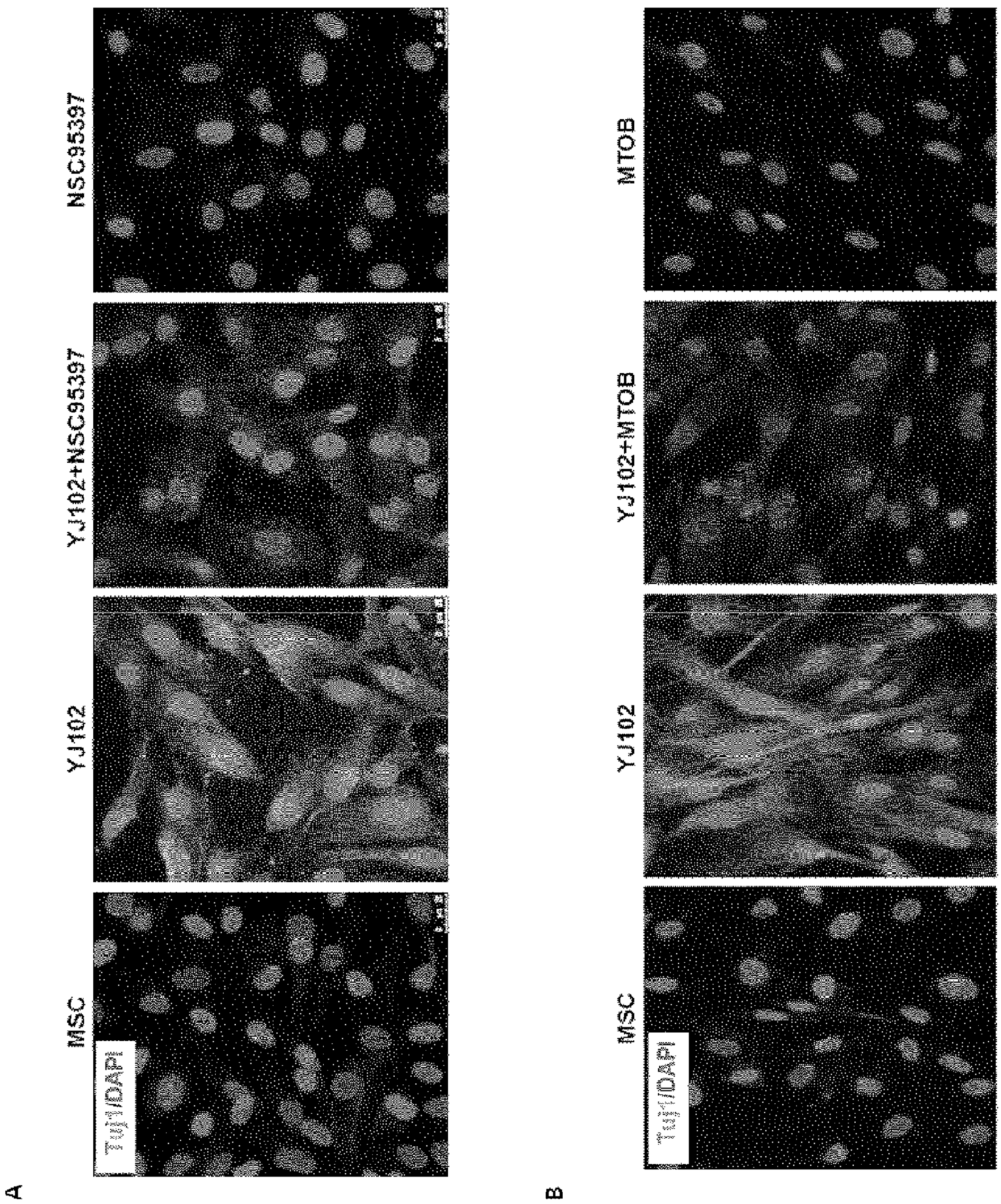

A
MSC
Tuj1/DAPI
YJ102
YJ102+NSC95397
NSC95397
B
MSC
Tuj1/DAPI
YJ102
YJ102+MTOB
MTOB

FIG. 6

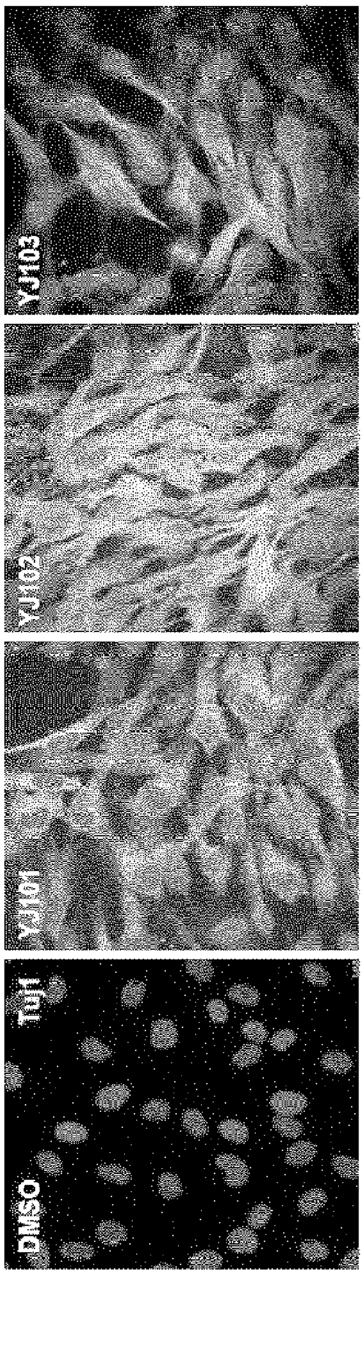

FIG. 7

A

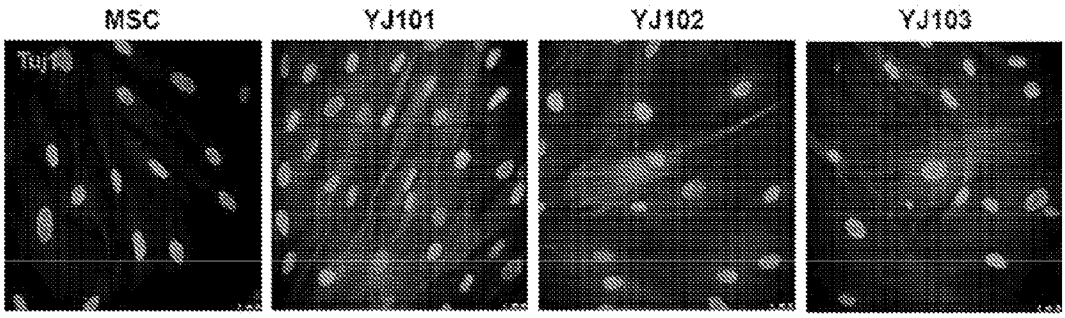


**Adipose-derived mesenchymal stem cell, Gibco**

B

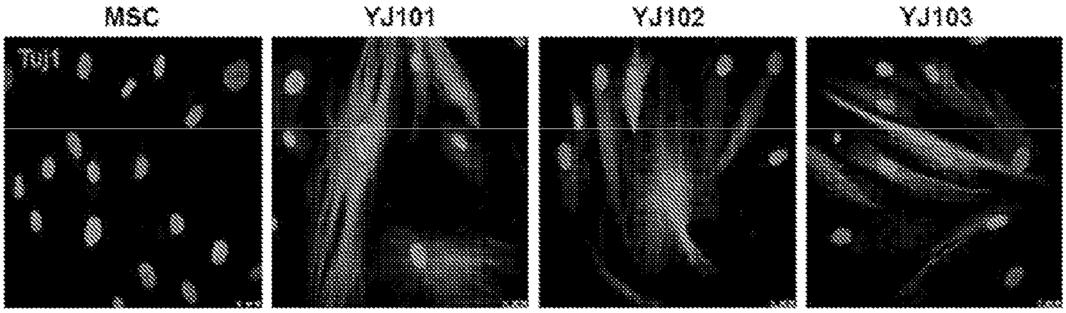


**Bone marrow-derived mesenchymal stem cell, ATCC**

C

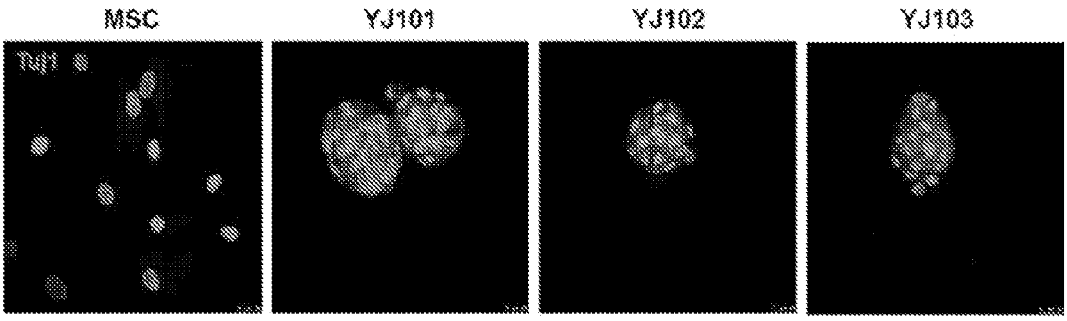


**Umbilical cord-derived mesenchymal stem cell, Promo cell**

FIG. 8

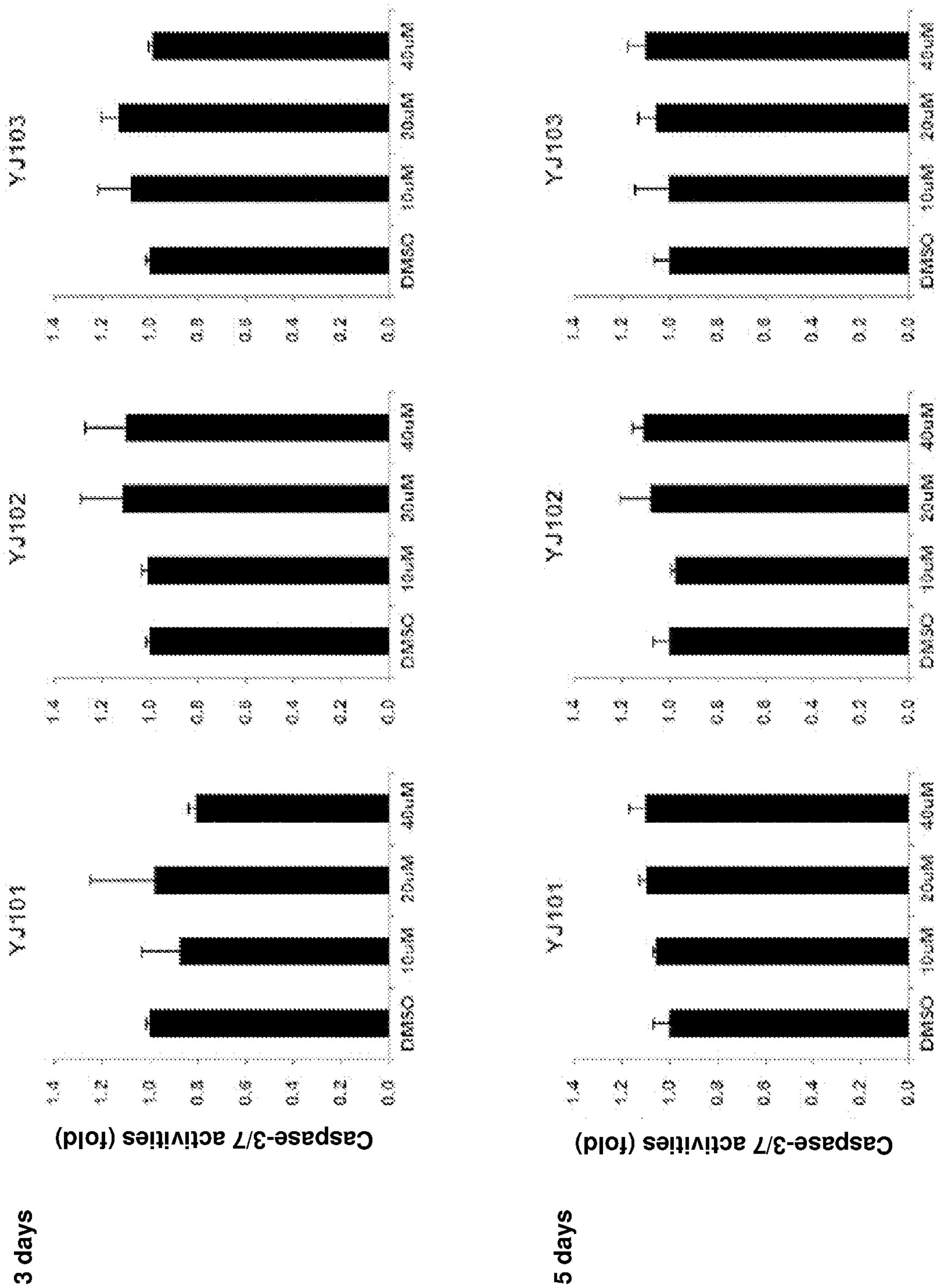
3 days
5 days
Caspase-3/7 activities (fold)
Caspase-3/7 activities (fold)
YJ101
YJ102
YJ103
YJ101
YJ102
YJ103
DMSO
10uM
20uM
40uM

FIG. 9

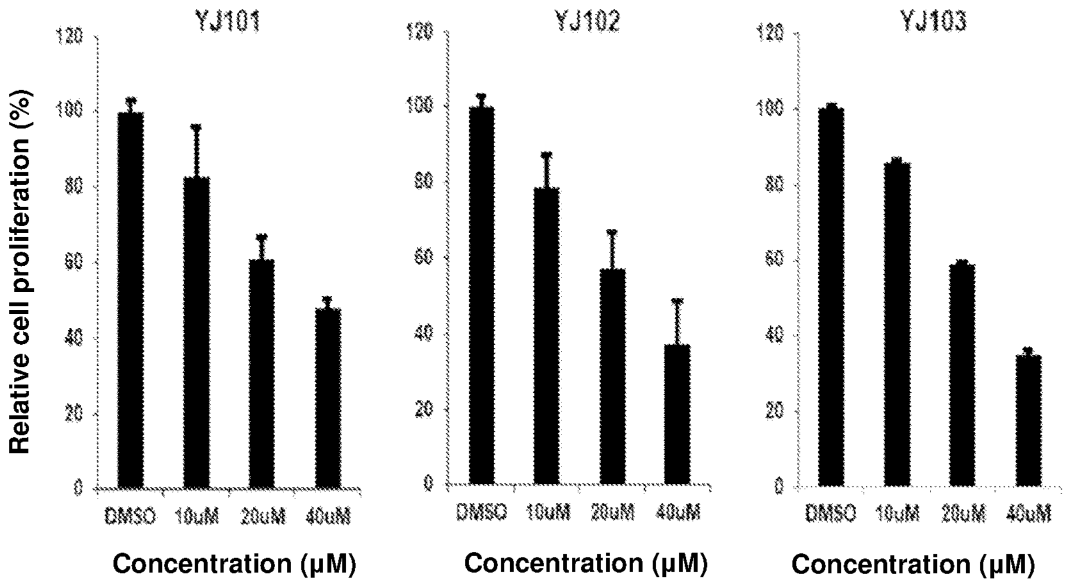
YJ101
YJ102
YJ103
Relative cell proliferation (%)
0
20
40
60
80
100
120
DMSO
10uM
20uM
40uM
Concentration (μM)

FIG. 10

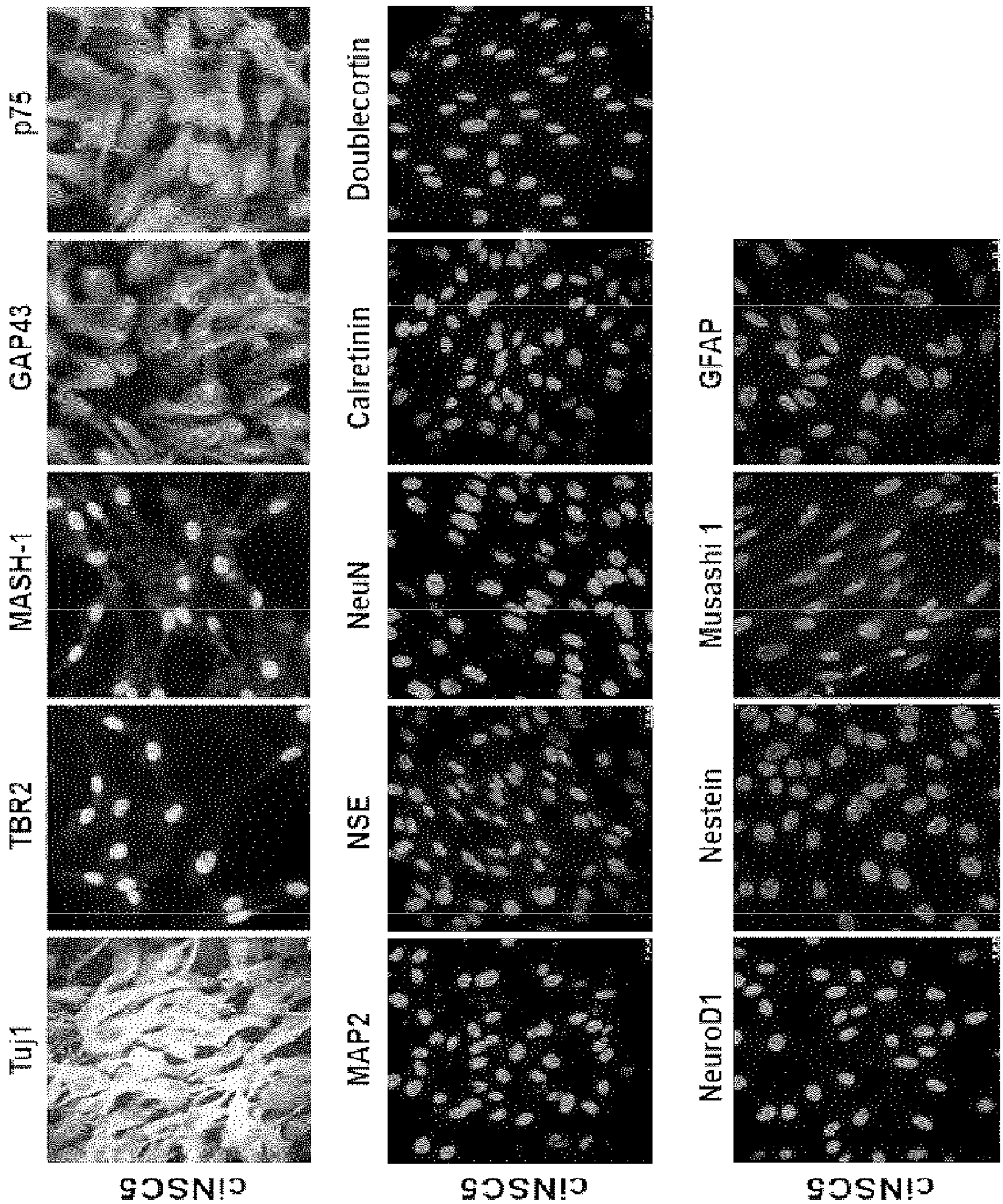
p75
GAP43
MASH-1
TBR2
Tuj1
ciNSC5
Doublecortin
Calretinin
NeuN
NSE
MAP2
ciNSC5
GFAP
Musashi 1
Nestein
NeuroD1
ciNSC5

FIG. 11

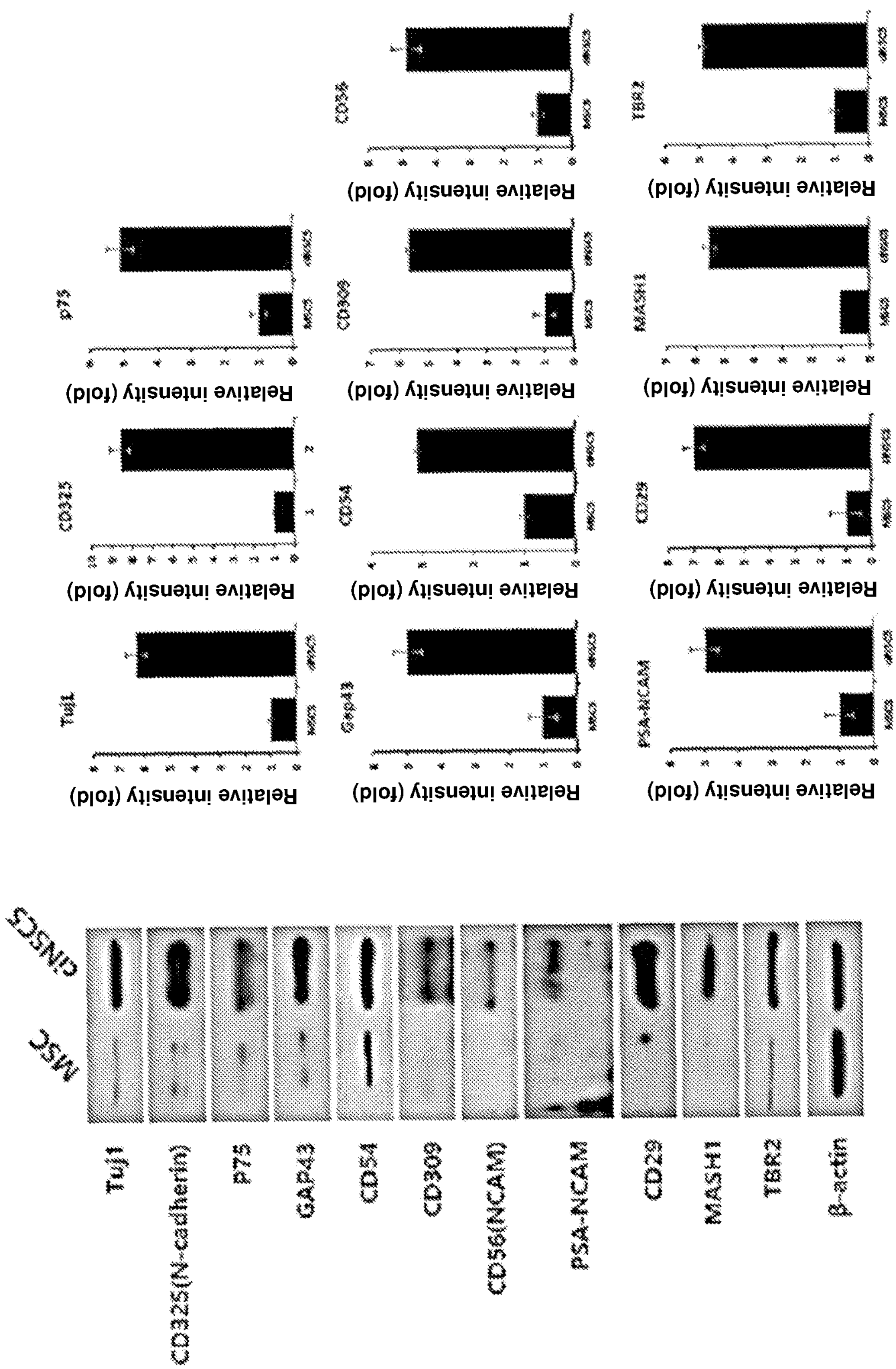
Tuj1
CD325(N-cadherin)
P75
GAP43
CD54
CD309
CD56(NCAM)
PSA-NCAM
CD29
MASH1
TBR2
β-actin
MSC
ciNSCs
Relative intensity (fold)
CD325
p75
Gap43
CD54
CD309
CD56
PSA-NCAM
CD29
MASH1
TBR2

FIG. 12

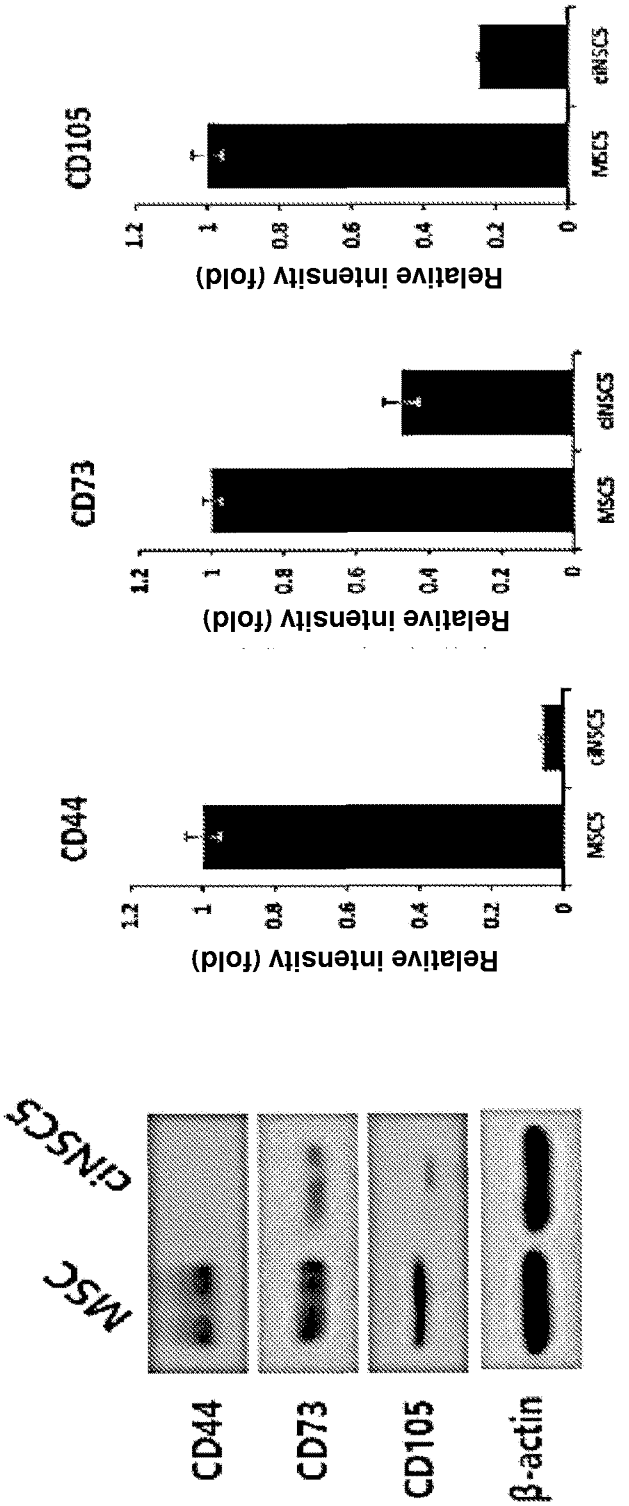

CD44
CD73
CD105
β-actin
MSC
ciNSCs
Relative intensity (fold)
1.2
1
0.8
0.6
0.4
0.2
0
MSCs
ciNSCs

FIG. 13

| Marker proteins | Rates of changes compared to MSC |
|---|---|
| Tuj1 | 6-fold or more increase |
| CD325 (N-cadherin) | 8-fold or more increase |
| P75 | 5-fold or more increase |
| GAP43 | 5-fold or more increase |
| CD54 | 3-fold or more increase |
| CD309 | 5-fold or more increase |
| CD56 (NCAM) | 4-fold or more increase |
| PSA-NCAM | 5-fold or more increase |
| CD29 | 6-fold or more increase |
| MASH1 | 6-fold or more increase |
| TBR2 | 4-fold or more increase |
| CD44 | Decrease by 90% or more |
| CD73 | Decrease by 50% or more |
| CD105 | Decrease by 80% or more |

FIG. 14

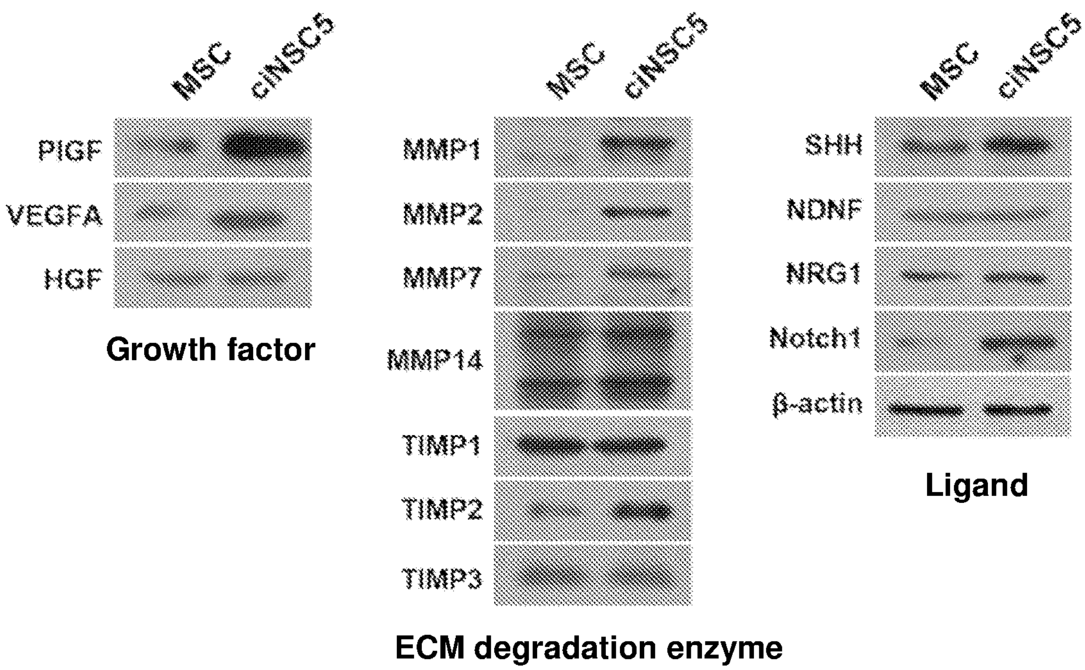

FIG. 15

| Category | Increase | Decrease | No change |
|---|---|---|---|
| Growth factor | PIGF<br>VEGFA | | HGF |
| ECM degradation enzyme | MMP1<br>MMP2<br>MMP7<br>TIMP2 | TIMP3 | TIMP1<br>MMP14 |
| Ligand<br>(differentiation) | SHH | | |
| Ligand<br>(neurogenesis, axon regeneration) | | | NDNF<br>NRG1 |
| Etc. | Notch1 | | |

FIG. 16

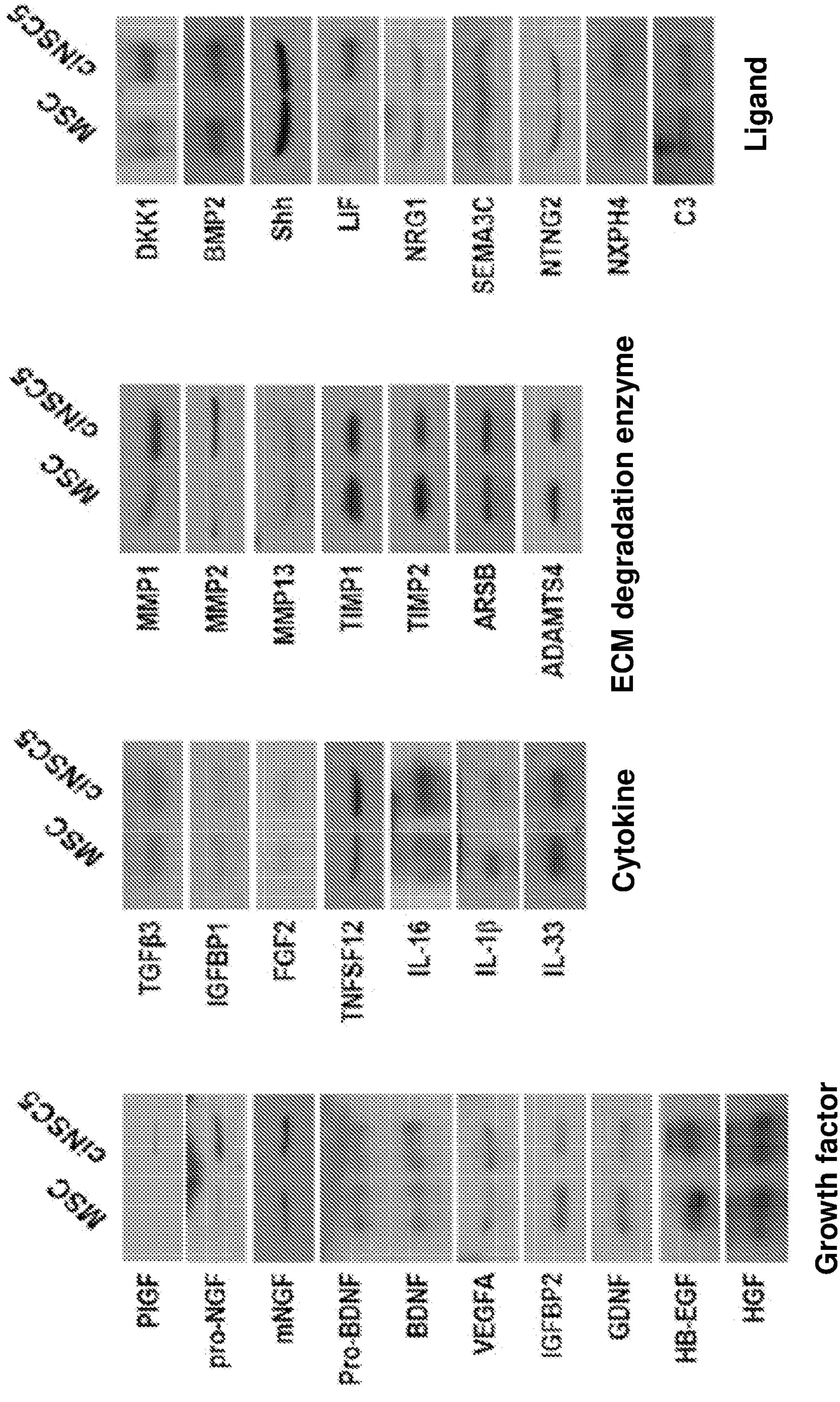
MSC
ciNSCs
DKK1
BMP2
Shh
LIF
NRG1
SEMA3C
NTNG2
NXPH4
C3
Ligand
MMP1
MMP2
MMP13
TIMP1
TIMP2
ARSB
ADAMTS4
ECM degradation enzyme
TGFβ3
IGFBP1
FGF2
TNFSF12
IL-16
IL-1β
IL-33
Cytokine
PlGF
pro-NGF
mNGF
Pro-BDNF
BDNF
VEGFA
IGFBP2
GDNF
HB-EGF
HGF
Growth factor

FIG. 17

| Category | Increase | Decrease | No change |
|---|---|---|---|
| Growth factor | PIGF<br>NGF<br>mBNDF<br>VEGF-A | IGFBP2<br>GDNF | HB-EGF<br>HGF<br>TGFβ3<br>IGFBP1<br>FGF2 |
| Cytokine | TNFSF12<br>IL-16 | IL-1β | IL-33 |
| Degradation enzyme | MMP1<br>MMP2 | TIMP1<br>TIMP2 | ARSB<br>ADAMTS4<br>MMP13 |
| Ligand<br>(differentiation) | DKK1 | | Wnt3<br>BMP2<br>SHH |
| Ligand<br>(neurogenesis, axon regeneration) | LIF | | NRG1<br>SEMA3C<br>NTNG2<br>NXPH4 |
| Etc. | | | C3 |

Changed factors in a medium after culturing ciNSC5 for 2 days

FIG. 18

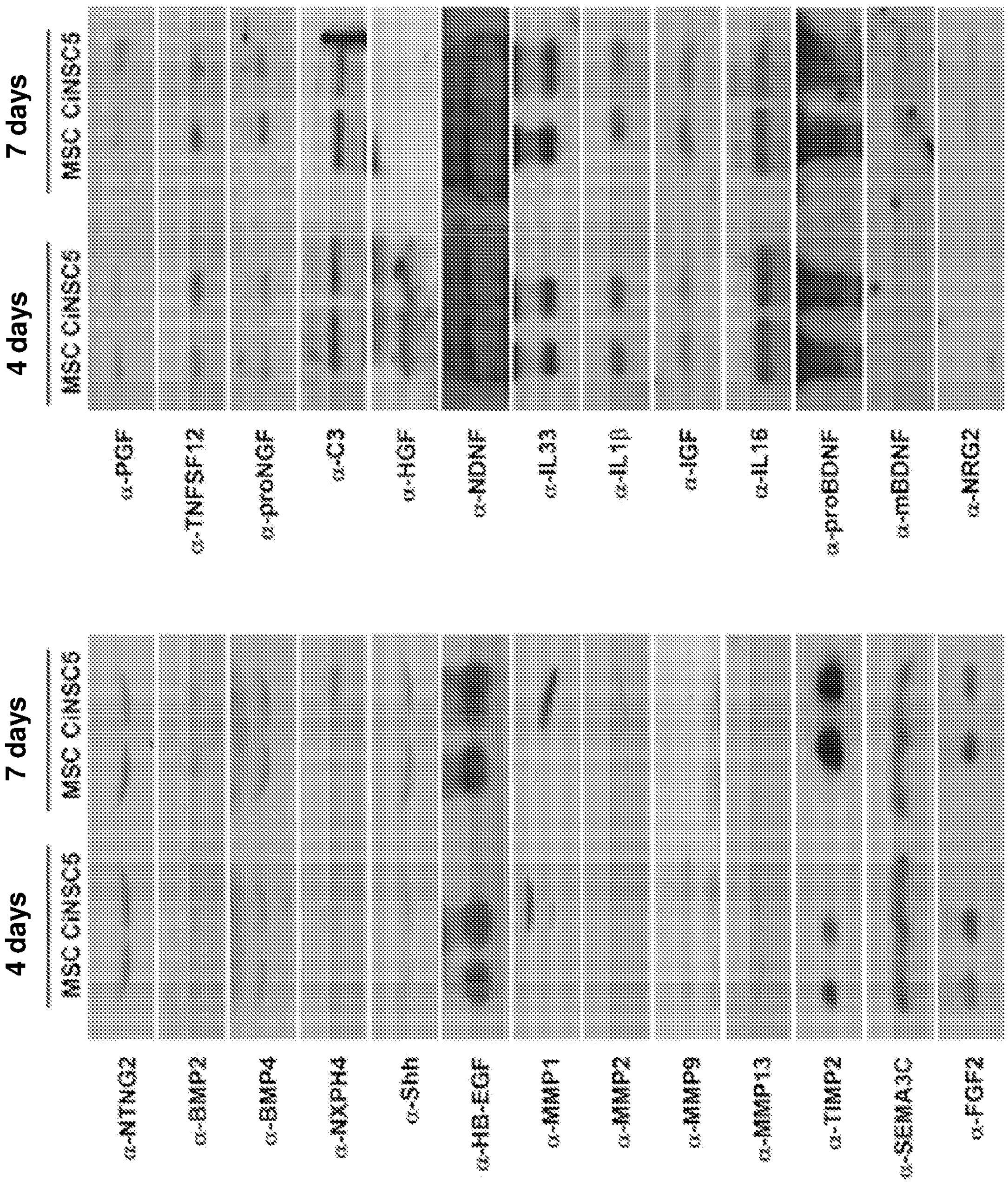
4 days
7 days
MSC CiNSC5
MSC CiNSC5
α-PGF
α-TNFSF12
α-proNGF
α-C3
α-HGF
α-NDNF
α-IL33
α-IL1β
α-IGF
α-IL16
α-proBDNF
α-mBDNF
α-NRG2
4 days
7 days
MSC CiNSC5
MSC CiNSC5
α-NTNG2
α-BMP2
α-BMP4
α-NXPH4
α-Shh
α-HB-EGF
α-MMP1
α-MMP2
α-MMP9
α-MMP13
α-TIMP2
α-SEMA3C
α-FGF2

FIG. 19

| | Category | Increase | Decrease | No change |
|---|---|---|---|---|
| P2 | Growth factor | proNGF<br>mBNDF<br>FGF2 | | HB-EGF<br>NDNF<br>IGF<br>HGF |
| | Cytokine | TNFSF12 | | IL33<br>IL1β<br>IL16 |
| | Degradation enzyme | MMP1<br>MMP9 | TIMP2 | MMP13 |
| | Ligand (differentiation) | | | BMP2<br>BMP4<br>SHH |
| | Ligand (neurogenesis, axon regeneration) | SEMA3C | | NTNG2 |
| | Etc. | | | C3 |
| P4 | Category | Increase | Decrease | No change |
| | Growth factor | PIGF | | HB-EGF<br>proNGF<br>IGF<br>FGF2 |
| | Cytokine | | IL1β | IL33<br>IL16<br>TNFSF12 |
| | Degradation enzyme | MMP1<br>MMP2 | TIMP2 | MMP13 |
| | Ligand (differentiation) | | BMP2 | BMP4<br>SHH |
| | Ligand (neurogenesis, axon regeneration) | NXPH4 | | SEMA3C<br>NTNG2<br>NRG2 |
| | Etc. | | | C3 |

FIG. 20

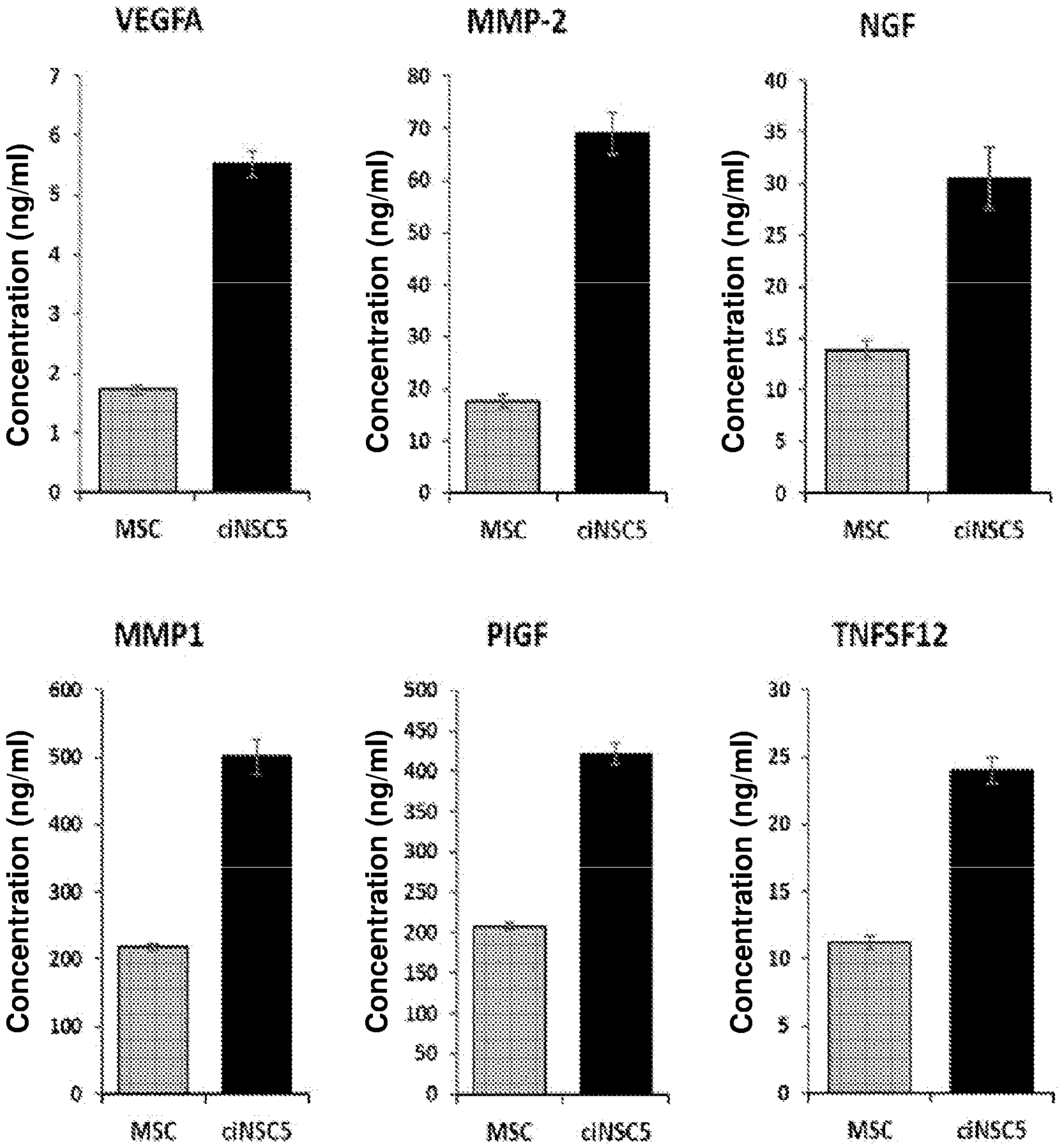
VEGFA
MMP-2
NGF
MMP1
PIGF
TNFSF12
Concentration (ng/ml)
MSC
ciNSC5

FIG. 21

| Category | Cell types | Amounts of protein (ng/ml) | Increased ratios compared to MSC |
|---|---|---|---|
| VEGFA | MSC | 1.72 | 4.0-fold |
| | ciNSC5 | 5.52 | |
| MMP-2 | MSC | 17.72 | 3.9-fold |
| | ciNSC5 | 69.06 | |
| NGF | MSC | 13.86 | 2.2-fold |
| | ciNSC5 | 30.55 | |
| MMP-1 | MSC | 217.73 | 2.3-fold |
| | ciNSC5 | 500.40 | |
| PIGF | MSC | 207.95 | 2.0-fold |
| | ciNSC5 | 420.85 | |
| TNFSF12 | MSC | 11.22 | 2.1-fold |
| | ciNSC5 | 24.01 | |

FIG. 22

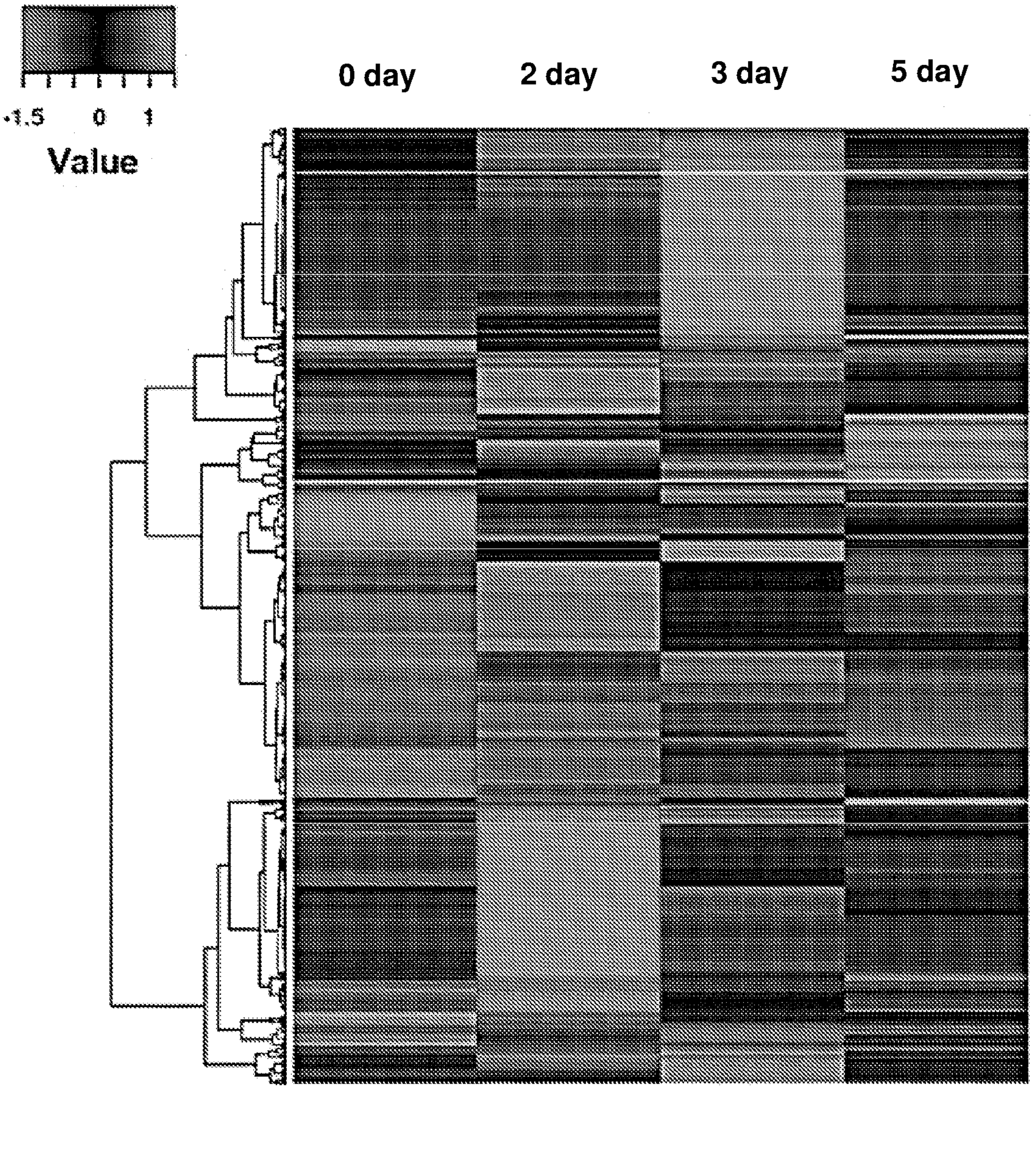

FIG. 23

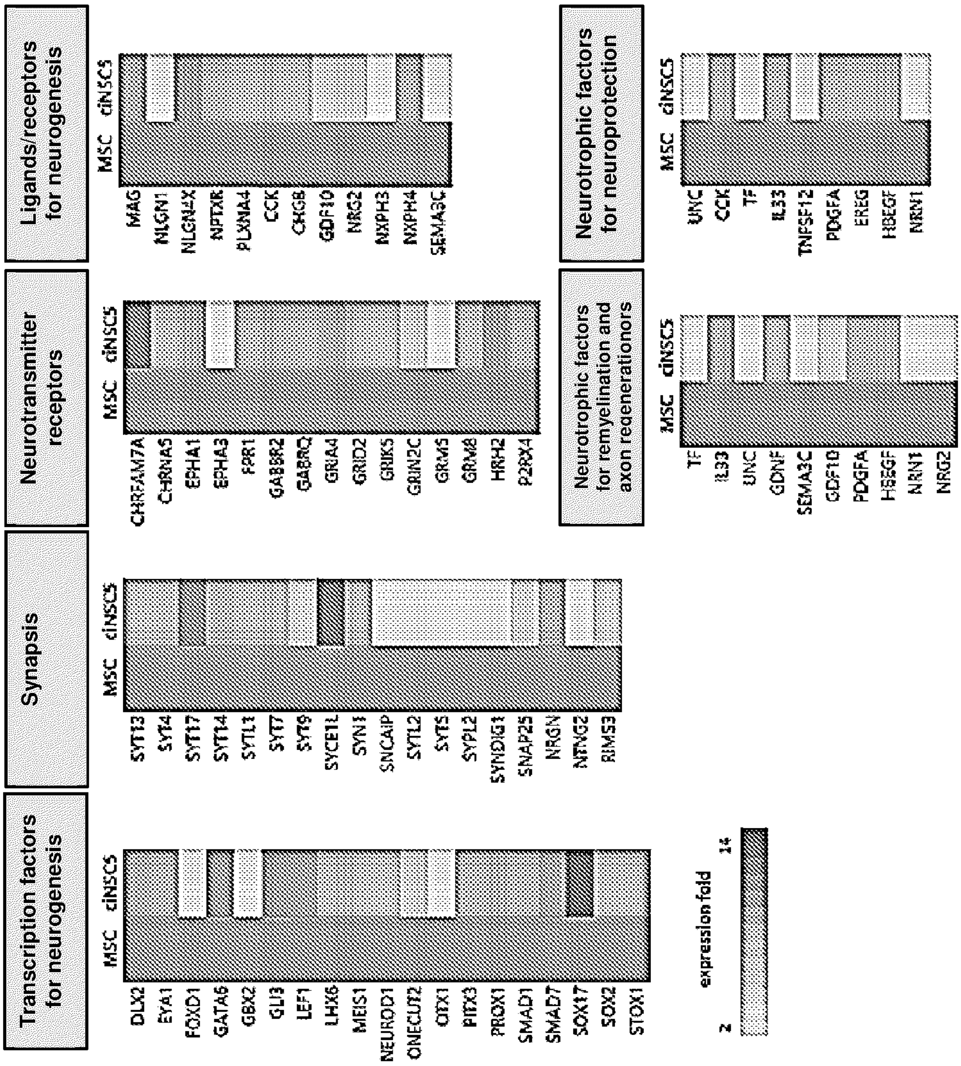


FIG. 24

| GO description | P-value |
|---|---|
| Nervous system development | 5.96E-14 |
| Brain development | 1.59E-09 |
| Neuron differentiation | 4.16E-10 |
| Axon induction | 3.28E-06 |
| Axonogenesis | 5.03E-06 |
| Regulation of neuron apoptotic process | 1.42E-03 |
| Regulation of neural projection development | 6.68E-04 |
| Synapse organization | 4.71E-06 |

FIG. 25

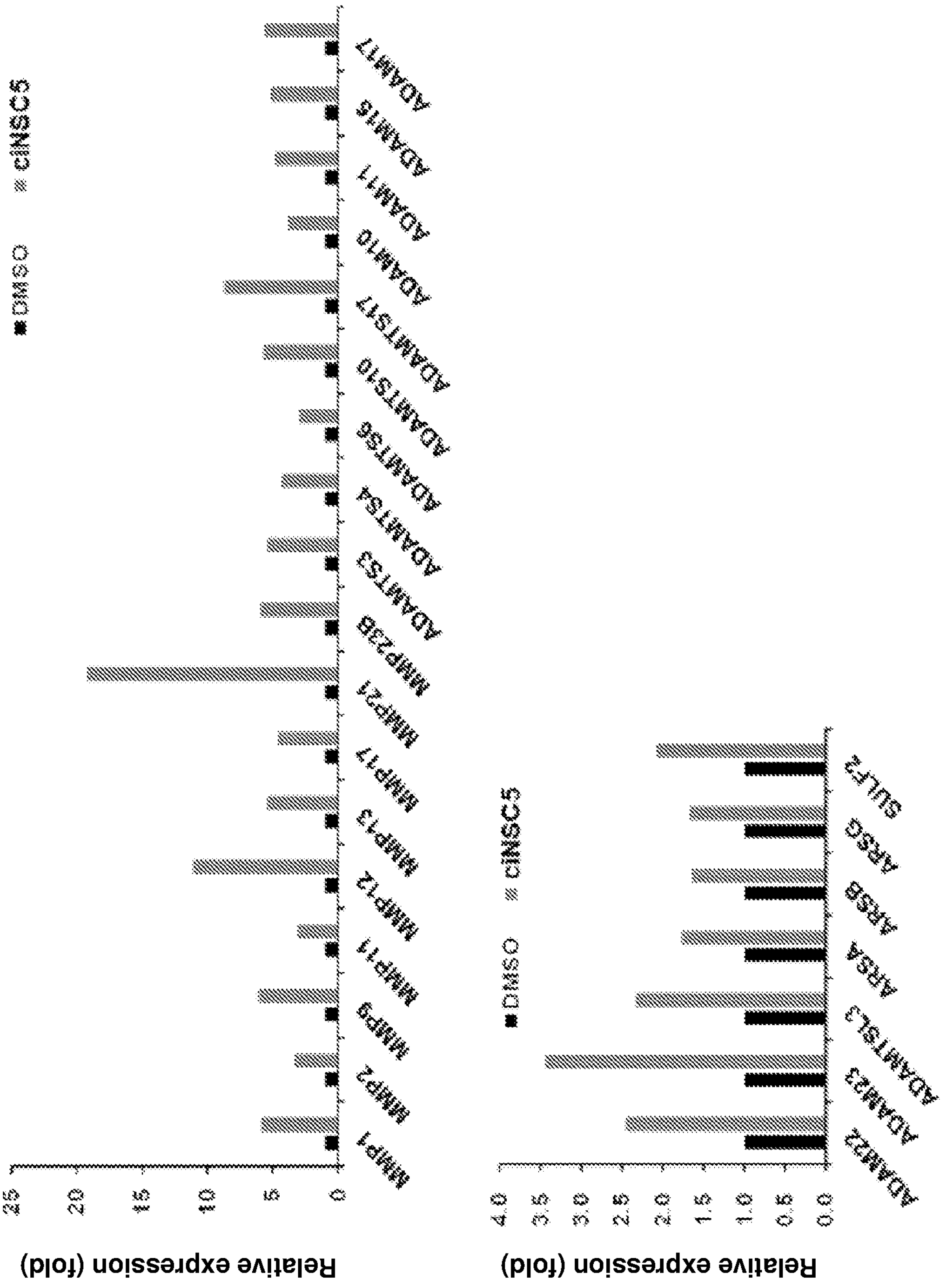
DMSO
ciNSC5
Relative expression (fold)
0
5
10
15
20
25
MMP1
MMP2
MMP9
MMP-11
MMP-12
MMP-13
MMP-17
MMP21
MMP23B
ADAMTS3
ADAMTS4
ADAMTS6
ADAMTS10
ADAMTS17
ADAM10
ADAM11
ADAM16
ADAM17
DMSO
ciNSC5
Relative expression (fold)
0.0
0.5
1.0
1.5
2.0
2.5
3.0
3.5
4.0
ADAM22
ADAM23
ADAMTSL3
ARSA
ARSB
ARSG
SULF2

FIG. 26

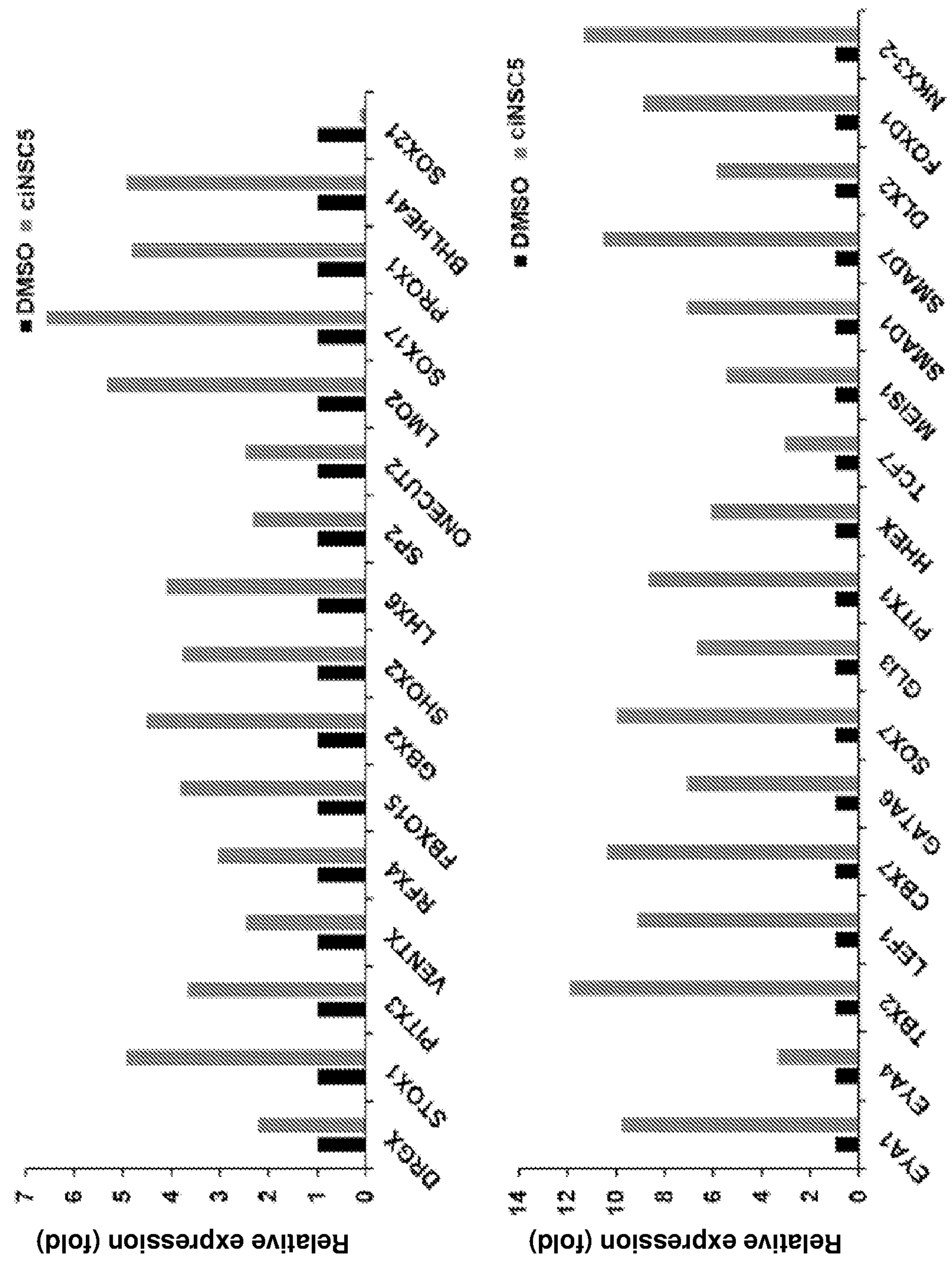
DMSO
ciNSC5
Relative expression (fold)
0
1
2
3
4
5
6
7
DRGX
STOX1
PITX3
VENTX
RFX4
FBXO15
GBX2
SHOX2
LHX6
SP2
ONECUT2
LMO2
SOX17
PROX1
BHLHE41
SOX21
DMSO
ciNSC5
Relative expression (fold)
0
2
4
6
8
10
12
14
EYA1
EYA4
TBX2
LEF1
CBX7
GATA6
SOX7
GLI3
PITX1
HHEX
TCF7
MEIS1
SMAD1
SMAD7
DLX2
FOXD1
NKX3-2

FIG. 27

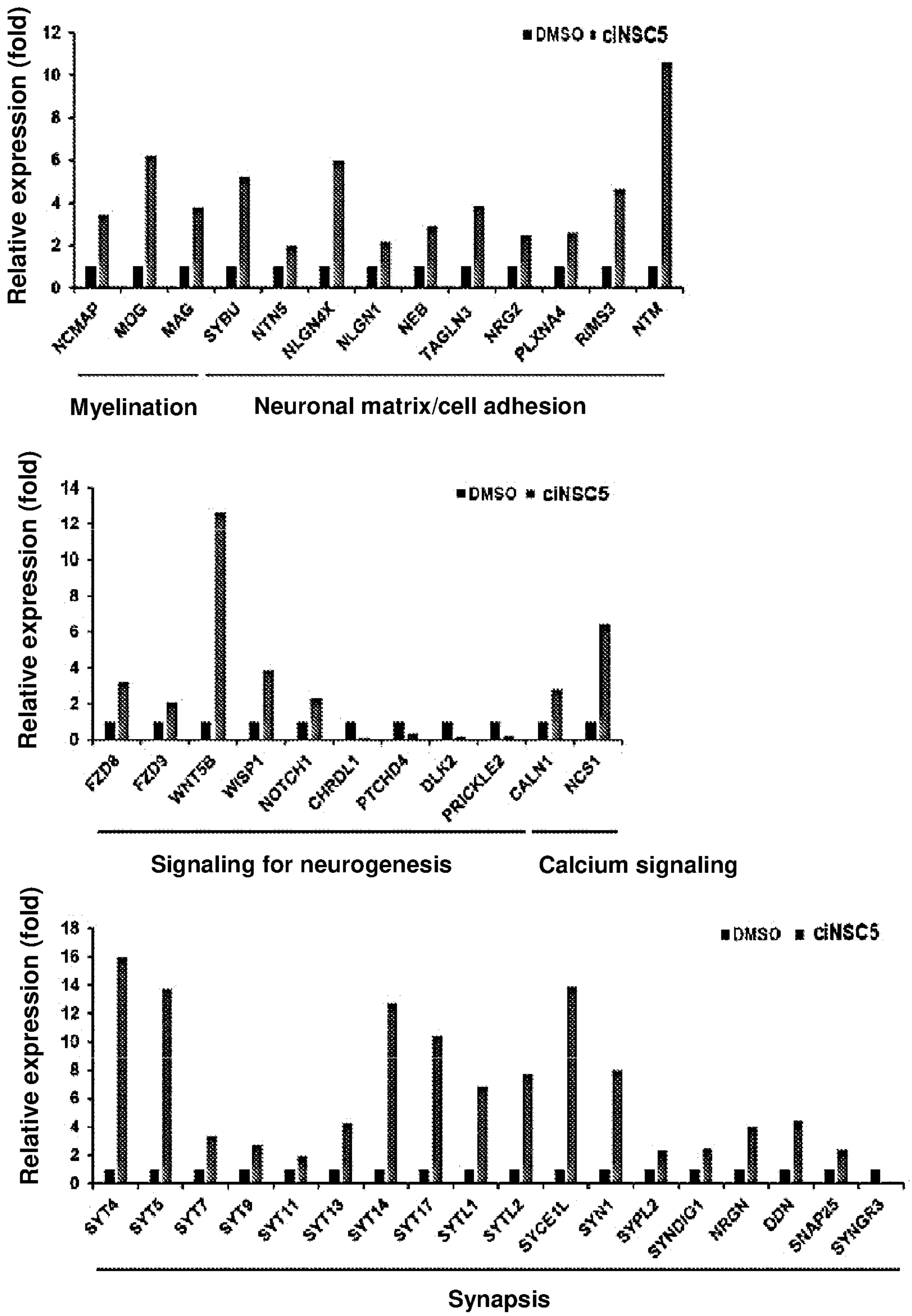
Relative expression (fold)
DMSO
ciNSC5
NCMAP
MOG
MAG
SYBU
NTN5
NLGN4X
NLGN1
NEB
TAGLN3
NRG2
PLXNA4
RIMS3
NTM
Myelination
Neuronal matrix/cell adhesion
FZD8
FZD9
WNT5B
WISP1
NOTCH1
CHRDL1
PTCHD4
DLK2
PRICKLE2
CALN1
NCS1
Signaling for neurogenesis
Calcium signaling
SYT4
SYT5
SYT7
SYT9
SYT11
SYT13
SYT14
SYT17
SYTL1
SYTL2
SYCE1L
SYN1
SYPL2
SYNDIG1
NRGN
DDN
SNAP25
SYNGR3
Synapsis

FIG. 28

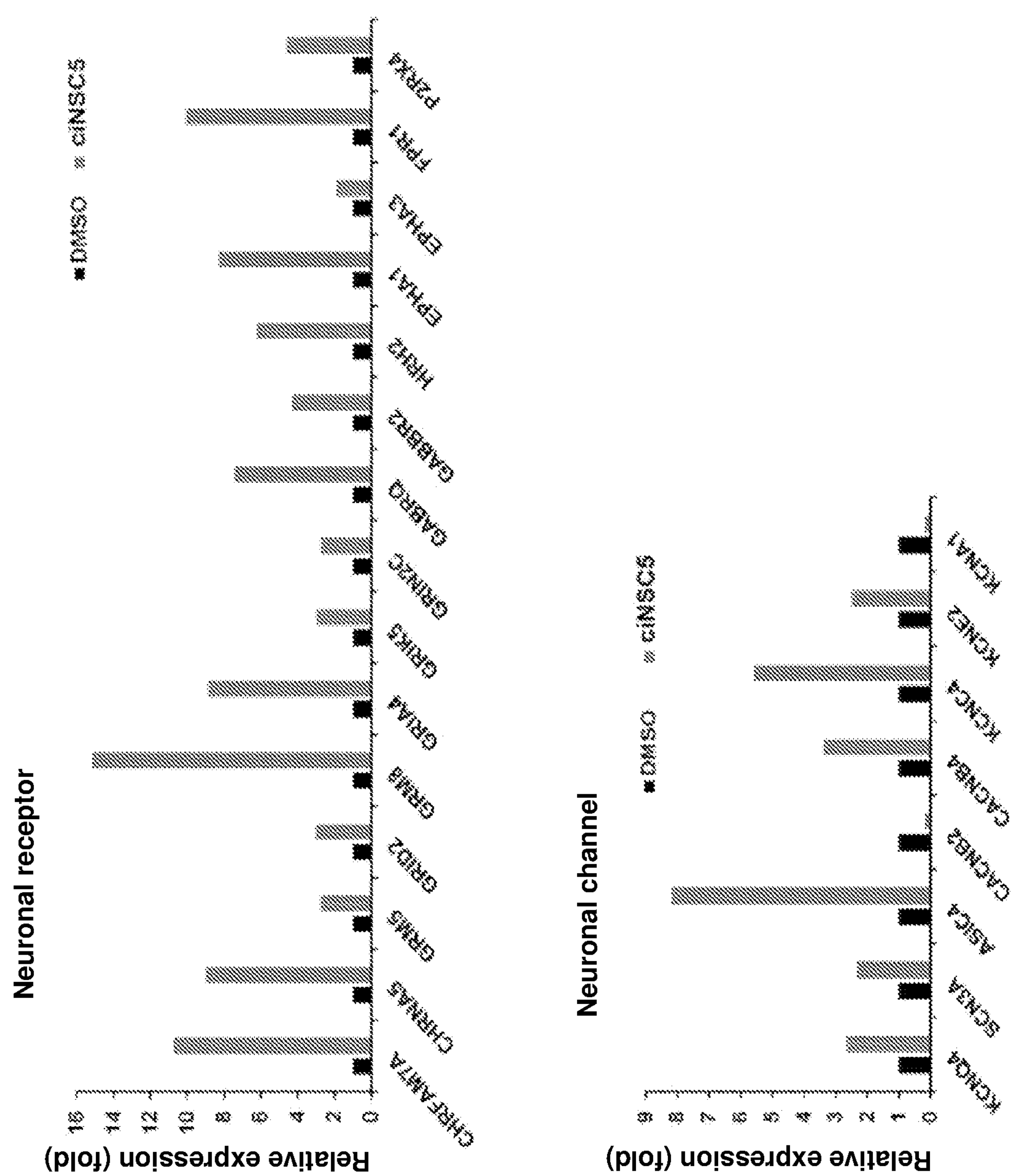
Neuronal receptor
DMSO
ciNSC5
Relative expression (fold)
CHRFAM7A
CHRNA5
GRM5
GRID2
GRM8
GRIA4
GRIK3
GRIN2C
GABRQ
GABBR2
HRH2
EPHA1
EPHA3
FPR1
P2RX4
Neuronal channel
DMSO
ciNSC5
Relative expression (fold)
KCNQ4
SCN3A
ASIC4
CACNB2
CACNB4
KCNC4
KCNE2
KCNA1

FIG. 29

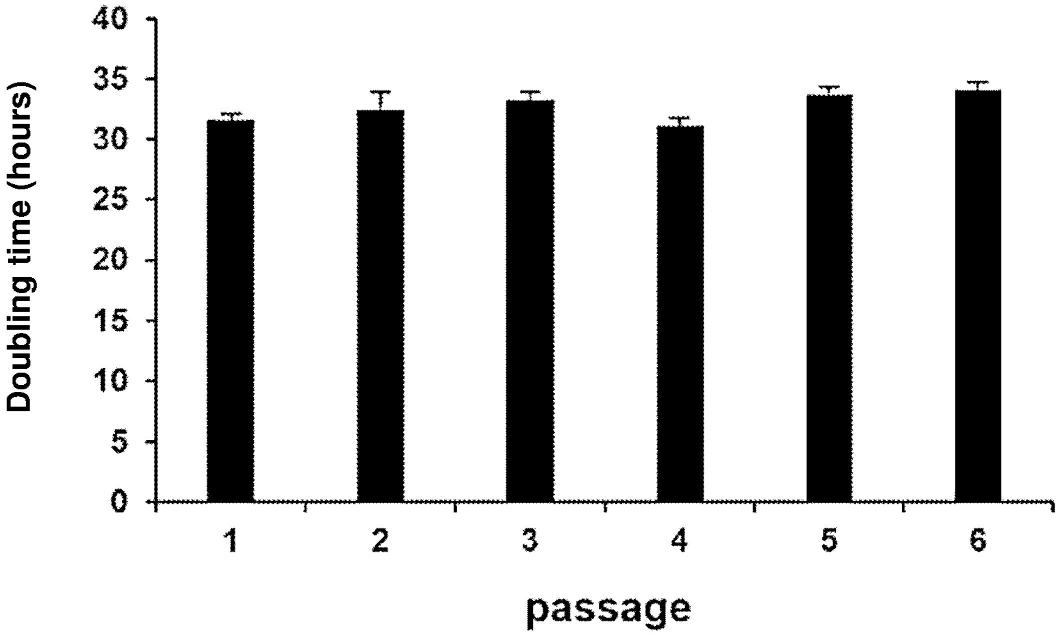
Doubling time (hours)
40
35
30
25
20
15
10
5
0
1
2
3
4
5
6
passage

FIG. 30

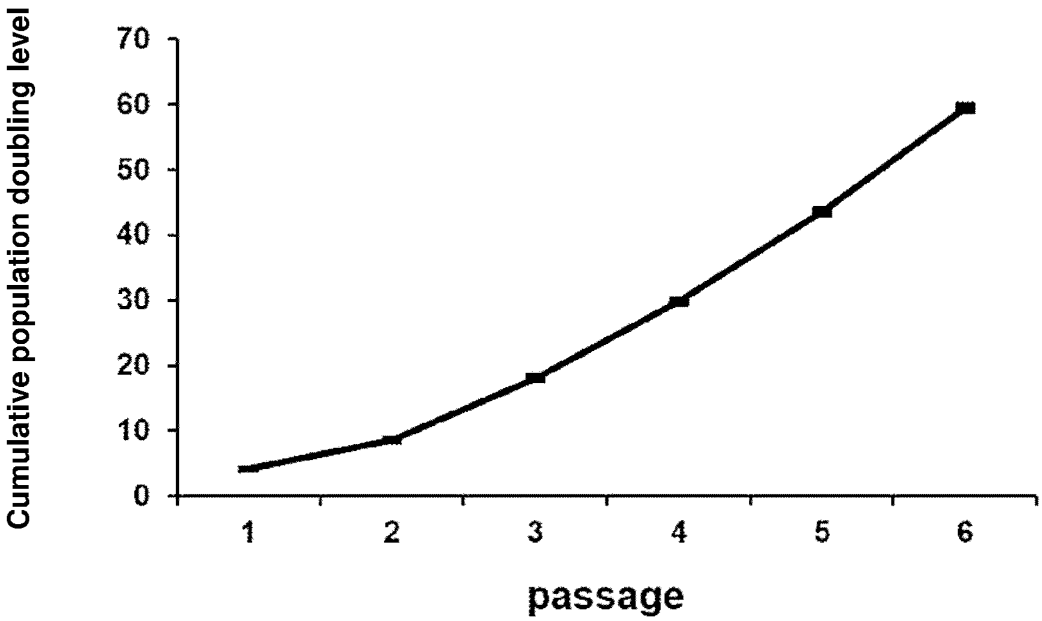
Cumulative population doubling level
70
60
50
40
30
20
10
0
1
2
3
4
5
6
passage

FIG. 31

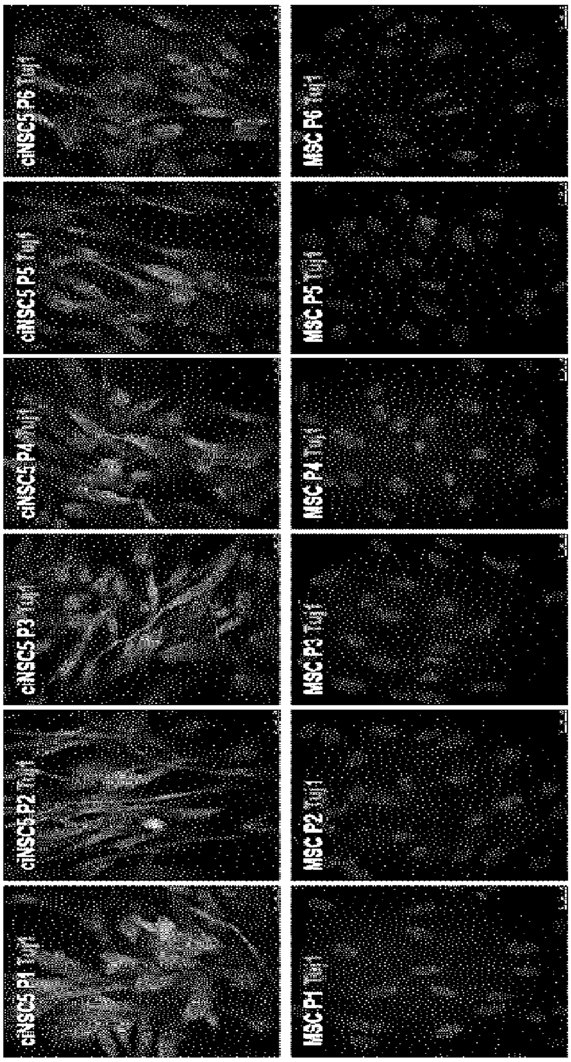
ciNSC5 P1 Tuj1
ciNSC5 P2 Tuj1
ciNSC5 P3 Tuj1
ciNSC5 P4 Tuj1
ciNSC5 P5 Tuj1
ciNSC5 P6 Tuj1
MSC P1 Tuj1
MSC P2 Tuj1
MSC P3 Tuj1
MSC P4 Tuj1
MSC P5 Tuj1
MSC P6 Tuj1

FIG. 32

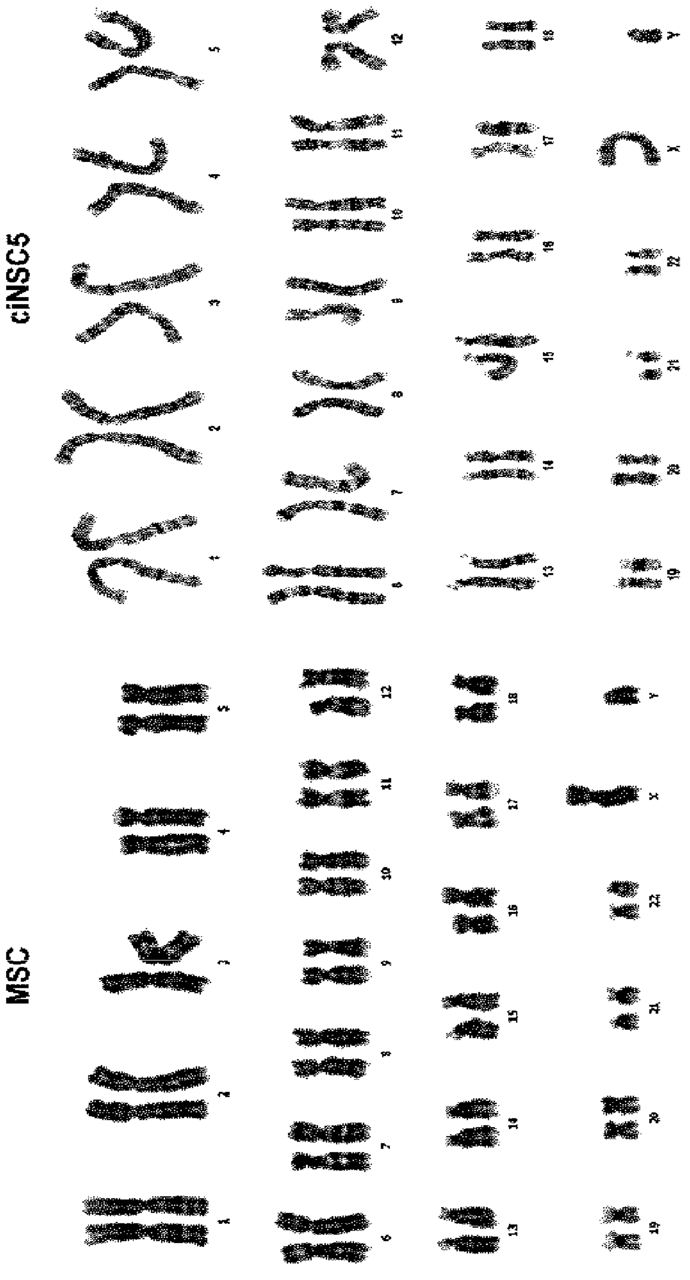

FIG. 33

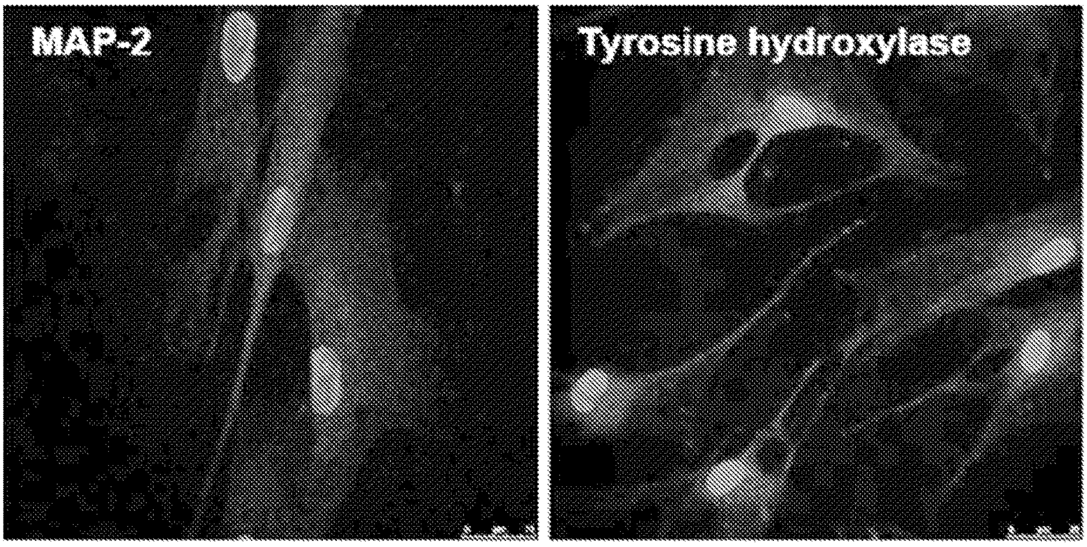


**Neuronal differentiation media (21 days)**

**Dopaminergic neuron differentiation media (3 days)**

FIG. 34

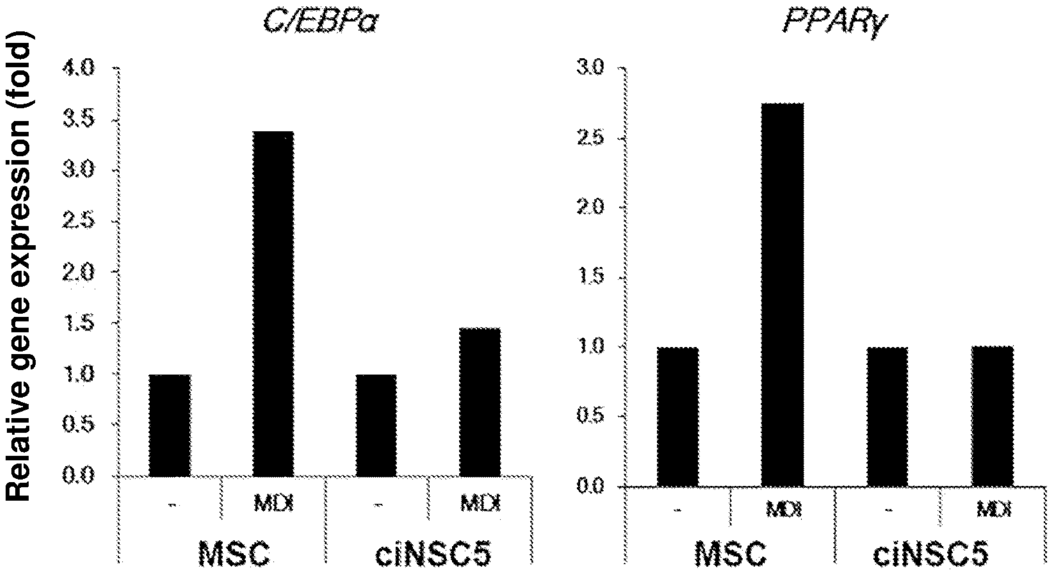

FIG. 35

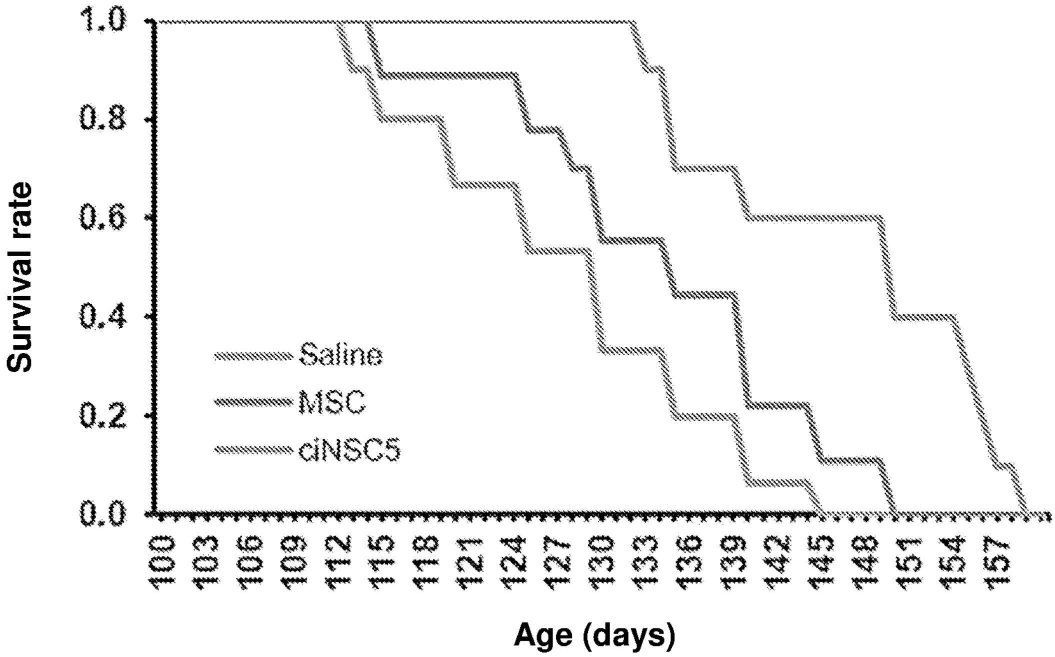
1.0
0.8
0.6
0.4
0.2
0.0
Survival rate
Saline
MSC
ciNSC5
100
103
106
109
112
115
118
121
124
127
130
133
136
139
142
145
148
151
154
157
Age (days)

FIG. 36

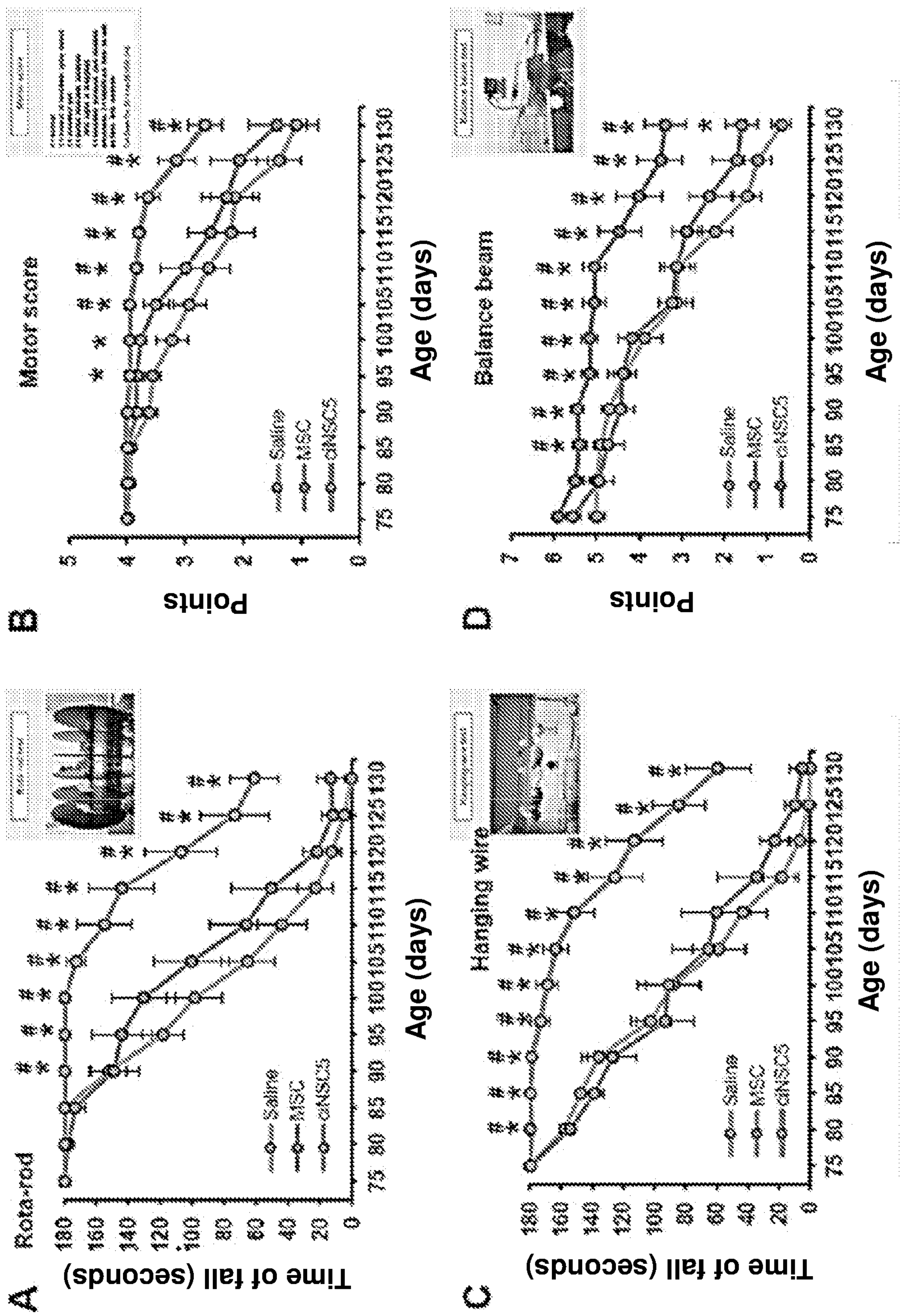
A
Rota-rod
Time of fall (seconds)
Age (days)
B
Motor score
Points
Age (days)
C
Hanging wire
Time of fall (seconds)
Age (days)
D
Balance beam
Points
Age (days)
Saline
MSC

FIG. 37

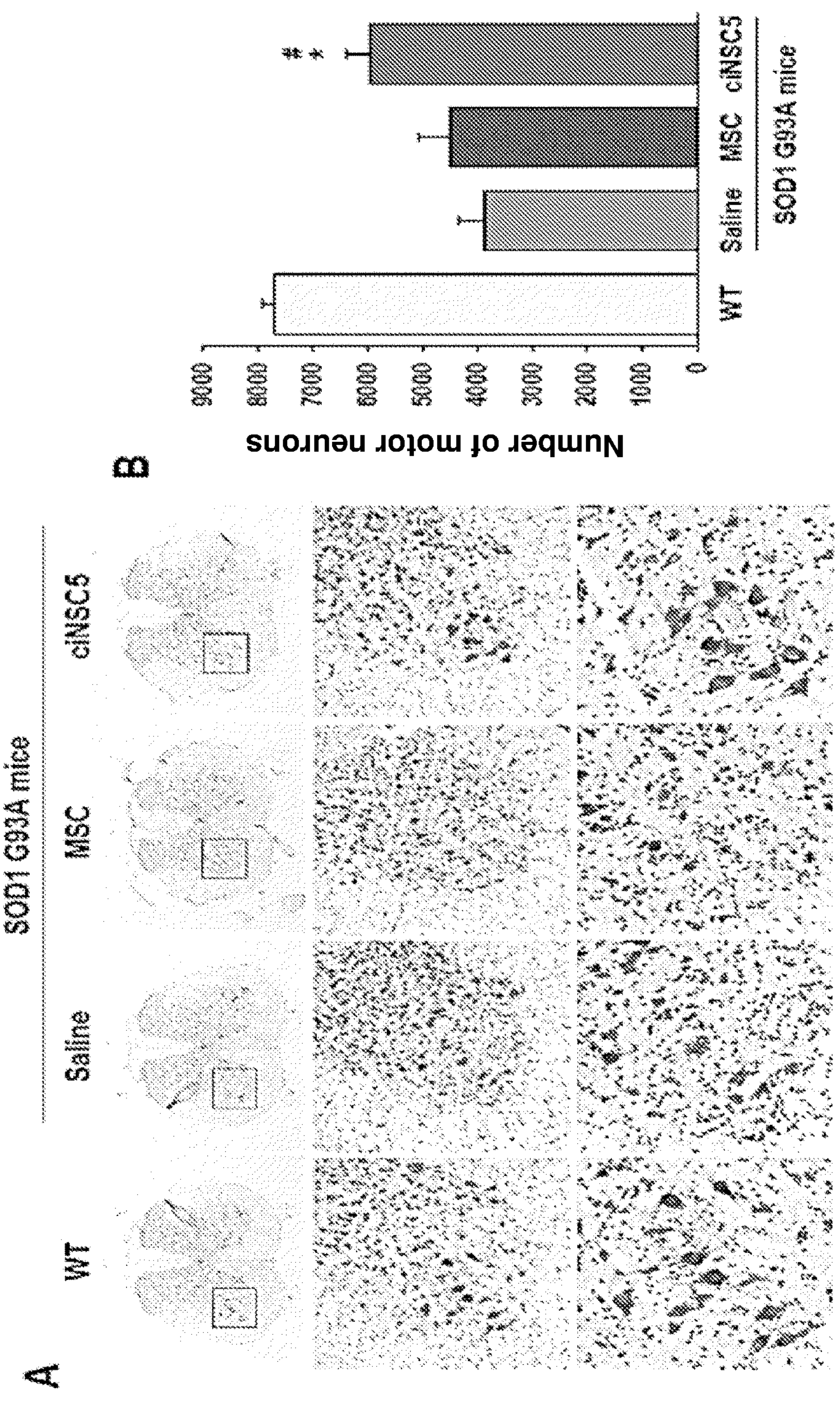
A
WT
Saline
MSC
ciNSC5
SOD1 G93A mice
B
Number of motor neurons
9000
8000
7000
6000
5000
4000
3000
2000
1000
0
WT
Saline
MSC
ciNSC5
SOD1 G93A mice
*
#

FIG. 38

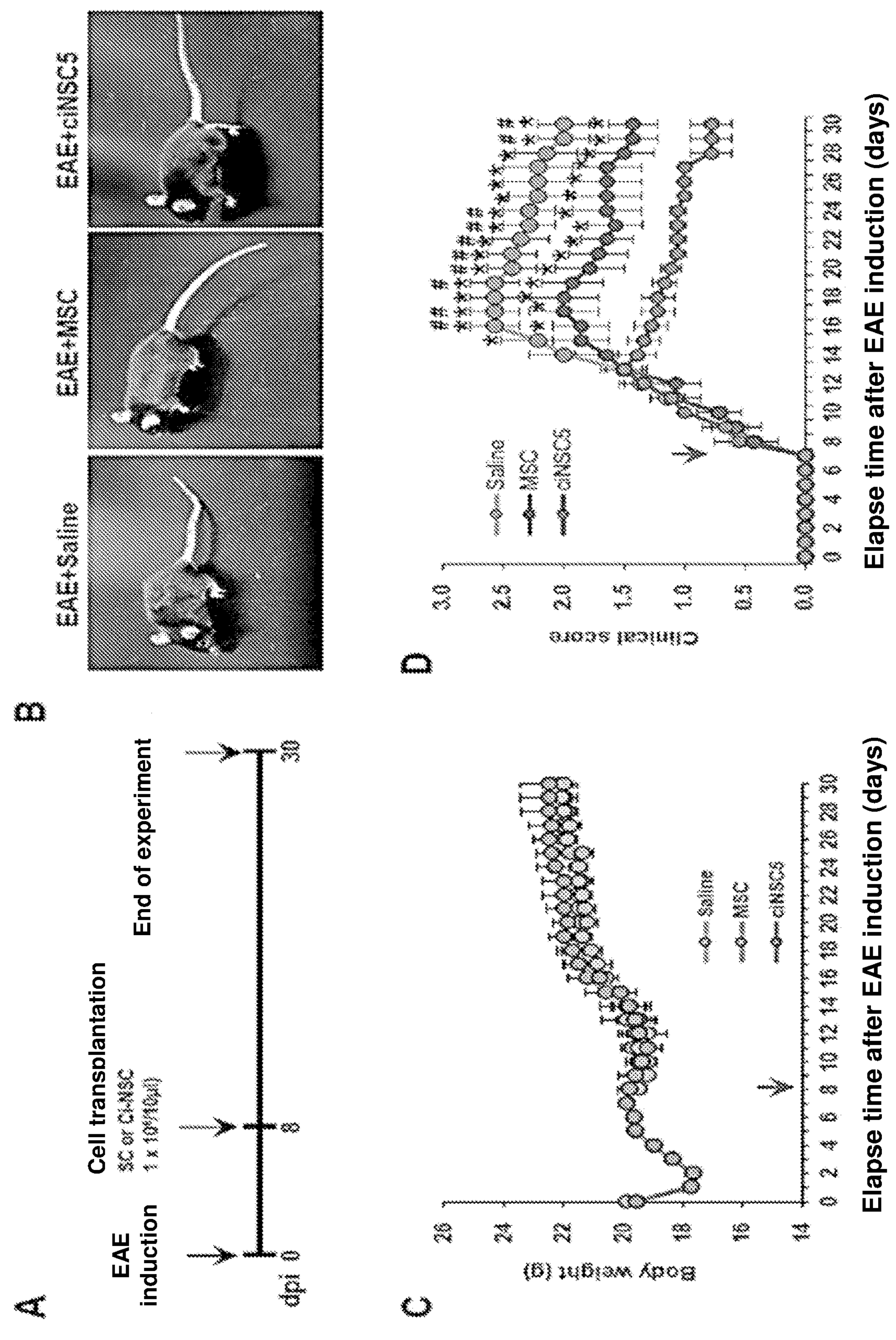
A
EAE induction
Cell transplantation
SC or Ci-NSC
1 x 10^6/10μl
End of experiment
dpi 0
8
30
B
EAE+Saline
EAE+MSC
EAE+ciNSC5
C
Body weight (g)
Saline
MSC
ciNSC5
Elapse time after EAE induction (days)
D
Clinical score
Saline
MSC
ciNSC5
Elapse time after EAE induction (days)

FIG. 39

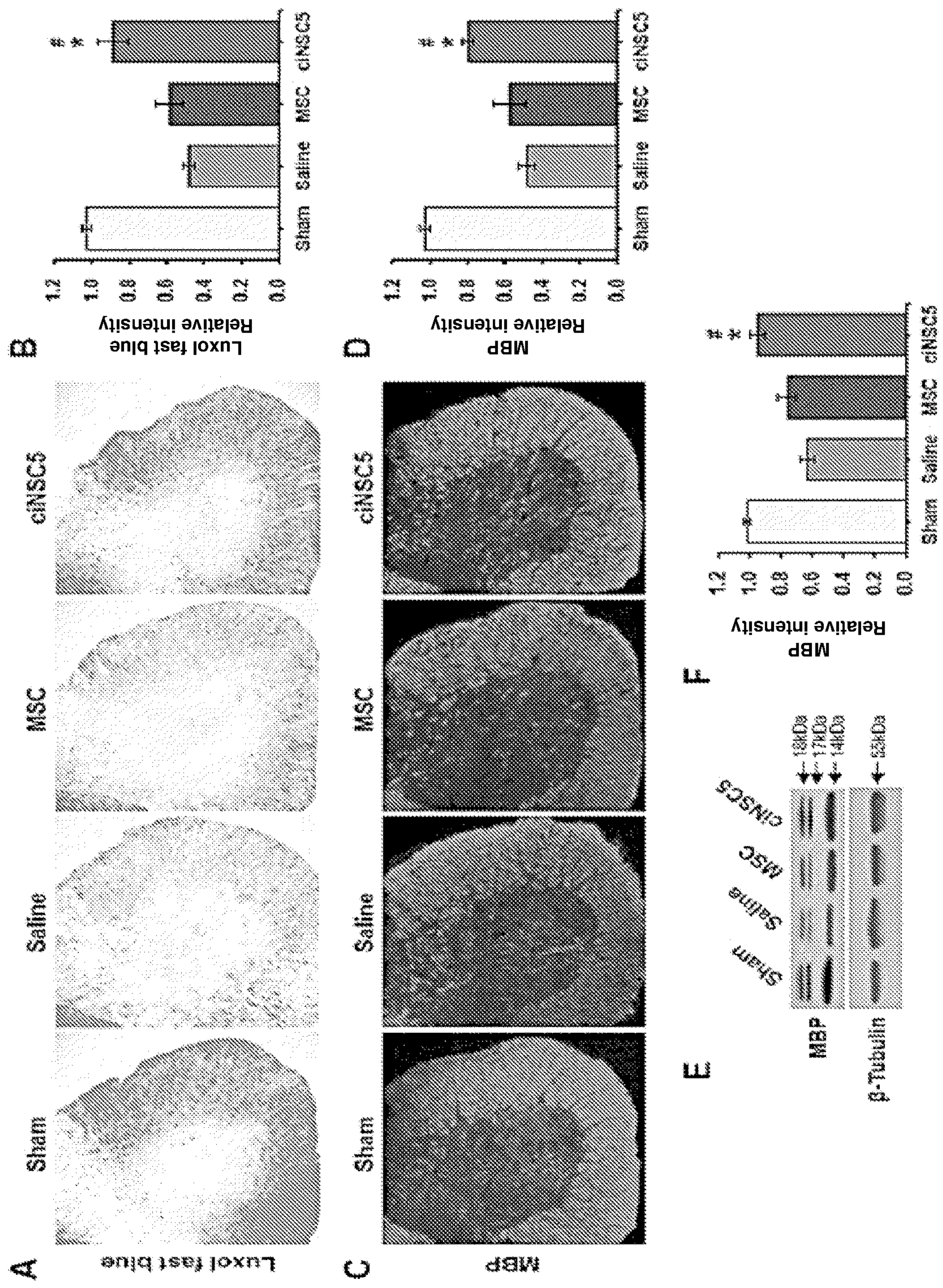
A
Luxol fast blue
Sham
Saline
MSC
ciNSC5
B
Luxol fast blue
Relative intensity
C
MBP
D
MBP
Relative intensity
E
MBP
β-Tubulin
18kDa
17kDa
14kDa
53kDa
F
MBP
Relative intensity

FIG. 40

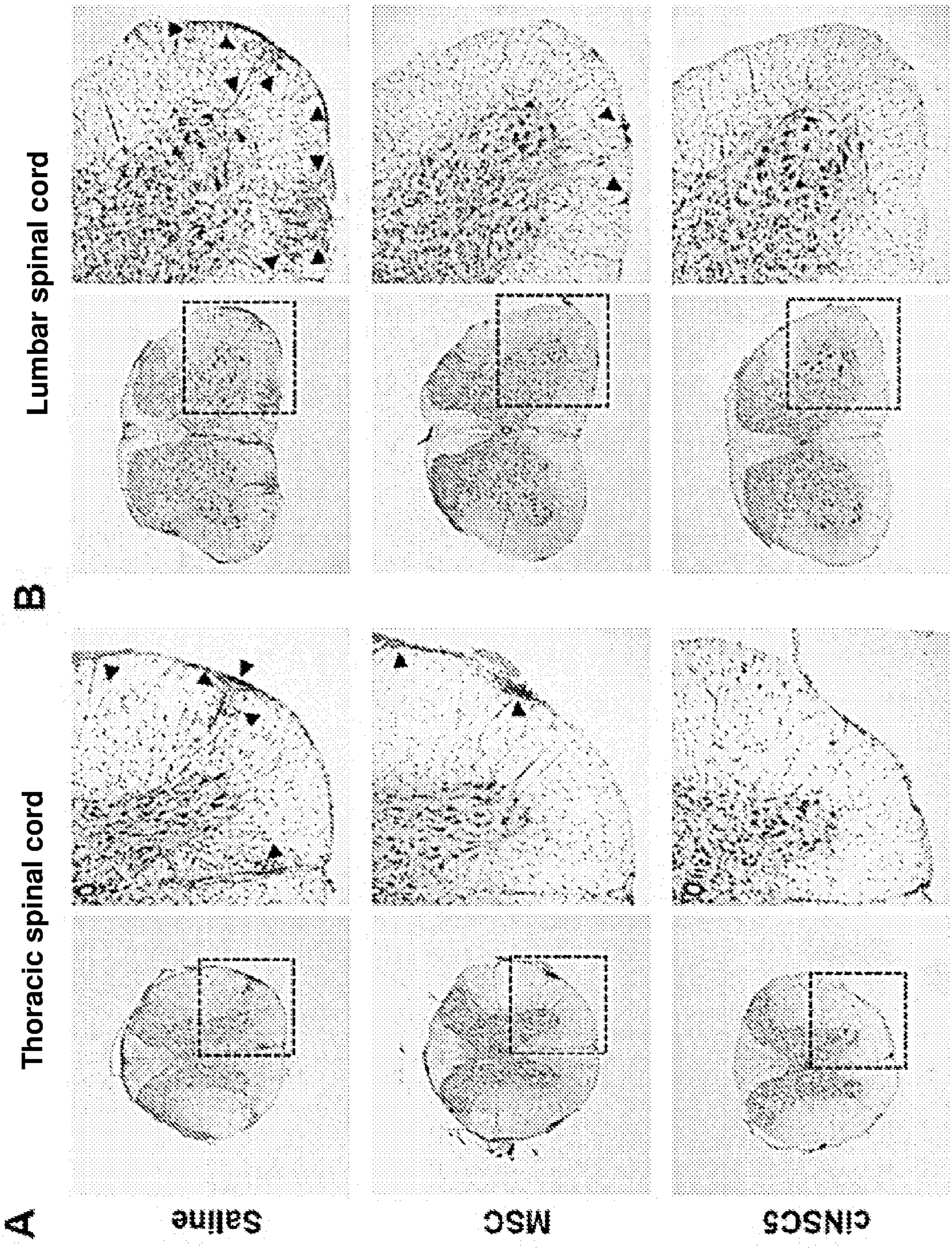
A
B
Thoracic spinal cord
Lumbar spinal cord
Saline
MSC
ciNSC5

FIG. 41

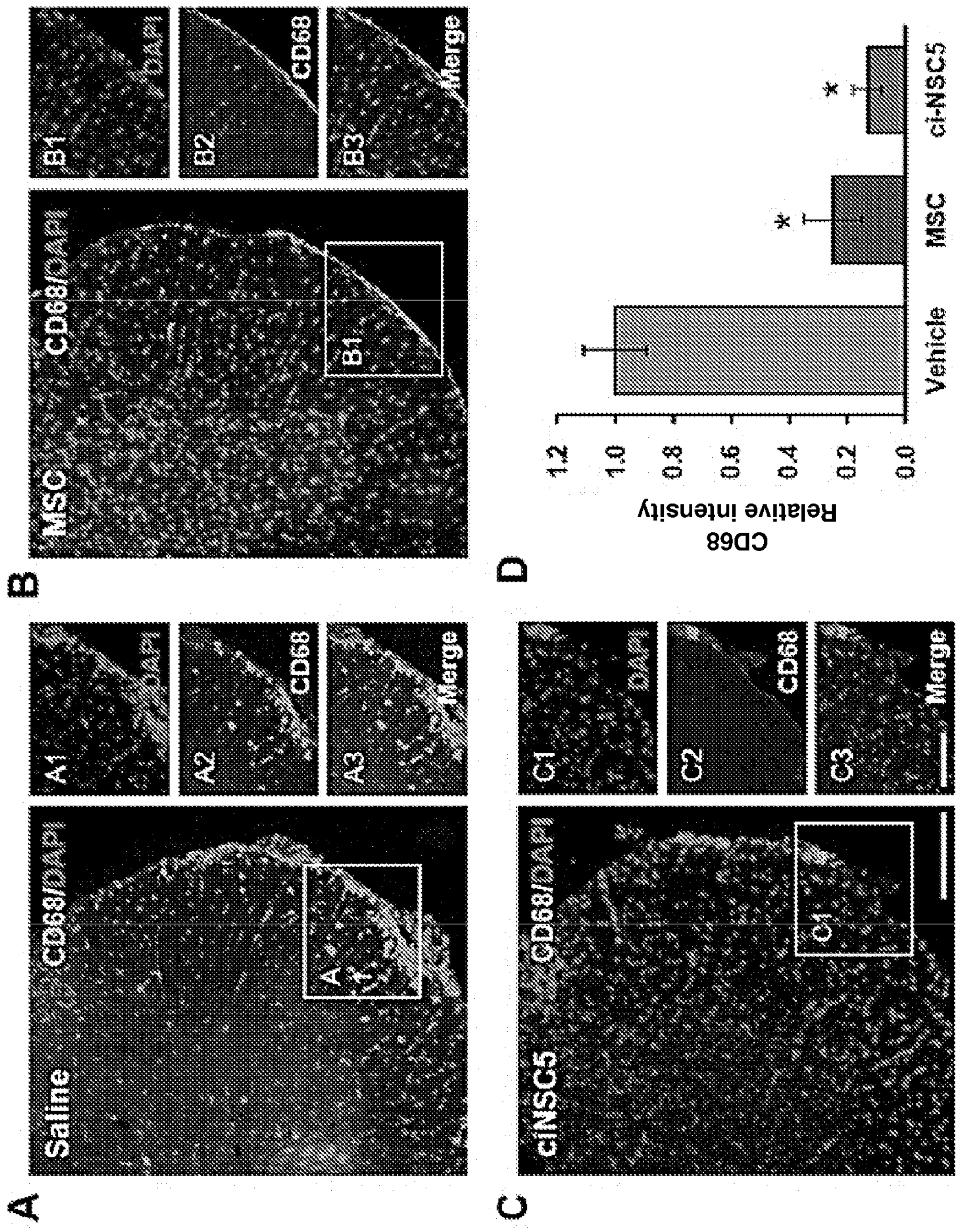
A
Saline
CD68/DAPI
A1
DAPI
A2
CD68
A3
Merge
B
MSC
CD68/DAPI
B1
DAPI
B2
CD68
B3
Merge
C
ciNSC5
CD68/DAPI
C1
DAPI
C2
CD68
C3
Merge
D
CD68
Relative intensity
1.2
1.0
0.8
0.6
0.4
0.2
0.0
Vehicle
MSC
ci-NSC5
*
*

FIG. 42

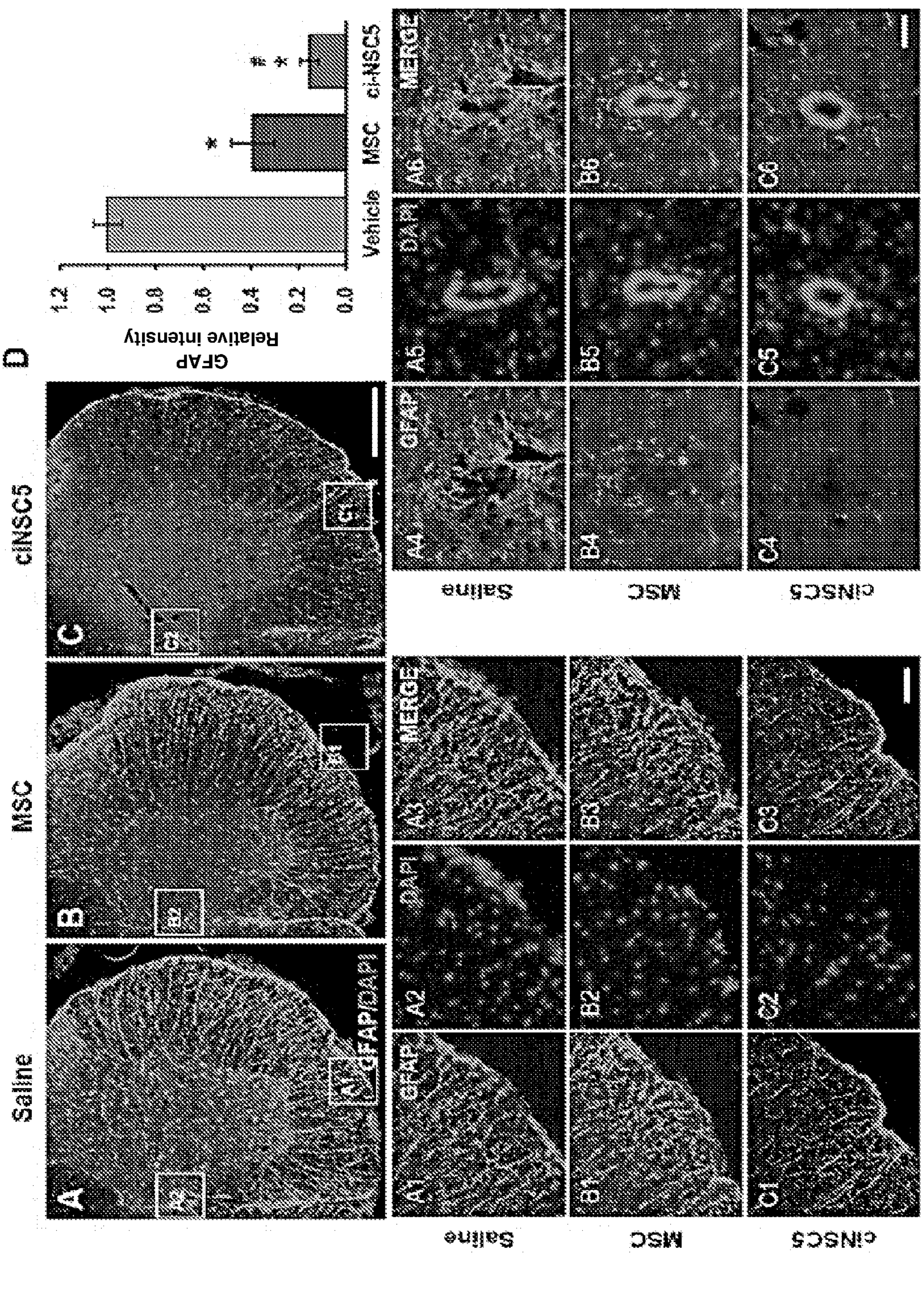

A
B
C
D
Saline
MSC
ciNSC5
GFAP/DAPI
GFAP
DAPI
MERGE
A1
A2
A3
A4
A5
A6
B1
B2
B3
B4
B5
B6
C1
C2
C3
C4
C5
C6
GFAP
Relative intensity
0.0
0.2
0.4
0.6
0.8
1.0
1.2
Vehicle
MSC
ciNSC5

FIG. 43

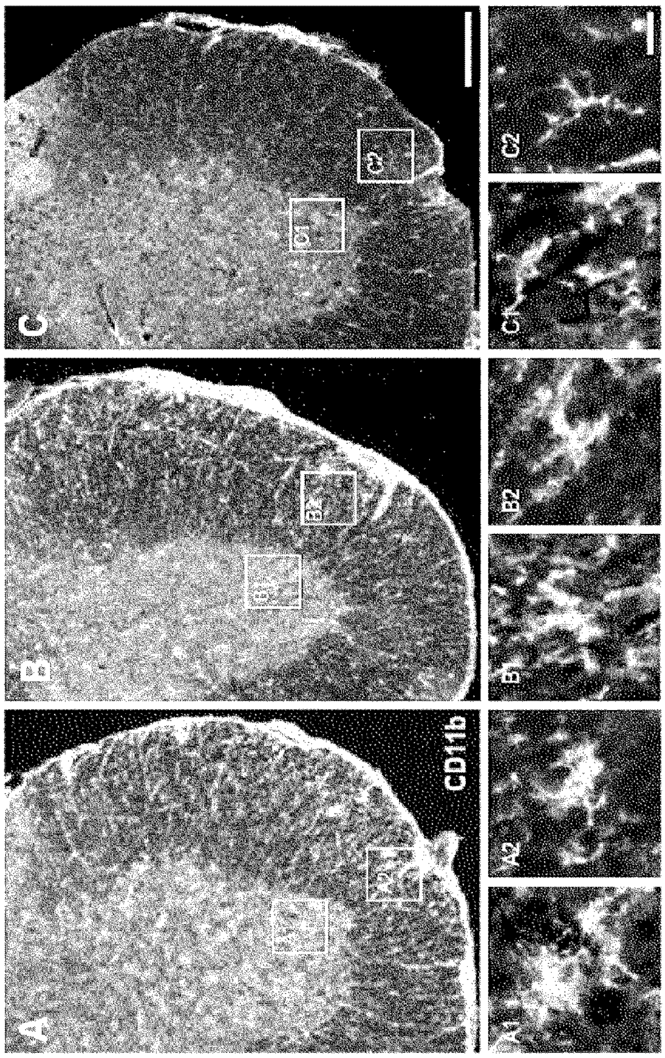

FIG. 44

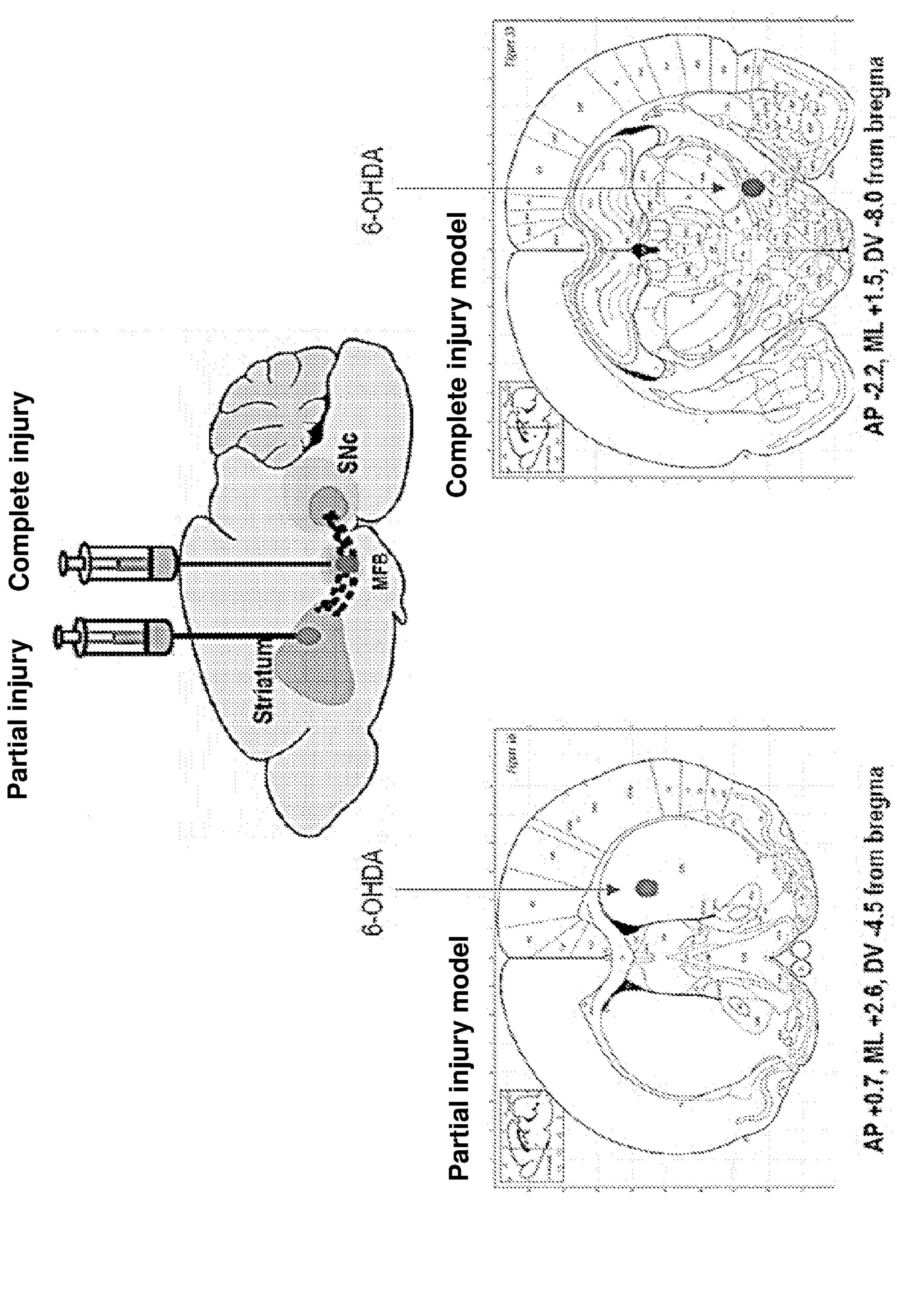
Partial injury
Complete injury
Striatum
SNc
MFB
6-OHDA
Partial injury model
AP +0.7, ML +2.6, DV -4.5 from bregma
6-OHDA
Complete injury model
AP -2.2, ML +1.5, DV -8.0 from bregma

FIG. 45

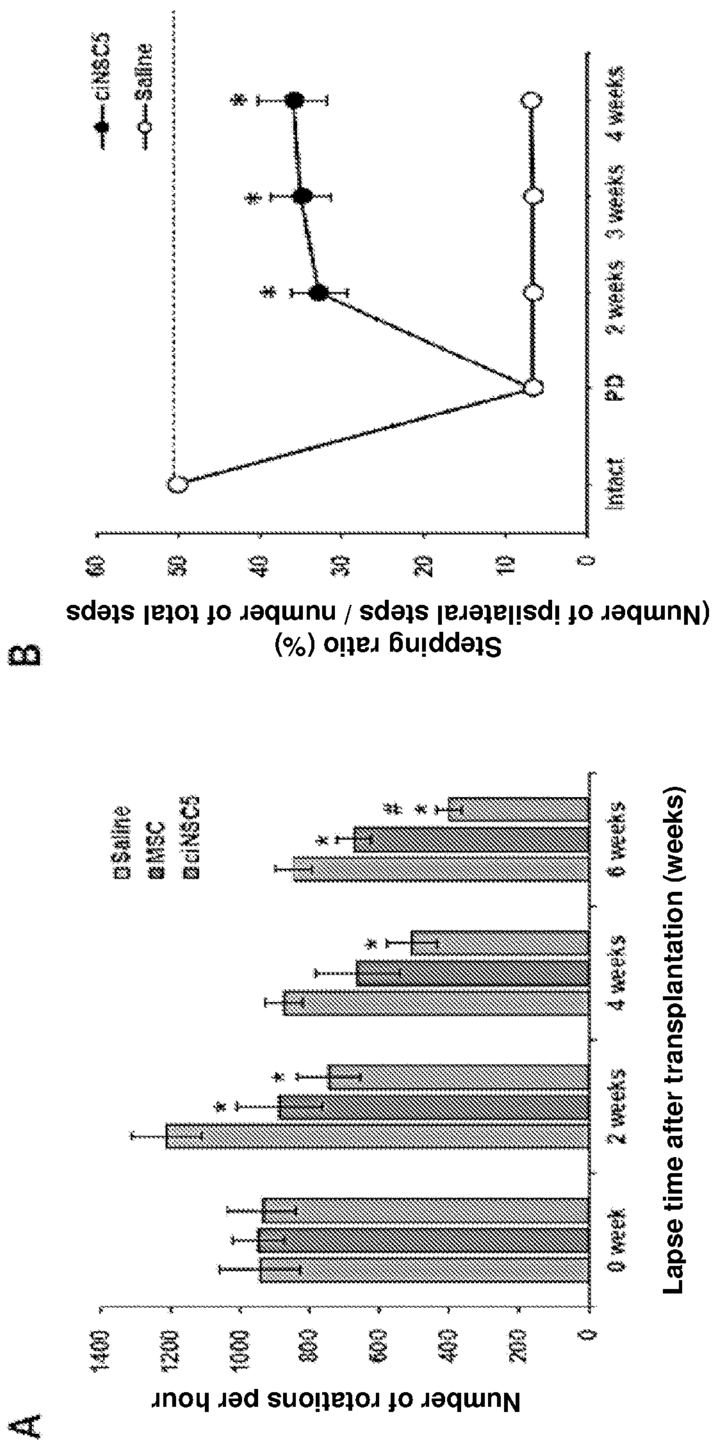
A
Number of rotations per hour
1400
1200
1000
800
600
400
200
0
0 week
2 weeks
4 weeks
6 weeks
Lapse time after transplantation (weeks)
Saline
MSC
ciNSC5
B
Stepping ratio (%)
(Number of ipsilateral steps / number of total steps
60
50
40
30
20
10
0
Intact
PD
2 weeks
3 weeks
4 weeks
ciNSC5
Saline

FIG. 46

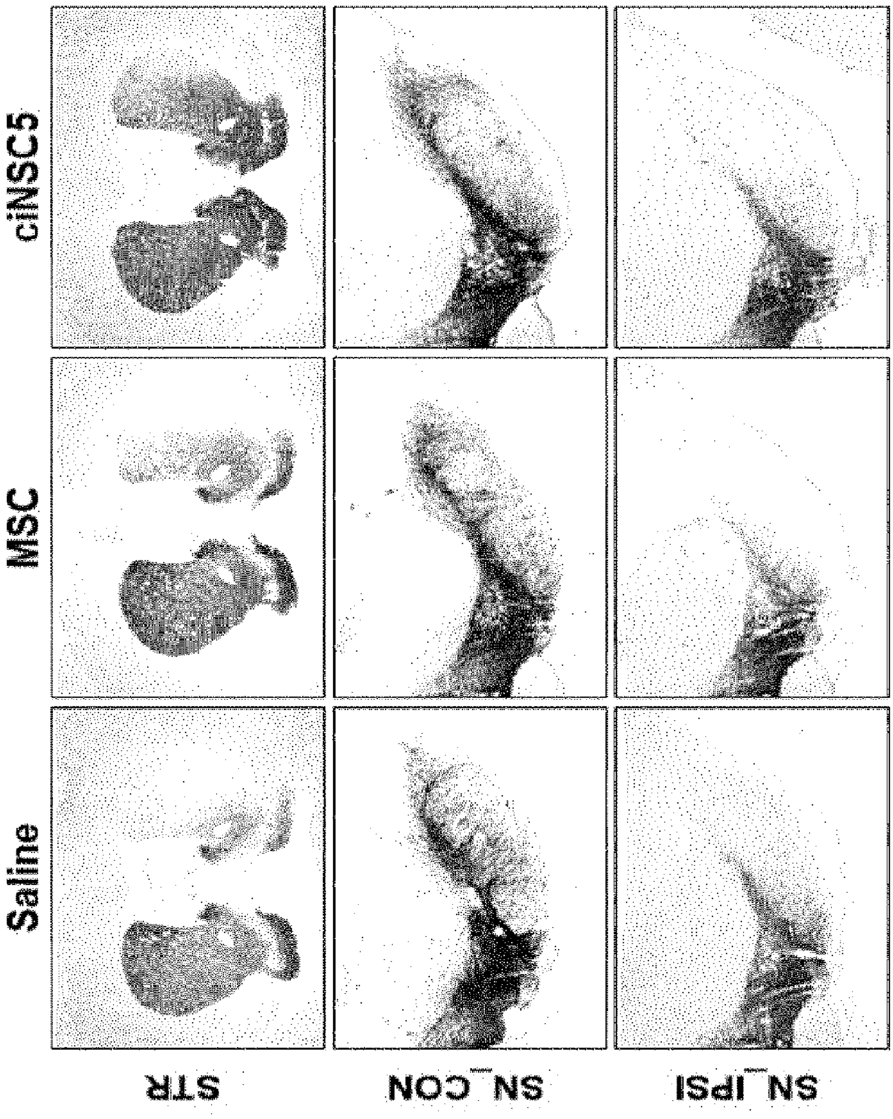

FIG. 47

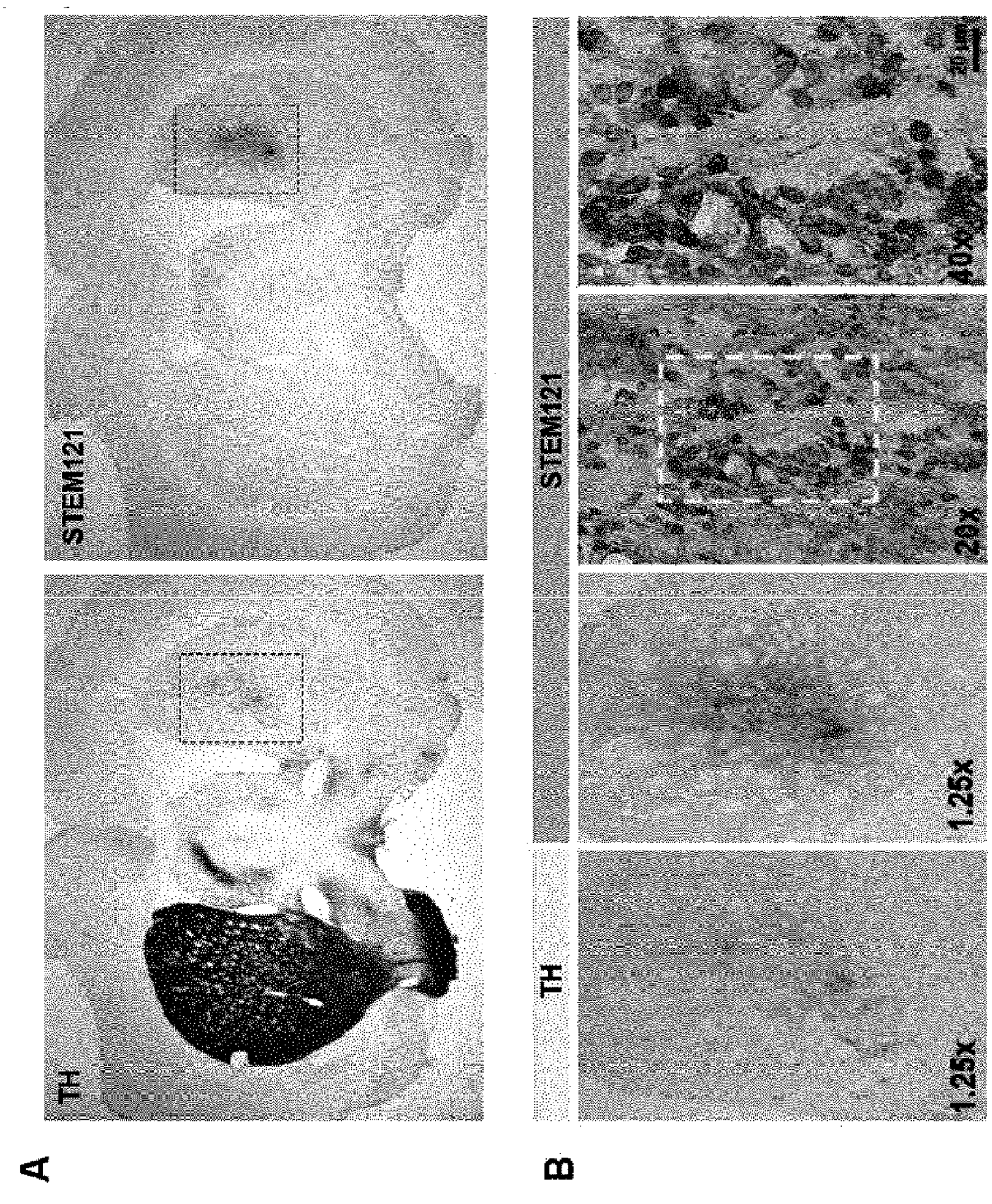

FIG. 48

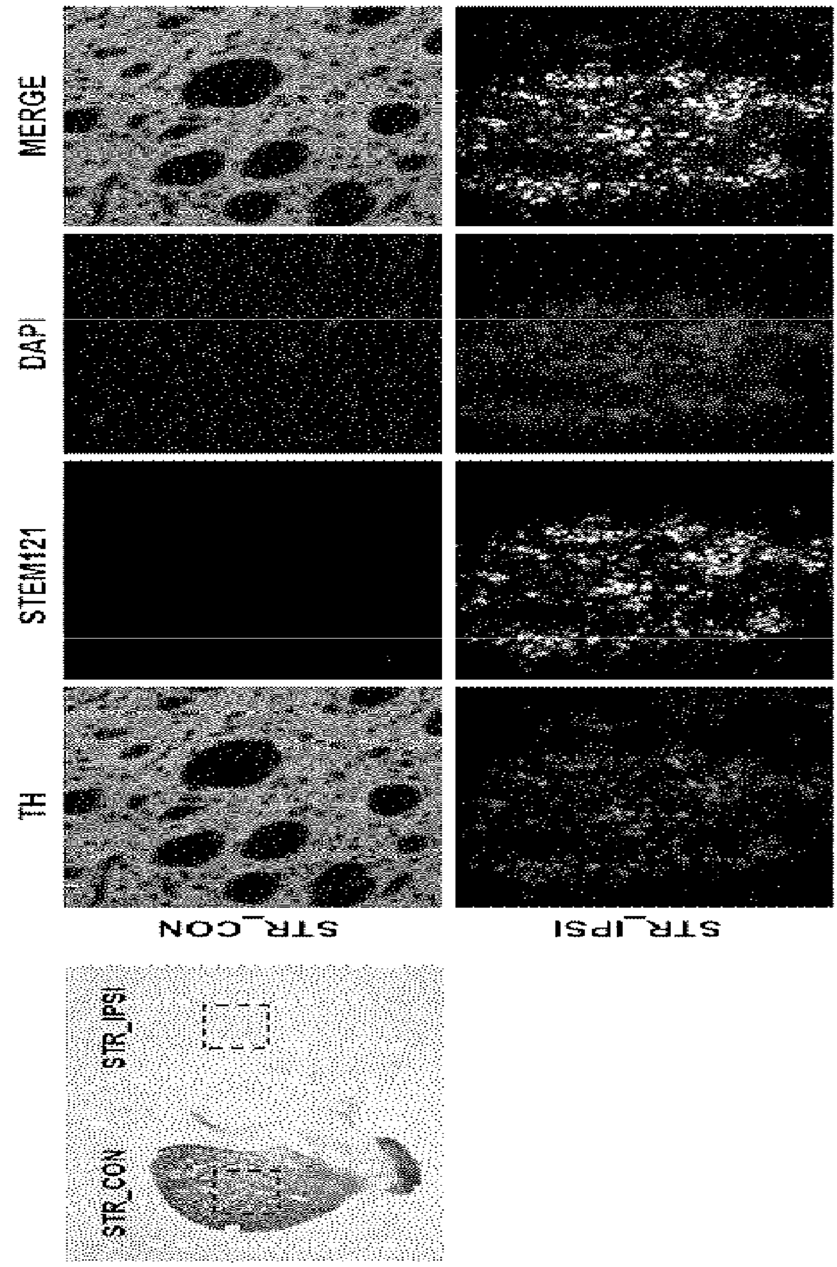

FIG. 49

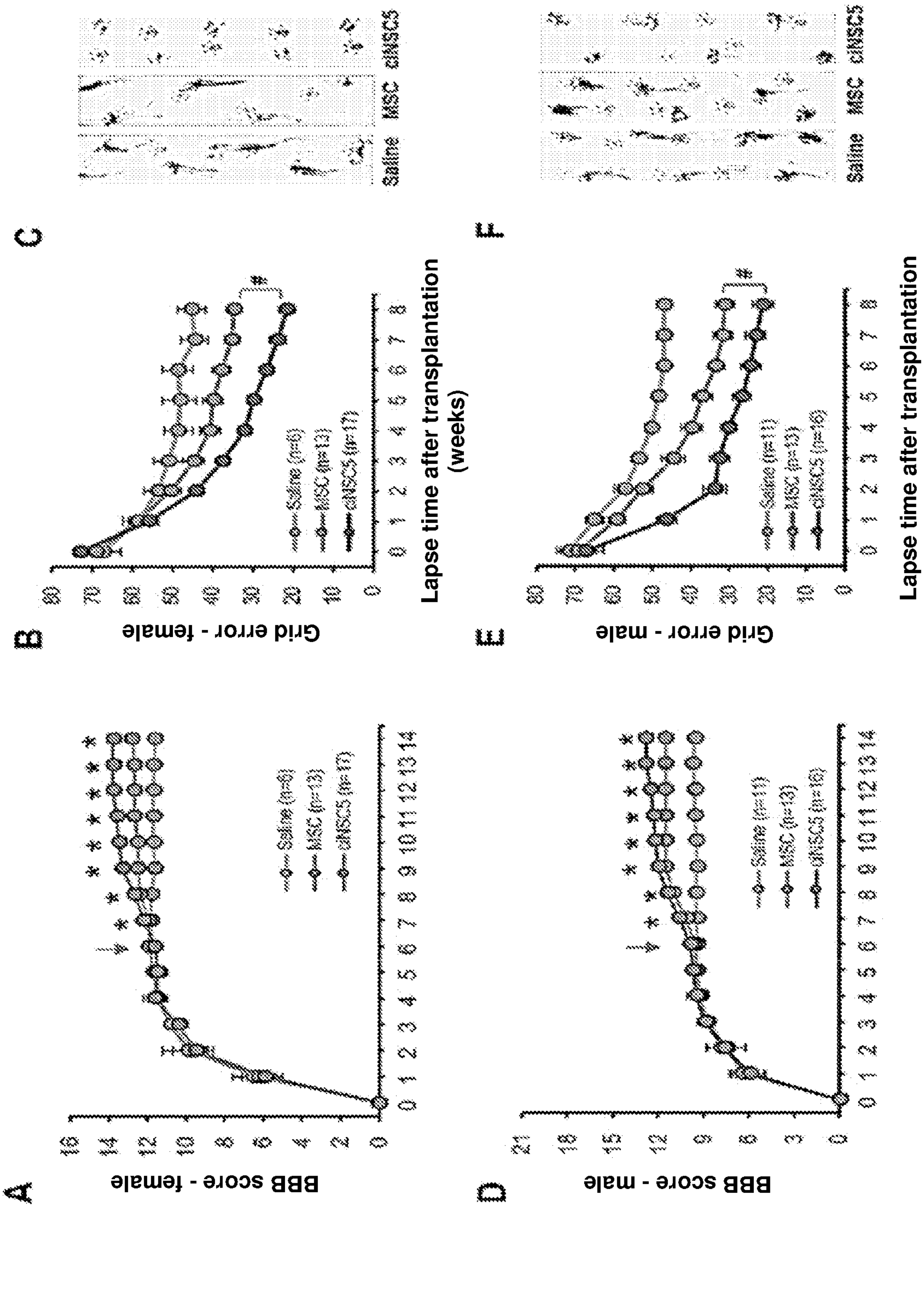
A
B
C
D
E
F
BBB score - female
BBB score - male
Grid error - female
Grid error - male
Lapse time after transplantation
(weeks)
Saline (n=6)
MSC (n=13)
ciNSC5 (n=17)
Saline (n=11)
MSC (n=13)
ciNSC5 (n=16)
Saline
MSC
ciNSC5

FIG. 50

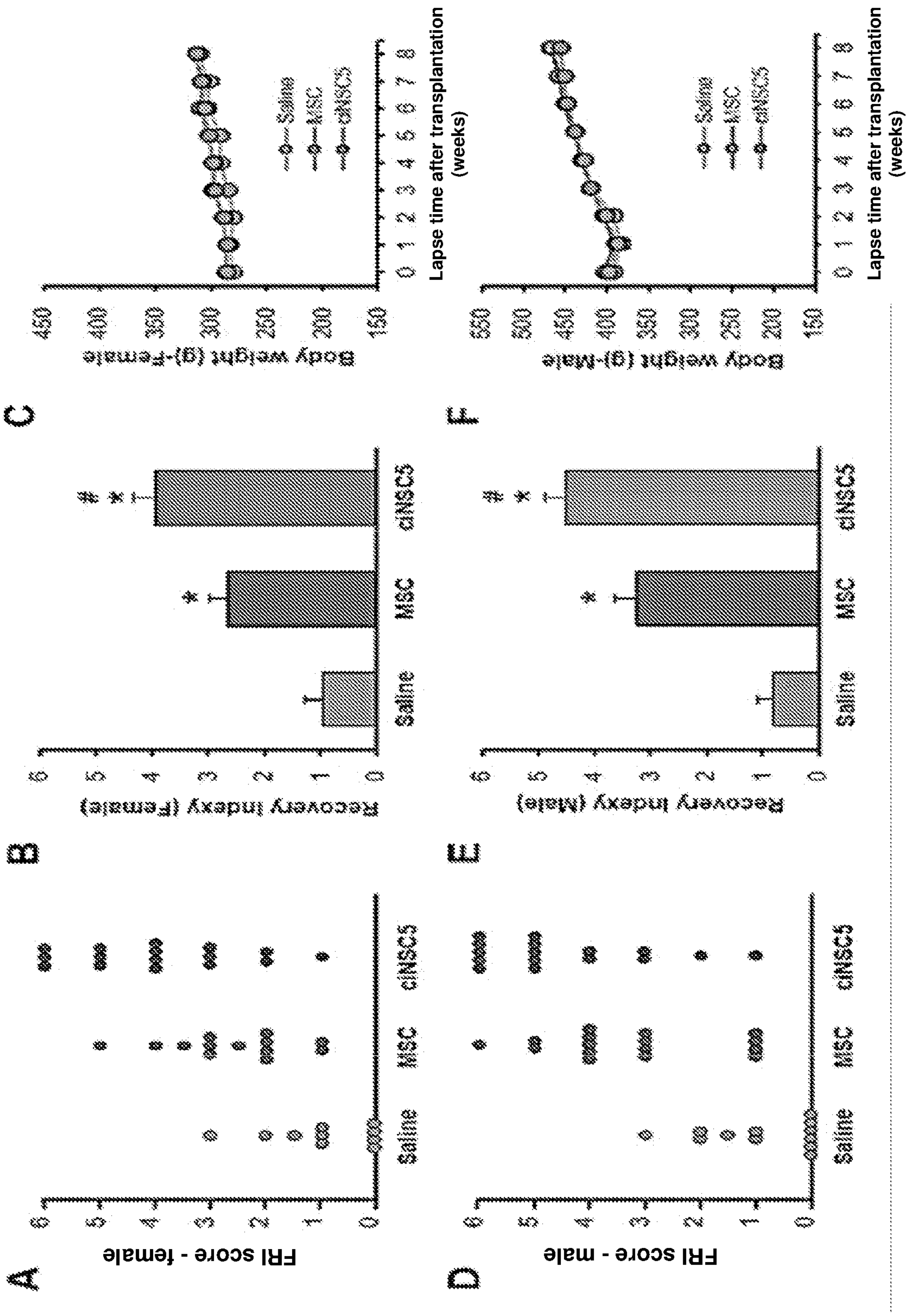
A
FRI score - female
Saline
MSC
ciNSC5
B
Recovery Indexy (Female)
C
Body weight (g)-Female
Lapse time after transplantation
(weeks)
D
FRI score - male
E
Recovery Indexy (Male)
F
Body weight (g)-Male

FIG. 51

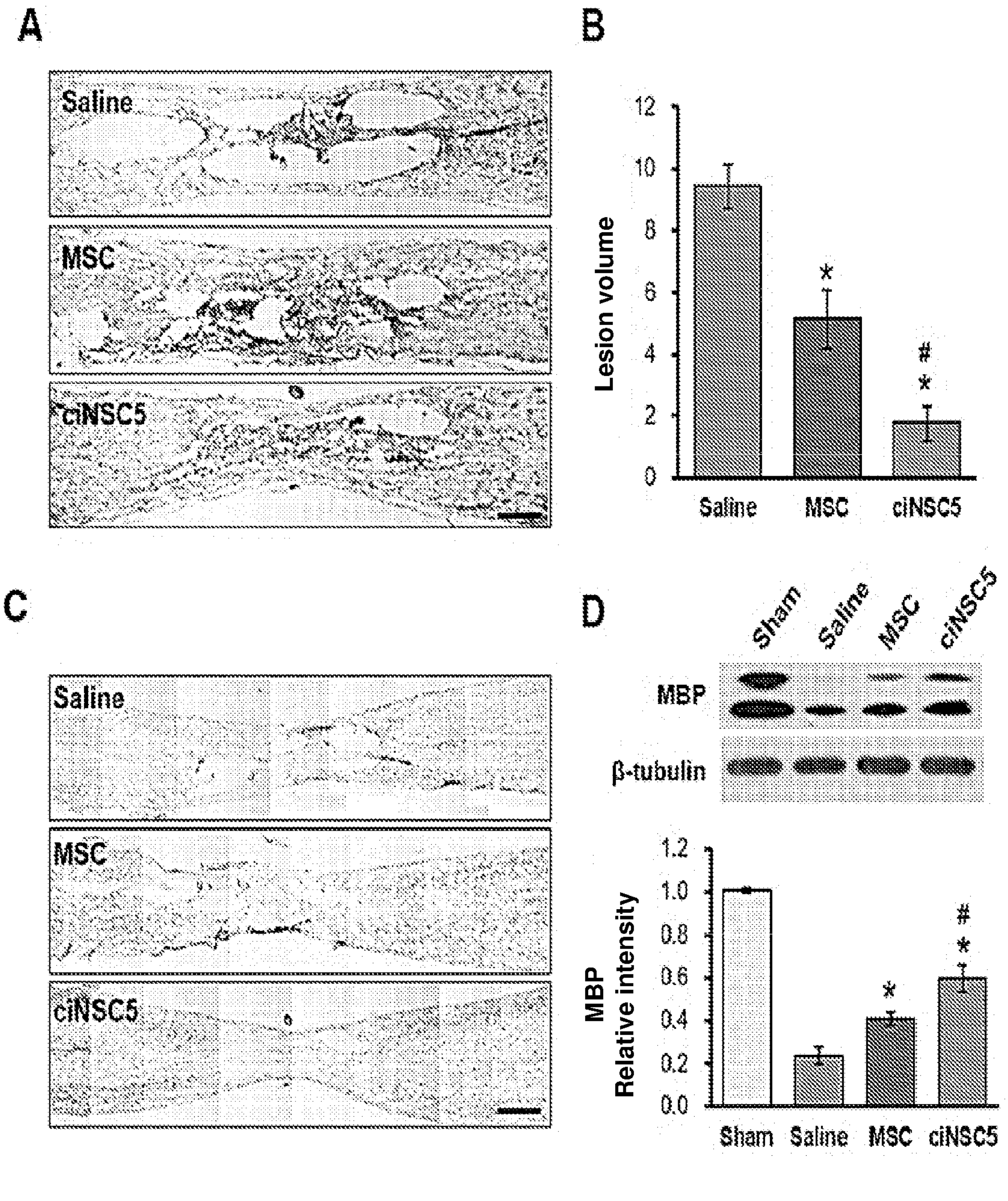
A
Saline
MSC
ciNSC5
B
Lesion volume
12
10
8
6
4
2
0
*
#
*
Saline
MSC
ciNSC5
C
Saline
MSC
ciNSC5
D
Sham
Saline
MSC
ciNSC5
MBP
β-tubulin
MBP
Relative intensity
1.2
1.0
0.8
0.6
0.4
0.2
0.0
*
#
*
Sham
Saline
MSC
ciNSC5

FIG. 52

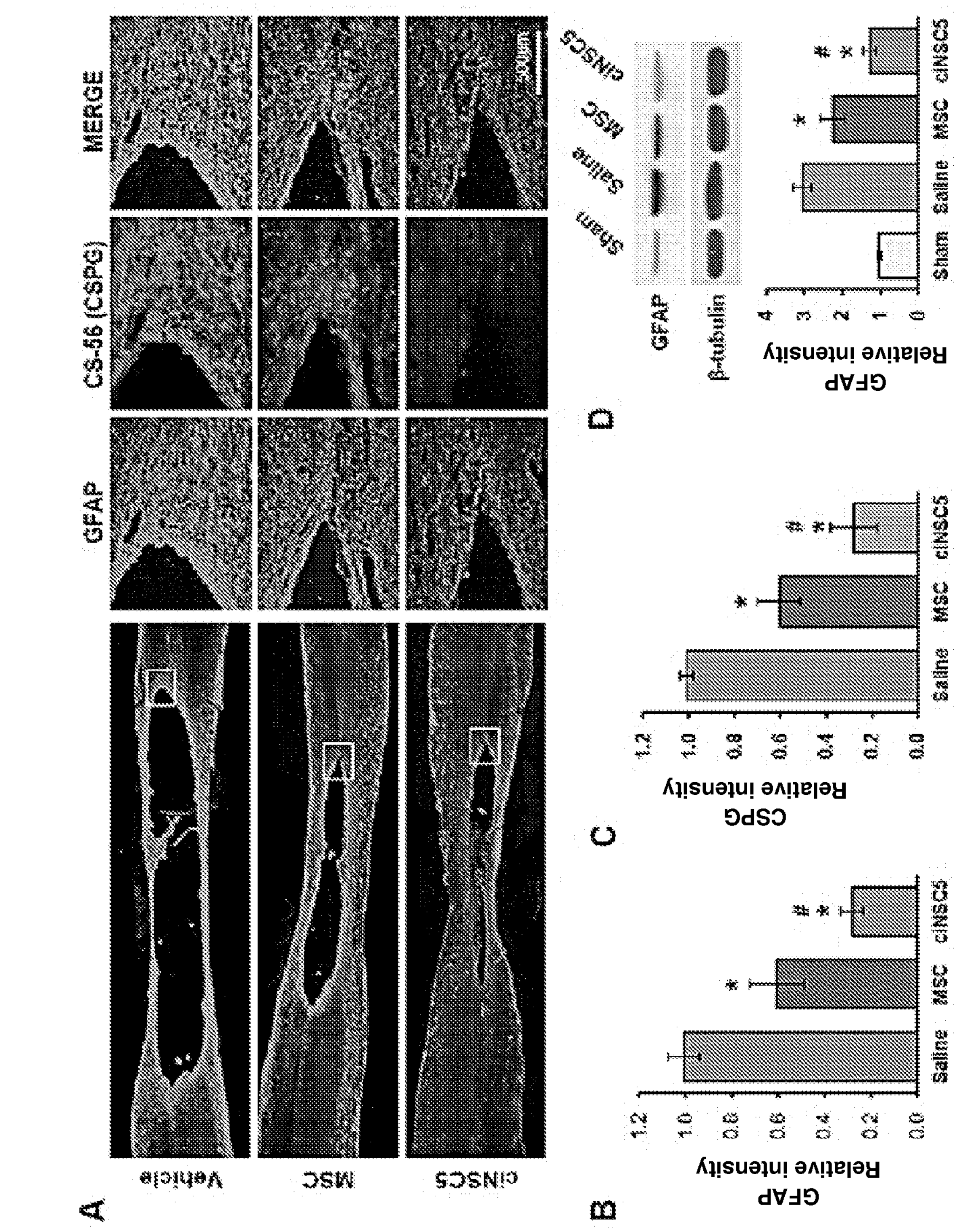
A
Vehicle
MSC
ciNSC5
GFAP
CS-56 (CSPG)
MERGE
B
GFAP
Relative intensity
Saline
MSC
ciNSC5
C
CSPG
Relative intensity
Saline
MSC
ciNSC5
D
Sham
Saline
MSC
ciNSC5
GFAP
β-tubulin
GFAP
Relative intensity
Sham
Saline
MSC
ciNSC5

FIG. 53

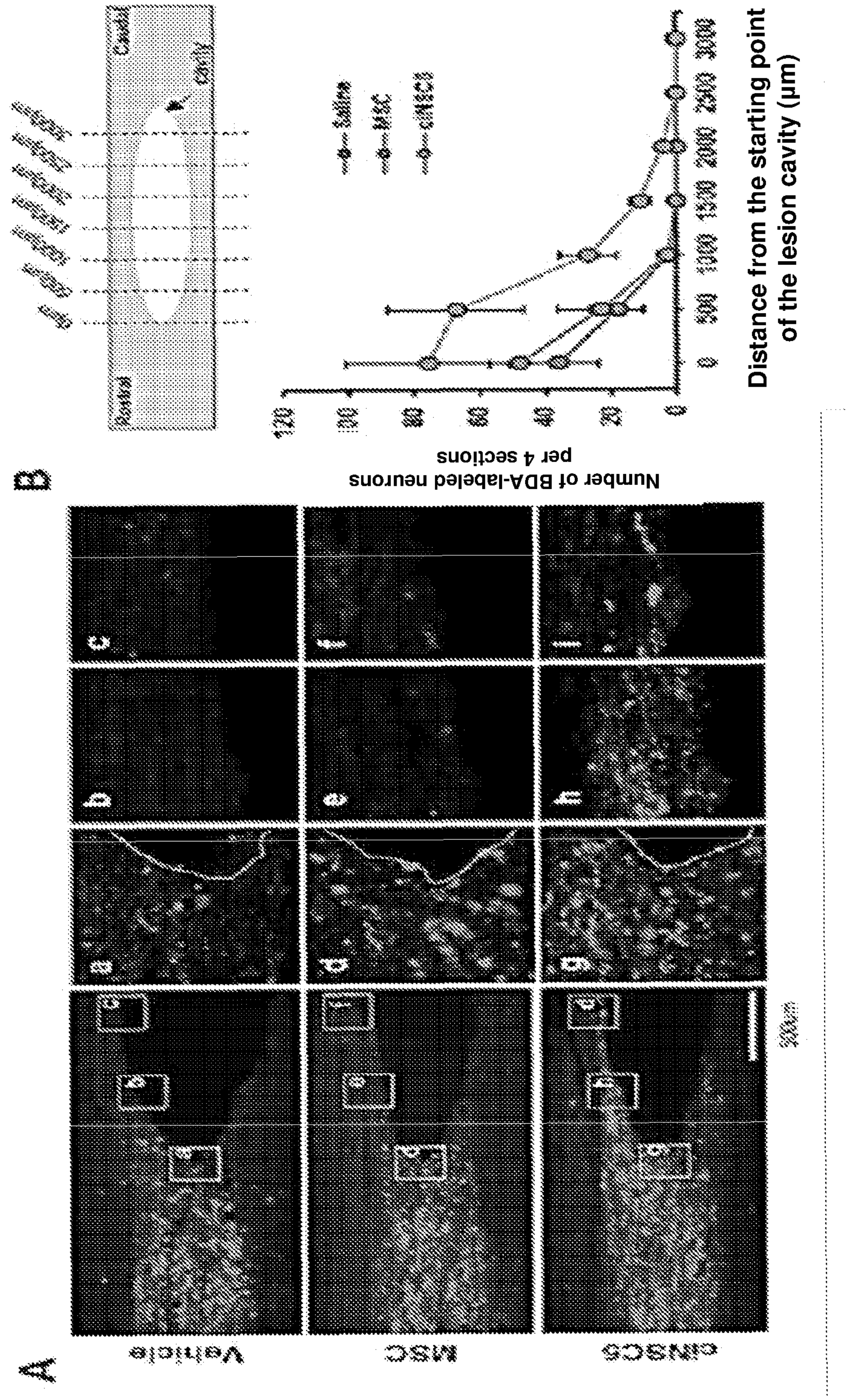
A
Vehicle
MSC
ciNSCs
B
Rostral
Caudal
Cavity
Number of BDA-labeled neurons per 4 sections
Distance from the starting point of the lesion cavity (µm)
Saline
MSC
ciNSCs
0
20
40
60
80
100
120
500
1000
1500
2000
2500
3000

FIG. 54

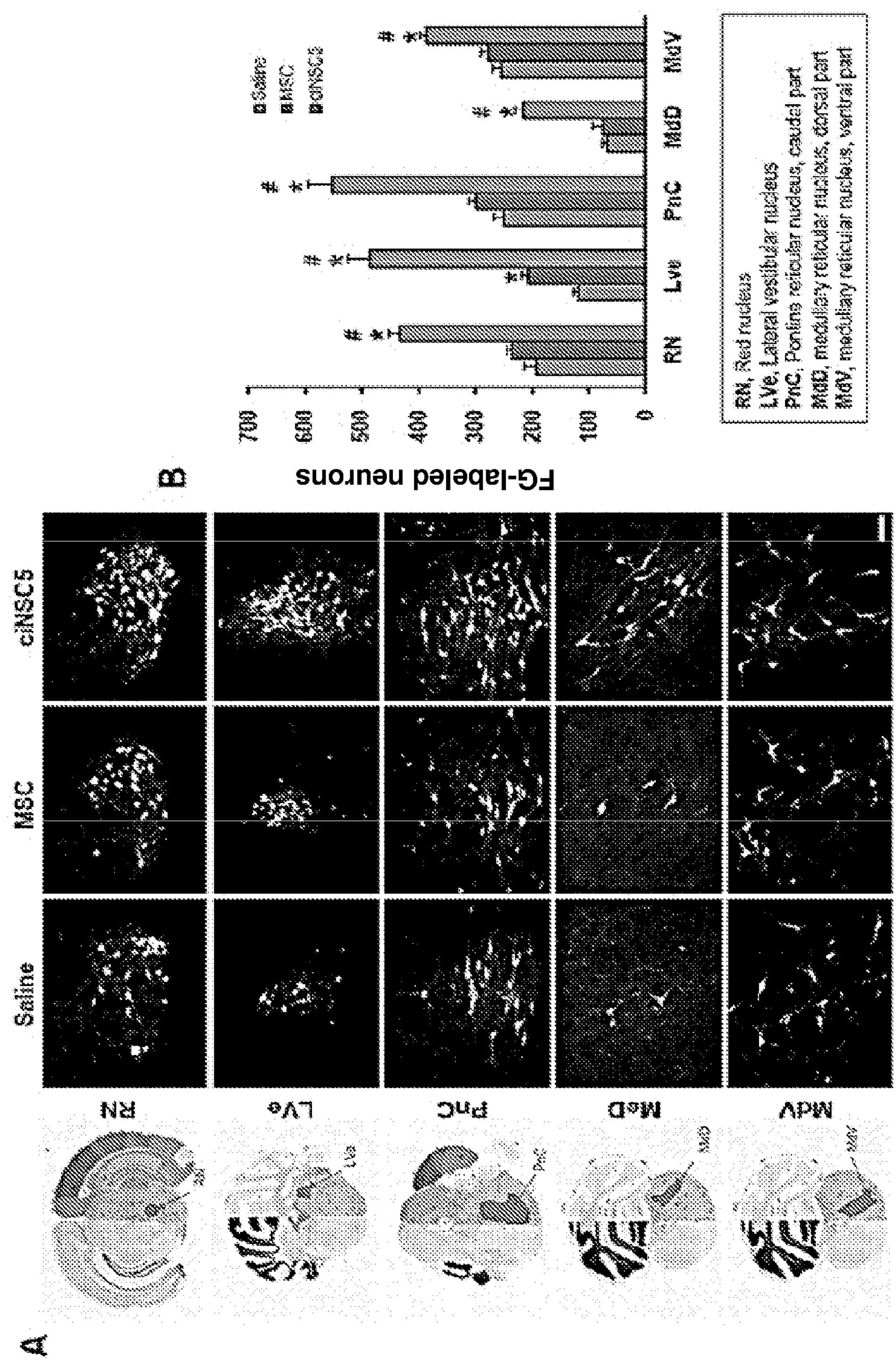
A
B
Saline
MSC
ciNSC5
RN
LVe
PnC
MdD
MdV
FG-labeled neurons
0
100
200
300
400
500
600
700
Saline
MSC
ciNSC5
RN, Red nucleus
LVe, Lateral vestibular nucleus
PnC, Pontine reticular nucleus, caudal part
MdD, medullary reticular nucleus, dorsal part
MdV, medullary reticular nucleus, ventral part

FIG. 55

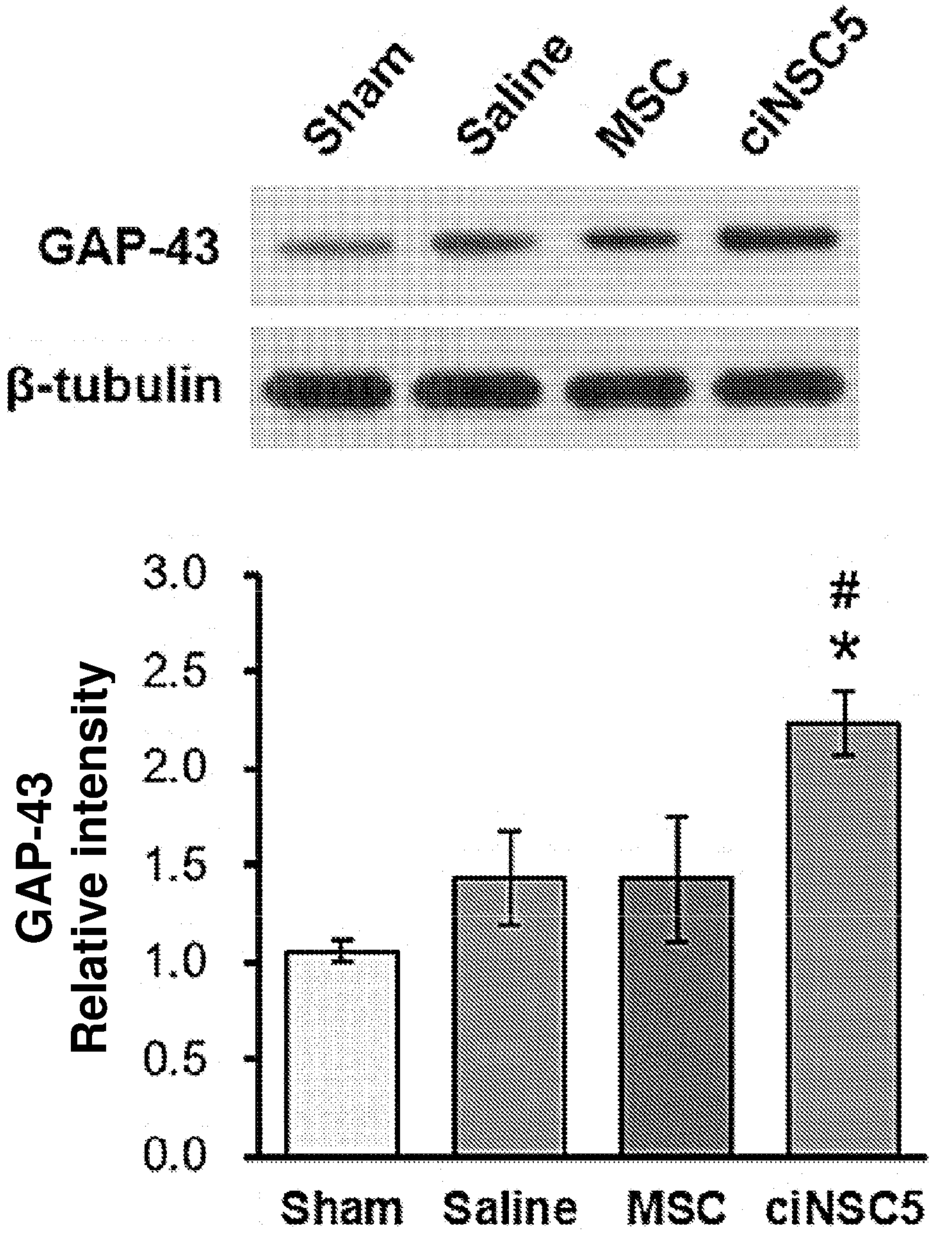
Sham
Saline
MSC
ciNSC5
GAP-43
β-tubulin
GAP-43
Relative intensity
3.0
2.5
2.0
1.5
1.0
0.5
0.0
#
*
Sham
Saline
MSC
ciNSC5

FIG. 56

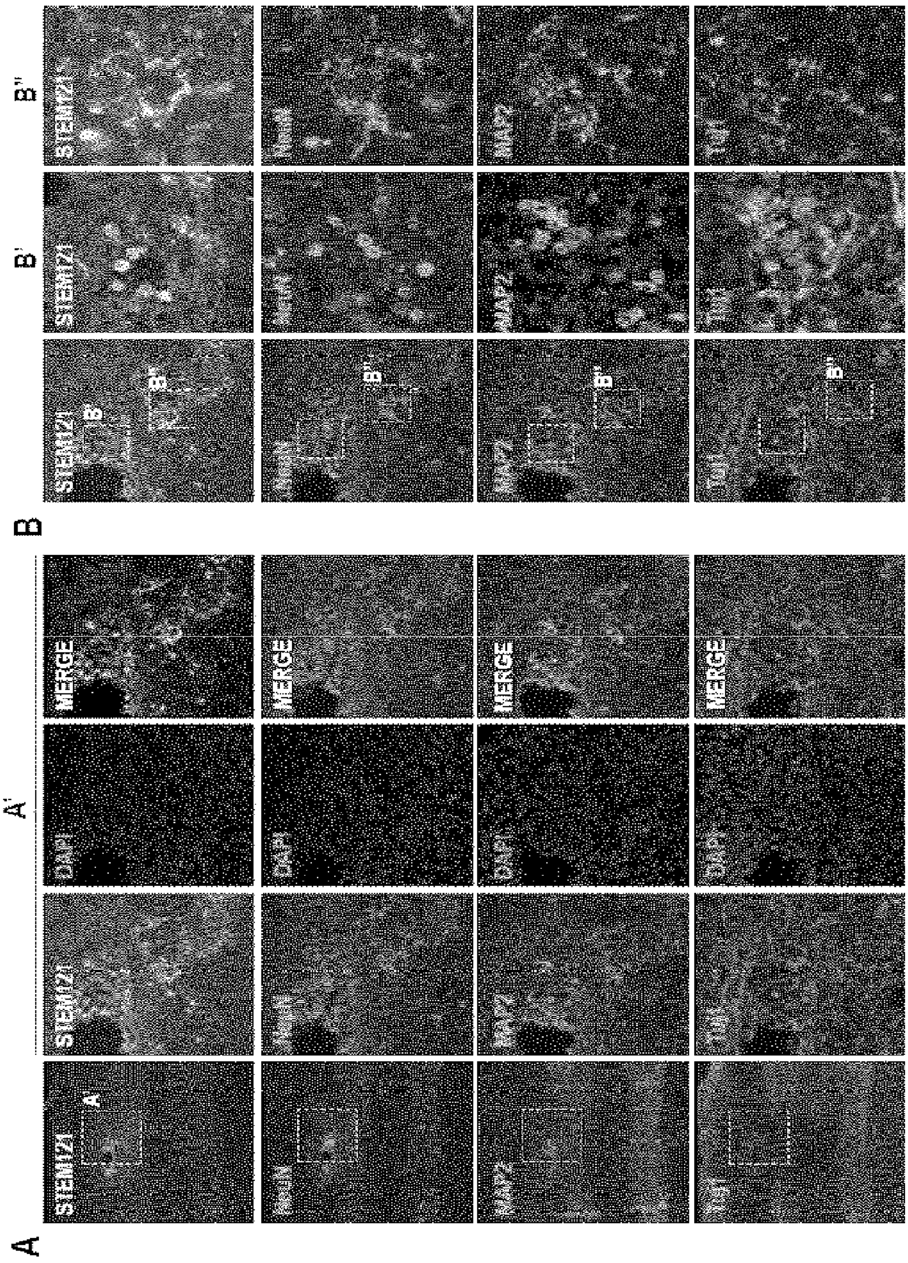

**INTERNATIONAL SEARCH REPORT**

International application No. **PCT/KR2022/018430**

**A. CLASSIFICATION OF SUBJECT MATTER**

**C12N 5/0775**(2010.01)i; **A61K 35/28**(2006.01)i; **A61P 25/00**(2006.01)i

According to International Patent Classification (IPC) or to both national classification and IPC

**B. FIELDS SEARCHED**

Minimum documentation searched (classification system followed by classification symbols)

C12N 5/0775(2010.01); A61K 31/423(2006.01); A61K 35/28(2006.01); A61P 25/00(2006.01); C07D 263/54(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 직접교차분화 (direct conversion), 올리고머화 (oligomerization), CtBPs (C-terminal binding proteins)

**C. DOCUMENTS CONSIDERED TO BE RELEVANT**

| Category* | Citation of document, with indication, where appropriate, of the relevant passages | Relevant to claim No. |
|---|---|---|
| X<br>A | KR 10-2019-0041897 A (EWHA UNIVERSITY - INDUSTRY COLLABORATION FOUNDATION) 23 April 2019 (2019-04-23)<br>See claims 1 and 18; paragraphs [0140], [0167] and [0354]-[0361]; and table 1. | 7-14,20-34,36-41,43,44<br>1-6,15-19,35,42 |
| X | KR 10-2019-0135112 A (UNIVERSITY-INDUSTRY COOPERATION GROUP OF KYUNG HEE UNIVERSITY) 06 December 2019 (2019-12-06)<br>See claims 1-7; and paragraph [0055]. | 24-27,30,31,<br>36,38-40,44 |
| X | WO 2021-100889 A1 (UNIVERSITY-INDUSTRY COOPERATION GROUP OF KYUNG HEE UNIVERSITY) 27 May 2021 (2021-05-27)<br>See claims 1-10; and paragraph [0060]. | 24-27,30,31,<br>36,38-40,44 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **16 March 2023** | **17 March 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

INTERNATIONAL SEARCH REPORT

International application No.
PCT/KR2022/018430

C. DOCUMENTS CONSIDERED TO BE RELEVANT

| Category* | Citation of document, with indication, where appropriate, of the relevant passages | Relevant to claim No. |
|---|---|---|
| A | RAY, S. K. et al. Oligomeric form of C-terminal-binding protein coactivates NeuroD1-mediated transcription. FEBS letters. 2017, vol. 591, pp. 205-212. See entire document. | 1-44 |
| A | STANKIEWICZ, T. R. et al. C-terminal binding proteins: central players in development and disease. Biomolecular concepts. 2014, vol. 5, no. 6, pp. 489-511. See entire document. | 1-44 |

Form PCT/ISA/210 (second sheet) (July 2022)

INTERNATIONAL SEARCH REPORT

International application No.
**PCT/KR2022/018430**

**Box No. II** **Observations where certain claims were found unsearchable (Continuation of item 2 of first sheet)**

This international search report has not been established in respect of certain claims under Article 17(2)(a) for the following reasons:

1. [x] Claims Nos.: **45**
because they relate to subject matter not required to be searched by this Authority, namely:

Claim 45 pertains to a method for treatment of the human body by surgery or therapy (PCT Article 17(2)(a)(i) and PCT Rule 39.1(iv)).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2022/018430**

| Patent document cited in search report | Publication date (day/month/year) | Patent family member(s) | Publication date (day/month/year) |
|---|---|---|---|
| KR 10-2019-0041897 A | 23 April 2019 | KR 10-2064611 B1 | 09 January 2020 |
| KR 10-2019-0135112 A | 06 December 2019 | KR 10-2064545 B1 | 09 January 2020 |
| WO 2021-100889 A1 | 27 May 2021 | CN 114760999 A | 15 July 2022 |
| | | EP 4046636 A1 | 24 August 2022 |
| | | US 2022-0401492 A1 | 22 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KIM et al.** *Exp. Neurobiol.,* 2014, vol. 23 (3), 207-214
- **TWELVES et al.** *Mov Disord,* vol. 18, 19-31
- **OLANOW et al.** *Annu Rev Neurosci,* vol. 22, 123-44
- **LANGSTON et al.** *Ann Neurol,* vol. 44 (1), 45-52
- Goodman and Gilman's The Pharmacological Basis of Therapeutics. Pergamon Press, 2001
- Remington's Pharmaceutical Sciences. Mack Publishing Co, 1990
- Remington's Pharmaceutical Science. Mack Publishing Company, 1990
- Merck Index. Merck & Co. Inc,